# EUROPEAN PATENT SPECIFICATION

(11) **EP 1 322 287 B1**
(45) Date of publication and mention of the grant of the patent: **22.03.2006**
(21) Application number: 01975584.2
(22) Date of filing: 28.09.2001
(51) Int. Cl.: A61K 9/00, A61K 9/16, A61K 9/107

(54) **MICROPARTICLES FOR DELIVERY OF THE HETEROLOGOUS NUCLEIC ACIDS**
MIKROPARTIKEL ZUR VERABREICHUNG VON HETEROLOGEN NUKLEINSÄURE
MICROPARTICULES DE TRANSPORT D'ACIDES NUCLEIQUES HETEROLOGUES

(30) Priority: 28.09.2000 US 236105 P; 30.08.2001 US 315905 P
(43) Date of publication of application: 02.07.2003
(73) Proprietor: CHIRON CORPORATION, Emeryville, California 94608-2916 (US)
(72) Inventor: O'HAGAN, Derek, Berkeley, CA 94705 (US); OTTEN, Gillis, Foster City, CA 94404 (US); DONNELLY, John, James, Moraga, CA 94556 (US); POLO, John, M., Hayward, CA 94542 (US); BARNETT, Susan, San Francisco, CA 94114 (US); SINGH, Manmohan, Hercules, CA 94547 (US); ULMER, Jeffrey, Danville, CA 94506 (US); DUBENSKY, Thomas, W., Jr., Piedmont, CA 94611 (US); Ott, Gary.S., Oakland, CA 94605-5820 (US)
(74) Representative: Marshall, Cameron John
(86) International application number: PCT/US2001/030540
(87) International publication number: WO 2002/026209

(56) References cited:
- WO-A-01/81609
- WO-A-98/33487
- J. KAZZAZ, ET AL.: "Induction of cytotoxic T-lymphocite activity in mice with HIV p24 gag protein adsorbed to the surface of poly(lactide-co glycolide) microparticles" PROCEED. INT'L. SYMP. CONTROL. REL. BIOACT. MATER., July 1999 (1999-07), pages 1046-1047, XP002216995
- K. S. DENIS-MIZE: "Plasmid DNA adsorbed onto cationic microparticles mediates target gene expression and antigen presentation by dendritic cells" GENE THERAPY, vol. 7, 2000, pages 2105-2112, XP002216996

## Description

### Technical Field

The present invention relates generally to pharmaceutical compositions. In particular, the invention relates to microparticles of polymers or submicron emulsions having adsorbent surfaces wherein biologically active agents, particularly nucleic acids, such as plasmid DNA, Eukaryotic Layered Vector Initiation Systems (ELVIS vectors) or RNA vector constructs, are adsorbed thereto, methods for preparing such microparticles and submicron emulsions, and uses thereof, including induction of immune responses, vaccines, and delivery of heterologous nucleotide sequences to eukaryotic cells and animals.

### Background

Particulate carriers have been used in order to achieve controlled, parenteral delivery of therapeutic compounds. Such carriers are designed to maintain the active agent in the delivery system for an extended period of time. Examples of particulate carriers include those derived from polymethyl methacrylate polymers, as well as microparticles derived from poly(lactides) (see, e.g., U.S. Patent No. 3,773,919), poly(lactide-co-glycolides), known as PLG (see, e.g., U.S. Patent No. 4,767,628) and polyethylene glycol, known as PEG (see, e.g., U.S. Patent No. 5,648,095). Polymethyl methacrylate polymers are nondegradable while PLG particles biodegrade by random nonenzymatic hydrolysis of ester bonds to lactic and glycolic acids which are excreted along normal metabolic pathways.

For example, U.S. Patent No. 5,648,095 describes the use of microspheres with encapsulated pharmaceuticals as drug delivery systems for nasal, oral, pulmonary and oral delivery. Slow-release formulations containing various polypeptide growth factors have also been described. See, e.g., International Publication No. WO 94/12158, U.S. Patent No. 5,134,122 and International Publication No. WO 96/37216.

Fattal et al., Journal of Controlled Release 53:137-143 (1998) describes nanoparticles prepared from polyalkylcyanoacrylates (PACA) having adsorbed oligonucleotides.

Particulate carriers, such as microparticles, have also been used with adsorbed or entrapped antigens in attempts to elicit adequate immune responses. Such carriers present multiple copies of a selected antigen to the immune system and promote trapping and retention of antigens in local lymph nodes. The particles can be phagocytosed by macrophages and can enhance antigen presentation through cytokine release. For example, commonly owned, co-pending Application No. 09/015,652, filed January 29, 1998, describes the use of antigen-adsorbed and antigen-encapsulated microparticles to stimulate cell-mediated immunological responses, as well as methods of making the microparticles.

In commonly owned Patent Application Serial No. 09/015,652, for example, a method of forming microparticles is disclosed which comprises combining a polymer with an organic solvent, then adding an emulsion stabilizer, such as polyvinyl alcohol (PVA), then evaporating the organic solvent, thereby forming microparticles. The surface of the microparticles comprises the polymer and the stabilizer. Macromolecules such as ELVIS vectors, other nucleotides (DNA or RNA), polypeptides, and antigens may then be adsorbed on those surfaces.

Adjuvants are compounds which are capable of potentiating an immune response to antigens. Adjuvants can potentiate both humoral and cellular immunity. However, it is preferable for certain pathogens to stimulate cellular immunity and, particularly, Th1 cells. In many instances, presently used adjuvants do not adequately induce Th1 cell responses, and/or have deleterious side effects.

Currently, the only adjuvants approved for human use in the United States are aluminum salts (alum). These adjuvants have been useful for some vaccines including hepatitis B, diphtheria, polio, rabies, and influenza, but may not be useful for others. For example, reports indicate that alum failed to improve the effectiveness of whooping cough and typhoid vaccines and provided only a slight effect with adenovirus vaccines. Additionally, problems such as, induction of granulomas at the injection site and lot-to-lot variation of alum preparations have been experienced.

Microparticles prepared from biodegradable and biocompatible polymers, known as the poly(lactide-co-glycolides) (PLG), have been demonstrated to be effective vehicles for a number of antigens. In addition, PLG microparticles can control the rate of release of entrapped antigens and, thus, offer potential for single-dose vaccines. Moreover, administration of biodegradable polymers with entrapped antigens has been demonstrated in a range of animal models to induce potent immune responses. O'Hagan *et al., Advanced Drug Deliv. Rev.,* **1998,** 32, 225-246 and Singh *et al., Advanced Drug Deliv. Rev.,* **1998,** *34*, 285-304, the disclosures of which are incorporated herein by reference in their entirety.

An emulsion comprising squalene, sorbitan trioleate (Span85™), and polysorbate 80 (Tween 80™) microfluidized to provide uniformly sized microdroplets, i.e. MF59, has also been shown to induce potent immune responses. MF59 formulations have been shown to induce antibody titers from 5 to >100 times greater than those obtained with aluminum salt adjuvants. MF59 has been demonstrated to enhance the immune response to antigens from numerous sources including, for example, herpes simplex virus (HSV), human immunodeficiency virus (HIV), influenza virus, hepatitis C virus (HCV), cytomegalovirus (CMV), hepatitis B virus (HBV), human papillomavirus (HPV), and malaria. Ott *et al., Vaccine Design: The Subunit And Adjuvant Approach,* **1995,** M.F. Powell and MJ. Newman, Eds., Plenum Press, New York, p. 277-296; Singh *et al., Vaccine,* **1998,** *16*,1822-1827; Ott *et al., Vaccine,* **1995,** *13*, 1557-1562; O'Hagan *et al., Mol. Medicine Today,* **1997,** February, 69-75; and Traquina *et al., J. Infect. Dis.,* **1996,** *174,* 1168-75, the disclosures of which are incorporated herein by reference in their entirety. MF59 adjuvant improves the immunogenicity of subunit antigens while maintaining the safety and tolerability profile of alum adjuvant. VanNest *et al*., *Vaccines 92,* **1992,** Cold Spring Harbor Laboratory Press, 57-62 and Valensi *et al*., *J. Immunol. ,* **1994**, *153*, 4029-39, the disclosures of which are incorporated herein by reference in their entirety. MF59 is further described in co-pending U.S. application Serial No. 08/434,512, filed May 4, 1995, which is assigned to the assignee of the present invention, the disclosure of which is incorporated herein by reference in its entirety. In animal studies, MF59 has not been found to be genotoxic, teratogenic, nor does it cause sensitization. The mechanism of action of MF59 appears to be dependent upon the generation of a strong CD4+ T cell, i.e., a Th2 cell response. MF59 adjuvants, however, elicit little, if any, Th1 responses, or cytotoxic T lymphocyte (CTL) responses.

Oligonucleotides comprising CpG motifs mixed with antigens have been demonstrated to induce strong Th1 immune responses. Roman *et al.*, *Nat. Med.*, **1997**, *3*, 849-854; Weiner *et al.*, *Proc. Natl. Acad. Sci. USA,* **1997**, 94, 10833-10837; Davis *et al.*, *J. Immunol.*, **1998**, *160*, 870-876; Chu. *et al.*, *J. Exp. Med.*, **1997**, 186, 1623-1631; Lipford *et al.*, *Eur. J. Immunol.*, **1997**, 27, 2340-2344; and Moldoveanu *et al.*, *Vaccine*, **1988**, *16*, 1216-1224, the disclosures of which are incorporated herein by reference in their entirety. Unmethylated CpG dinucleotides are relatively common in bacterial DNA, but are underrepresented and methylated in vertebrate DNA Bird, *Trends Genet.*, **1987**, *3*, 342-347. Bacterial DNA or synthetic oligonucleotides containing unmethylated CpG motifs are also known to induce immune responses including, for example, B cell proliferation, interleukin-6 and immunoglobulin secretion, and apoptosis resistance. Krieg *et al.*, *Nature*, **1995**, *374,* 546-549; Klinman *et al.*, *Proc. Natl. Acad Sci. USA*, **1996**, *93*, 2879-2883; Ballas *et al.*, *J. Immunol.,* **1996**, *157*, 1840-1845; Cowdery *et al.*, *J. Immunol*, **1996**, *156*, 4570-4575; Halpern *et al.*, *Cell. Immunol.*, **1996**, *167,* 72-78; Yamamoto *et al.*, *Jpn. J. Cancer Res.,* **1988**, *79*, 866-873; Stacey *et al.*, *J. Immunol.,* **1996**, *157*, 2116-2122; Messina *et al.*, *J. Immunol.,* **1991**, *147*, 1759-1764; Yi *et al.*, *J. Immunol.,* **1996**, *157,* 4918-4925; Yi *et al.*, *J. Immunol.*, **1996**, *157*, 5394-5402; Yi *et al.*, *J. Immunol.*, **1998**, *160*, 4755-4761; and Yi *et al.*, *J. Immunol.*, **1998**, *160,* 5898-5906; PCT Publication WO 96/02555; PCT Publication WO 98/16247; PCT Publication WO 98/18810; PCT Publication WO 98/40100; PCT Publication WO 98/55495; PCT Publication WO 98/37919; and PCT Publication WO 98/52581, the disclosures of which are incorporated herein by reference in their entirety.

It has also been shown that cationic lipid-based emulsions may be used as gene carriers. See, e.g., Yi et al, Proc. Int'I. Symp. Control. ReL Bioact. Mater., 24:653-654 (1997); Kim et al., Proc. Int'I. Symp. Control. Rel. Bioact. Mater., 25:344-345 (1998); Kim et al., Proc. Int'I. Symp. Control. ReL Bioact. Mater., 26, #5438 (1999). Cationic submicron emulsions, a somewhat recent approach to pharmaceutical delivery, were first shown to have carrying capacity for small molecule drugs (Elbaz et al 1993 Int. J. Pharm. 96 R1-R6). Use of the charged surface to stably bind and protect oligonucleotides in serum has been demonstrated for both small oligomers (Teixera et al (1999) Pharm Res 16 30-36) and plasmid DNA (Yi et al (2000) Pharm Res 17 314-320.) DOTAP-based emulsions have been shown to enhance transfection in vitro and in vivo. (Kim et al., *supra).*

An adjuvant which results in the increase of a Th1 cell response which can be used for prophylactic and therapeutic treatment is thus desirable. Such a response would be helpful in treatment of, for example, viral infections as well as for immimizing individuals susceptible to viral infections.

U.S. Patents 5,814,482 and 6,015,686 disclose Eukaryotic Layered Vector Initiation Systems (ELVIS vectors), particularly those derived and constructed from alphavirus genomes (such as Sindbis virus), for use in stimulating an immune response to an antigen, in methods of inhibiting pathogenic agents, and in delivery of heterologous nucleotide sequences to eukaryotic cells and animals, among others.

Commonly owned International patent application PCT/US99/17308 and U.S. patent application 09/715,902 disclose methods of making microparticles having adsorbed macromolecules, such as a pharmaceutical, a polynucleotide, a polypeptide, a protein, a hormone, an enzyme, a transcription or translation mediator, an intermediate in a metabolic pathway, an immunomodulator, an antigen, an adjuvant, or combinations thereof, and the like.

Commonly owned International patent application PCT/US00103331 discloses methods of making submicron emulsions having adsorbed macromolecules, such as a pharmaceutical, a polynucleotide, a polypeptide, a protein, a hormone, an enzyme, a transcription or translation mediator, an intermediate in a metabolic pathway, an immunomodulator, an antigen, an adjuvant, or combinations thereof, and the like.

### Summary of the Invention

The inventors herein have discovered that the effectiveness of the various uses of nucleic acids, particularly vector constructs capable of expressing a nucleic acid sequence, and more particularly vector constructs comprising a heterologous nucleic acid sequence encoding an antigen, such as such pCMV vectors, ELVIS vectors or RNA vector constructs may be enhanced by adsorbing the vector constructs to polymer microparticles or submicron emulsions with adsorbent surfaces, which facilitates introduction of the vector constructs, and ofheterologous nucleic acid sequences comprised in the vector constructs, into the cells of an animal.

As disclosed in above described International Patent Application PCT/US99/17308, a method of forming microparticles with adsorbent surfaces capable of adsorbing a wide variety of macromolecules has been invented. In one embodiment, the microparticles are comprised of both a polymer and a detergent. The microparticles of the present invention adsorb such macromolecules more efficiently than other microparticles currently available.

Several embodiments of the present invention utilize microparticles that are derived from a polymer, such as a poly(α-hydroxy acid), a polyhydroxy butyric acid, a polycaprolactone, a polyorthoester, a polyanhydride, a polyalkylcyanoacrylate, a polycyanoacrylate, and the like, and are formed with detergents, such as cationic, anionic, or nonionic detergents, which detergents may be used in combination. The present inventors have discovered that these microparticles yield improved adsorption of vector constructs (e.g., ELVIS vectors, RNA vector constructs), as well as viral antigens, and provide for superior immune responses.

As disclosed in above-described International Patent Application PCT/US00/03331, a microparticle preparation comprising oil droplet submicron emulsions with ionic surfactants has been invented. Such compositions readily adsorb macromolecules such as DNA, protein, and other antigenic molecules. Several embodiments of the present invention utilize microparticles that are derived from an oil droplet emulsion that preferably comprises a metabolizable oil and an emulsifying agent which are preferably present in the form of an oil-in-water emulsion having oil droplets substantially all of which are less than 1 micron in diameter, preferably smaller than 250 nm. Preferably, the composition exists in the absence of any polyoxypropylene-polyoxyethylene block copolymer. The oil is preferably an animal oil, an unsaturated hydrocarbon, a terpenoid such as, for example, squalene, or a vegetable oil. The composition preferably comprises 0.5 to 20 % by volume of the oil in an aqueous medium. The emulsifying agent preferably comprises a non-ionic detergent such as a polyoxyethylene sorbitan mono-, di-, or triester or a sorbitan mono-, di-, or triether. Preferably, the composition comprises about 0.01 to about 5 % by weight of the emulsifying agent.

Hence, in some embodiments, the particulate portion of the invention's composition is a microparticle with an adsorbent surface, wherein the microparticle comprises a polymer selected from the group consisting of a poly(α-hydroxy acid), a polyhydroxy butyric acid, a polycaprolactone, a polyorthoester, a polyanhydride, and a polycyanoacrylate.

In another embodiments, the particulate portion of the invention's composition is a submicron emulsion which comprise an oil droplet emulsion formulated with an ionic detergent.

The microparticle further comprises vector constructs capable of expressing a nucleic acid sequence, such as a selected ELVIS vector or RNA vector construct adsorbed on the microparticle's surface, with the vector construct comprising a heterologous nucleotide sequence encoding a polypeptide, a protein, a hormone, an enzyme, a transcription or translation mediator, an intermediate in a metabolic pathway, an immunomodulator, an antigen, an adjuvant, or combinations thereof, and the like.

In other embodiments, the invention is directed to a microparticle composition comprising a nucleic acid, preferably vector constructs capable of expressing a nucleic acid sequence, such as a selected pCMV vector, ELVIS vector or RNA vector construct, adsorbed to a microparticle of the invention, and a pharmaceutically acceptable excipient.

In other embodiments, the invention is directed to a method of producing a microparticle with an adsorbed nucleic acid, preferably vector constructs capable of expressing a nucleic acid sequence, such as an ELVIS vector or RNA vector construct, the method comprising:
(a) combining a polymer solution comprising a polymer selected from the group consisting of a poly(α-hydroxy acid), a polyhydroxy butyric acid, a polycaprolactone, a polyorthoester, a polyanhydride, and a polycyanoacrylate, wherein the polymer is present at a concentration of about 1% to about 30% in an organic solvent;
   and an anionic, cationic, or nonionic detergent to the polymer solution, wherein the detergent is present at a ratio of 0.001 to 10 (w/w) detergent to polymer, to form a polymer/detergent mixture;
(b) dispersing the polymer/detergent mixture;
(c) removing the organic solvent;
(d) recovering the microparticle; and
(f) adsorbing an ELVIS vector or RNA vector construct to the surface of the microparticle, wherein the ELVIS vector or RNA vector construct comprises a heterologous nucleotide sequence encoding a polypeptide, a protein, a hormone, an enzyme, a transcription or translation mediator, an intermediate in a metabolic pathway, an immunomodulator, an antigen, an adjuvant, or combinations thereof, and the like.

Preferably, the polymer/detergent mixture is emulsified to form an emulsion prior to removing the organic solvent.

In other embodiments, the invention is directed to a microparticle produced by the above-described methods. More preferably, a microparticle composition is produced, which also comprises a pharmaceutically acceptable excipient.

In still other embodiments, the invention is directed to the described microparticles for use in delivering a heterologous nucleotide sequence to a vertebrate subject, which comprises administering to a vertebrate subject any of the compositions described above.

In additional embodiments, the invention is directed to the described microparticles for use in eliciting a cellular immune response in a vertebrate subject comprising administering to the vertebrate subject a therapeutically effective amount of a selected heterologous nucleotide sequence adsorbed to a microparticle of the invention.

In other embodiments, the invention is directed to the described microparticles for use in immunization which comprises administering to a vertebrate subject a therapeutically effective amount of any of the microparticle compositions above. The composition may optionally contain unbound macromolecules, and also may optionally contain adjuvants, including aluminum salts such as aluminum phosphate, or an oligonucleotide comprising at least one CpG motif

In several preferred embodiments, the microparticles are formed from a poly(α-hydroxy acid); more preferably, a poly(D,L-lactide-co-glycolide); and most preferably, a poly(D,L-lactide-co-glycolide).

Each of the nonexhaustive previously described adsorbent microparticles may optionally also have macromolecules entrapped within them, or in free solution. Thus, the invention encompasses a variety of combinations wherein nucleic acid molecules are adsorbed on microparticles and other nucleic acid molecules are entrapped or adsorbed. Moreover, the microparticles of the invention may have more than one species of nucleic acid adsorbed thereon, as well as other antigenic macromolecules adsorbed thereon. Additionally, the microparticles may have several species of nucleic acid and/or other antigenic macromolecules entrapped within.

In other preferred embodiments, the microparticles are prepared in the form of submicron emulsions as described above.

The present invention is also directed to immunogenic compositions comprising an immunostimulating amount of a nucleic acid (e.g., a vector construct capable of expressing a nucleic acid sequence, such as a selected ELVIS vector or RNA vector construct, where the heterologous nucleotide sequence portion of the ELVIS vector or RNA vector construct may encode an antigen), and an immunostimulating amount of an adjuvant composition described herein. In some embodiments of the invention, the immunogenic composition comprises a CpG oligonucleotide in combination with the nucleic acid- adsorbed microparticles. The adsorbed macromolecule itself is preferably an ELVIS vector or RNA vector construct encoding an antigenic polypeptide.

In some preferred embodiments of the invention, the antigenic polypeptide is from a virus such as, for example, hepatitis C virus (HCV), hepatitis B virus (HBV), herpes simplex virus (HSV), human immunodeficiency virus (HIV), cytomegalovirus (CMV), influenza virus (flu), and rabies virus. Preferably, the antigenic polypeptide is selected from the group consisting ofHSV glycoprotein gD, HIV glycoprotein gp120, HIV glycoprotein gp140, HIV p55 gag, and polypeptides from the pol and tat regions. In other preferred embodiments of the invention, the antigenic polypeptide is from a bacterium such as, for example, cholera, diphtheria, tetanus, streptococcus (e.g., streptococcus B), pertussis, *Neisseria meningitidis* (e.g., meningitis B); *Neisseria gonorrhoeae*, *Helicobacter pylori,* and *Haemophilus influenza*. In still other preferred embodiments of the invention, the antigenic polypeptide is from a parasite such as, for example, a malaria parasite.

Adjuvant compositions may comprise, for example, aluminum salts. Alternatively, adjuvant compositions may comprise an oligonucleotide comprising at least one CpG motif. The adjuvant composition can also comprise an optional component which results in a positively charged emulsion. The oligonucleotide preferably comprises at least one phosphorothioate bond or peptide nucleic acid bond. In preferred embodiments of the invention, the oligonucleotide comprises a nucleotide sequence selected from the group consisting of SEQ ID NOs: 1-28. In other preferred embodiments of the invention, the oligonucleotide comprises a CpG motif flanked by two purines immediately 5' to the motif and two pyrimidines immediately 3' to the motif In other preferred embodiments of the invention, the oligonucleotide comprises a nucleotide sequence selected from the group consisting of SEQ ID NOs: 19-28. Most preferred is SEQ ID NO:28. In some preferred embodiments of the invention, the adjuvant composition further comprises a separate immunostimulating agent which is preferably selected from the group consisting of alum, a bacterial cell wall component, and muramyl peptide. The adjuvant composition itself may be in the form of a second microparticle. The second microparticle may have adsorbed and/or entrapped within a variety of nucleic acids and/or antigenic polypeptides, or other antigenic macromolecules. Additionally, the immunogenic compositions may include the presence of free nucleic acid in solution.

The present invention is also directed to the described microparticles for use in stimulating an immune response in a host animal comprising administering to the animal an immunogenic composition described herein in an amount effective to induce an immune response. The host animal is preferably a mammal, more preferably a Rhesus macaque, and still more preferably a human.

The present invention is also directed to the described microparticles for use in immunizing a host animal against a viral, bacterial, or parasitic infection comprising administering to the animal an immunogenic composition described herein in an amount effective to induce a protective response. The host animal is preferably a mammal, more preferably a Rhesus macaque, and still more preferably a human.

The present invention is also directed to the described microparticles for use in increasing a Th1 immune response, or a CTL response, or lyphoproliferation, or cytokine production in a host animal comprising administering to the animal an immunogenic composition described herein in an amount effective to induce the Th1 immune response, or the CTL response, or lyphoproliferation, or cytokine production. The host animal is preferably a mammal, more preferably a Rhesus macaque, and still more preferably a human.

The present invention is also directed to the use of the described microparticles in the manufacture of a medicament for raising an immune response in a host animal in which a microparticle-adsorbed macromolecule comprising a heterologous nucleic acid sequence encoding a first antigen (e.g., plasmid DNA, such as pCMV or an ELVIS vector, or an RNA vector construct) is first administered to the animal in an amount effective to elicit an immunological response. Subsequently, a second antigen is administered to the animal

The first antigen and the second antigen in these embodiments can be the same or different, and are preferably the same. Preferred antigens include bacterial and viral antigens, such as HIV antigens (e.g., gp120, gp140, gp160, p24gag and p55gag), hepatitis C virus antigens, influenza A virus antigens, meningitis B bacterial antigens, and streptococcus B bacterial antigens. The second antigen is preferably adsorbed to the microparticles described herein, or is coadministered with an adjuvant, such as MF59. The macromolecule can also be coadmmistered with an adjuvant, if desired. In some preferred embodiments, the macromolecule is administered two or more times before the second antigen, which can also be administered two or more times.

According to one specific embodiment: (1) the macromolecule is administered (a) at a time of initial administration, (b) at a time period ranging 1-8 weeks from the initial administration, and (c) at a time period ranging 4-32 weeks from the initial administration, and (2) the second antigen is administered (a) at a time period ranging from 8-50 weeks from the initial administration and (b) at a time period ranging from 8-100 weeks from the initial administration.

Delivery of the microparticle compositions of the invention may be performed by any known method, including direct injection (e.g., subcutaneously, intraperitoneally, intravenously or intramuscularly), and such delivery may also be enhanced by the use of electroporation *(see, e.g.,* U.S. patent application no. 09/499,023, the disclosure of which is hereby incorporated by reference in its entirety). Electroporation is the application of short electrical pulses to cells to increase permeability of the cell membranes, thus facilitating DNA uptake by cells. Recently it has been found that applying an electric field to tissues in vivo significantly increases DNA uptake and gene expression (Mathiesen, I., 1999, Gene Therapy 6:508). Among the tissues targeted for in vivo electroporation have been skin, liver, tumors, and muscle. For DNA vaccine application, Widera et al have shown that in vivo electroporation substantially enhances DNA vaccine potency in mice, guinea pigs, and rabbits (Widera, G., et al., 2000, J. Immunol. 164:4635).

The ELVIS vectors of the above-described embodiments are generally DNA molecules comprising a promoter that functions in a eukaryotic cell, a cDNA sequence for which the transcription product is an RNA vector construct (e.g., alphavirus RNA vector replicon), and a 3' termination region. The RNA vector constructs preferably comprise an RNA genome from a picornavirus, togavirus, flavivirus, coronavirus, paramyxovirus, yellow fever virus, or alphavirus (e.g., Sindbis virus, Semliki Forest virus, Venezuelan equine encephalitis virus, or Ross River virus), and more preferably an alphavirus genome, which has been modified by the replacement of one or more structural protein genes with a selected heterologous nucleic acid sequence encoding a gene product of interest. The RNA vector constructs of the present invention generally are obtained by transcription *in vitro* from a DNA template.

These and other aspects and embodiments of the present invention will readily occur to those of ordinary skill in the art in view of the disclosure herein.

### Brief Description of the Drawings

Fig. 1 provides a DNA sequence (SEQ ID NO:63) encoding a modified HIV-1 p55gag polypeptide.
Fig. 2 provides a DNA sequence (SEQ ID NO:64) encoding a modified HIV-1 p55gag polypeptide.
Fig. 3 provides a DNA sequence (SEQ ID NO:65) encoding a modified HIV-1 envelope polypeptide.
Fig. 4 provides a DNA sequence (SEQ ID NO:66) encoding a modified HIV-1 envelope polypeptide.
Fig. 5 provides a DNA sequence (SEQ ID NO:67) encoding a modified HIV-1 p55gag polypeptide.
Fig. 6 provides a DNA sequence (SEQ ID NO:68) encoding a modified HIV-1 p55gag polypeptide.

### Detailed Description of the Invention

The present invention is based upon the surprising discovery that microparticles with adsorbed nucleic acid molecules, preferably vector constructs capable of expressing a nucleic acid sequence, and more preferably vector constructs comprising a heterologous nucleic acid sequence encoding an antigen, such as pCMV vectors, ELVIS vectors or RNA vector constructs, elicit enhanced immune responses. Additionally, the combination of microparticles with adsorbed nucleic acid molecules (for example, microparticles with adsorbed pCMV vectors, ELVIS vectors or RNA vector constructs) and adjuvants is useful for eliciting enhanced immune responses.

The invention is also based upon the surprising discovery that vector constructs comprising antigen-encoding nucleic acid sequences, such as pCMV vectors, ELVIS vectors or RNA vector constructs, in association with subsequent administration of antigen, elicit enhanced immune responses.

The practice of the present invention will employ, unless otherwise indicated, conventional methods of chemistry, polymer chemistry, biochemistry, molecular biology, immunology and pharmacology, within the skill of the art. Such techniques are explained fully in the literature. See, *e.g., Remington's Pharmaceutical Sciences,* 18th Edition (Easton, Pennsylvania: Mack Publishing Company, 1990); *Methods In Enzymology* (S. Colowick and N. Kaplan, eds., Academic Press, Inc.); *Handbook of Experimental Immunology,* Vols. I-IV (D.M Weir and C.C. Blackwell, eds., 1986, Blackwell Scientific Publications); Sambrook, et al., *Molecular Cloning: A Laboratory Manual* (2nd Edition, 1989); *Handbook of Surface and Colloidal Chemistry* (Birdi, K.S., ed, CRC Press, 1997) and *Seymour*/*Carraher's Polymer Chemistry* (4th edition, Marcel Dekker Inc., 1996).

All publications, patents and patent applications cited herein, whether *supra* or *infra*, are hereby incorporated by reference in their entirety.

As used in this specification and the appended claims, the singular forms "a," "an" and "the" include plural references unless the content clearly dictates otherwise. Thus, for example, the term "microparticle" refers to one or more microparticles, and the like.

### A. Definitions

In describing the present invention, the following terms will be employed, and are intended to be defined as indicated below.

Unless stated otherwise, all percentages and ratios herein are given on a weight basis.

The term "microparticle" as used herein, refers to a particle of about 10 nm to about 150 µm in diameter, more preferably about 200 nm to about 30 µm in diameter, and most preferably about 500 nm to about 10 µm in diameter. Preferably, the microparticle will be of a diameter that permits parenteral or mucosal administration without occluding needles and capillaries. Microparticle size is readily determined by techniques well known in the art, such as photon correlation spectroscopy, laser diffractometry and/or scanning electron microscopy. The term "particle" may also be used to denote a microparticle as defined herein. Microparticle may alternatively refer to a submicron emulsion composition as described herein.

Polymer microparticles for use herein are formed from materials that are sterilizable, non-toxic and biodegradable. Such materials include, without limitation, poly(α-hydroxy acid), polyhydroxybutyric acid, polycaprolactone, polyorthoester, polyanhydride, PACA, and polycyanoacrylate. Preferably, microparticles for use with the present invention are polymer microparticles derived from a poly(α-hydroxy acid), in particular, from a poly(lactide) ("PLA") or a copolymer of D,L-lactide and glycolide or glycolic acid, such as a poly(D,L-lactide-co-glycolide) ("PLG" or "PLGA"), or a copolymer of D,L-lactide and caprolactone. The polymer microparticles may be derived from any of various polymeric starting materials which have a variety of molecular weights and, in the case of the copolymers such as PLG, a variety of lactide:glycolide ratios, the selection of which will be largely a matter of choice, depending in part on the coadministered macromolecule. These parameters are discussed more fully below. Alternatively, microparticles of the invention are comprised in a submicron emulsion.

As used herein, the phrase "oil droplet emulsion" refers to an emulsion comprising a metabolizable oil and an emulsifying agent. The term "submicron emulsion" as used herein refers to an oil droplet emulsion of the invention comprising droplets ranging in size from about 10 nm to about 1000 nm.

As used herein, the term "microparticle" may refer to a polymer microparticle as described herein or a submicron emulsion composition as described herein.

The term "detergent" as used herein includes surfactants, dispersing agents, suspending agents, and emulsion stabilizers. Anionic detergents include, but are not limited to, SDS (sodium dodecyl sulfate), SLS (sodium lauryl sulfate), DSS (disulfosuccinate), sulphated fatty alcohols, and the like. Cationic detergents include, but are not limited to, cetrimide (cetyl trimethyl ammonium bromide, or "CTAB"), benzalkonium chloride, DDA (dimethyl dioctodecyl ammonium bromide), DOTAP(dioleoyl-3-trimethylammonium-propane), and the like. Nonionic detergents include, but are not limited to, PVA, povidone (also known as polyvinylpyrrolidone or PVP), sorbitan esters, polysorbates, polyoxyethylated glycol monoethers, polyoxyethylated alkyl phenols, poloxamers, and the like.

The term "zeta potential" as used herein, refers to the electrical potential that exists across the interface of all solids and liquids, i.e., the potential across the diffuse layer of ions surrounding a charged colloidal particle. Zeta potential can be calculated from electrophoretic mobilities, i.e., the rates at which colloidal particles travel between charged electrodes placed in contact with the substance to be measured, using techniques well known in the art.

The term "macromolecule" as used herein refers to, without limitation, a pharmaceutical, a polynucleotide, a polypeptide, a hormone, an enzyme, a transcription or translation mediator, an intermediate in a metabolic pathway, an immunomodulator, an antigen, an adjuvant, or combinations thereof Particular macromolecules for use with the present invention are described in more detail below.

The term "pharmaceutical" refers to biologically active compounds such as antibiotics, antiviral agents, growth factors, hormones, and the like, discussed in more detail below.

The term "adjuvant" refers to any substance that assists or modifies the action of a pharmaceutical, including but not limited to immunological adjuvants, which increase or diversify the immune response to an antigen.

A "polynucleotide" is a nucleic acid polymer, which typically encodes a biologically active (e.g., immunogenic or therapeutic) protein or polypeptide. Depending on the nature of the polypeptide encoded by the polynucleotide, a polynucleotide can include as little as 10 nucleotides, e.g., where the polynucleotide encodes an antigen. Furthermore, a "polynucleotide" can include both double- and single-stranded sequences and refers to, but is not limited to, cDNA from viral, procaryotic or eucaryotic mRNA, genomic RNA and DNA sequences from viral (e.g. RNA and DNA viruses and retroviruses) or procaryotic DNA, and especially synthetic DNA sequences. The term also captures sequences that include any of the known base analogs of DNA and RNA. The term further includes modifications, such as deletions, additions and substitutions (generally conservative in nature), to a native sequence, preferably such that the nucleic acid molecule encodes a therapeutic or antigenic protein. These modifications may be deliberate, as through site-directed mutagenesis, or may be accidental, such as through mutations ofhosts which produce the antigens.

The terms "polypeptide" and "protein" refer to a polymer of amino acid residues and are not limited to a minimum length of the product. Thus, peptides, oligopeptides, dimers, multimers, and the like, are included within the definition. Both full-length proteins and fragments thereof are encompassed by the definition. The terms also include modifications, such as deletions, additions and substitutions (generally conservative in nature), to a native sequence, preferably such that the protein maintains the ability to elicit an immunological response or have a therapeutic effect on a subject to which the protein is administered.

By "antigen" is meant a molecule which contains one or more epitopes capable of stimulating a host's immune system to make a cellular antigen specific immune response when the antigen is presented in accordance with the present invention, or a humoral antibody response. An antigen may be capable of eliciting a cellular or humoral response by itself or when present in combination with another molecule. Normally, an epitope will include between about 3-15, preferably about 5-15, and more preferably about 7-15 amino acids. Epitopes of a given protein can be identified using any number of epitope mapping techniques, well known in the art. S ee, e.g., *Epitope Mapping Protocols* in Methods in Molecular Biology, Vol. 66 (Glenn E. Morris, Ed., 1996) Humana Press, Totowa, New Jersey. For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Patent No. 4,708,871; Geysen et al. (1984) *Proc. Natl. Acad. Sci. USA.* 81:3998-4002; Geysen et al. (1986) *Molec. Immunol.* 23:709-715, all incorporated herein by reference in their entireties. Similarly, conformational epitopes are readily identified by determining spatial conformation of amino acids such as by, e.g., x-ray crystallography and 2-dimensional nuclear magnetic resonance. See, e.g., *Epitope Mapping Protocols, supra.*

The term "antigen" as used herein denotes both subunit antigens, i.e., antigens which are separate and discrete from a whole organism with which the antigen is associated in nature, as well as killed, attenuated or inactivated bacteria, viruses, parasites or other microbes. Antibodies such as anti-idiotype antibodies, or fragments thereof, and synthetic peptide mimotopes, which can mimic an antigen or antigenic determinant, are also captured under the definition of antigen as used herein. Similarly, an oligonucleotide or polynucleotide which expresses a therapeutic or immunogenic protein, or antigenic determinant *in vivo,* such as in gene therapy and nucleic acid immunization applications, is also included in the definition of antigen herein.

Further, for purposes of the present invention, antigens can be derived from any of several known viruses, bacteria, parasites and fungi, as well as any of the various tumor antigens. Furthermore, for purposes of the present invention, an "antigen" refers to a protein which includes modifications, such as deletions, additions and substitutions (generally conservative in nature), to the native sequence, so long as the protein maintains the ability to elicit an immunological response. These modifications may be deliberate, as through site-directed mutagenesis, or may be accidental, such as through mutations of hosts which produce the antigens.

An "immunological response" or ''immune response" to an antigen or composition is the development in a subject of a humoral and/or a cellular immune response to molecules present in the composition of interest. For purposes of the present invention, a "humoral immune response" refers to an immune response mediated by antibody molecules, while a "cellular immune response" is one mediated by T-lymphocytes and/or other white blood cells. One important aspect of cellular immunity involves an antigen-specific response by cytolytic T-cells ("CTLs"). CTLs have specificity for peptide antigens that are presented in association with proteins encoded by the major histocompatibility complex (MHC) and expressed on the surfaces of cells. CTLs help induce and promote the intracellular destruction of intracellular microbes, or the lysis of cells infected with such microbes. Another aspect of cellular immunity involves an antigen-specific response by helper T-cells. Helper T-cells act to help stimulate the function, and focus the activity of, nonspecific effector cells against cells displaying peptide antigens in association with MHC molecules on their surface. A "cellular immune response" also refers to the production of cytokines, chemokines and other such molecules produced by activated T-cells and/or other white blood cells, including those derived from CD4+ and CD8+ T-cells.

A composition, such as an immunogenic composition, or vaccine that elicits a cellular immune response may serve to sensitize a vertebrate subject by the presentation of antigen in association with MHC molecules at the cell surface. The cell-mediated immune response is directed at, or near, cells presenting antigen at their surface. In addition, antigen-specific T-lymphocytes can be generated to allow for the future protection of an immunized host.

The ability of a particular antigen or composition to stimulate a cell-mediated immunological response may be determined by a number of assays, such as by lymphoproliferation (lymphocyte activation) assays, CTL cytotoxic cell assays, by assaying for T-lymphocytes specific for the antigen in a sensitized subject, or by measurement of cytokine production by T cells in response to restimulation with antigen. Such assays are well known in the art. See, e.g., Erickson et al., *J. Immunol.* (1993) 151:4189-4199; Doe et al., *Eur. J. Immunol.* (1994) 24:2369-2376; and the examples below.

Thus, an immunological response as used herein may be one which stimulates the production of CTLs, and/or the production or activation of helper T-cells. The antigen of interest may also elicit an antibody-mediated immune response. Hence, an immunological response may include one or more of the following effects: the production of antibodies by B-cells; and/or the activation of suppressor T-cells and/or γδ T-cells directed specifically to an antigen or antigens present in the composition or vaccine of interest. These responses may serve to neutralize infectivity, and/or mediate antibody-complement, or antibody dependent cell cytotoxicity (ADCC) to provide protection to an immunized host. Such responses can be determined using standard immunoassays and neutralization assays, well known in the art.

A composition which contains a selected antigen adsorbed to a microparticle, displays "enhanced immunogenicity" when it possesses a greater capacity to elicit an immune response than the immune response elicited by an equivalent amount of the antigen when delivered without association with the microparticle. Thus, a composition may display "enhanced immunogenicity" because the antigen is more strongly immunogenic by virtue of adsorption to the microparticle, or because a lower dose of antigen is necessary to achieve an immune response in the subject to which it is administered. Such enhanced immunogenicity can be determined by administering the microparticle/antigen composition, and antigen controls to animals and comparing antibody titers against the two using standard assays such as radioimmunoassay and ELISAs, well known in the art.

The terms "effective amount" or "pharmaceutically effective amount" of a given composition, as provided herein, refer to a nontoxic but sufficient amount of the composition to provide a desired response, such as an immunological response, and corresponding therapeutic effect, or in the case of delivery of a therapeutic protein, an amount sufficient to effect treatment of the subject, as defined below. As will be pointed out below, the exact amount required will vary from subject to subject, depending on the species, age, and general condition of the subject, the severity of the condition being treated, and the particular macromolecule of interest, mode of administration, and the like. An appropriate "effective" amount in any individual case may be determined by one of ordinary skill in the art using routine experimentation.

By "vertebrate subject" is meant any member of the subphylum cordata, including, without limitation, mammals such as cattle, sheep, pigs, goats, horses, and humans; domestic animals such as dogs and cats; and birds, including domestic, wild and game birds such as cocks and hens including chickens, turkeys and other gallinaceous birds. The term does not denote a particular age. Thus, both adult and newborn animals are intended to be covered.

By "pharmaceutically acceptable" or "pharmacologically acceptable" is meant a material which is not biologically or otherwise undesirable, i.e., the material may be administered to an individual along with the microparticle formulation without causing any undesirable biological effects in the individual or interacting in a deleterious manner with any of the components of the composition in which it is contained.

The term "excipient" refers to substances that are commonly provided within finished dosage forms, and include vehicles, binders, disintegrants, fillers (diluents), lubricants, glidants (flow enhancers), compression aids, colors, sweeteners, preservatives, suspensing/dispersing agents, film formers/coatings, flavors and printing inks.

By "physiological pH" or a "pH in the physiological range" is meant a pH in the range of approximately 7.2 to 8.0 inclusive, more typically in the range of approximately 7.2 to 7.6 inclusive.

As used herein, "treatment" (including variations thereof, for example, "treat" or ''treated'') refers to any of (i) the prevention of infection or reinfection, as in a traditional vaccine, (ii) the reduction or elimination of symptoms, and (iii) the substantial or complete elimination of the pathogen or disorder in question. Treatment may be effected prophylactically (prior to infection) or therapeutically (following infection).

As used herein, the phrase "nucleic acid" refers to DNA, RNA, or chimeras formed therefrom

As used herein, the phrase "oligonucleotide comprising at least one CpG motif' refers to a polynucleotide comprising at least one CpG dinucleotide. Oligonucleotides comprising at least one CpG motif can comprise multiple CpG motifs. These oligonucleotides are also known as "CpG oligonucleotides" in the art. As used herein, the phrase "CpG motif' refers to a dinucleotide portion of an oligonucleotide which comprises a cytosine nucleotide followed by a guanosine nucleotide. 5-methylcytosine can also be used in place of cytosine.

As used herein, "alphavirus RNA vector replicon," "RNA vector replicon," "RNA vector construct," and "replicon" refer to an RNA molecule which is capable of directing its own amplification or self-replication in vivo, within a target cell. An alphavirus-derived RNA vector replicon should contain the following ordered elements: 5' viral sequences required in cis for replication (also referred to as 5' CSE), sequences which, when expressed, code for biologically active alphavirus nonstructural proteins (e.g., nsP1, nsP2, nsP3, nsP4), 3' viral sequences required in cis for replication (also referred to as 3' CSE), and a polyadenylate tract. An alphavirus-derived RNA vector replicon also may contain a viral subgenomic "junction region" promoter, sequences from one or more structural protein genes or portions thereof, extraneous nucleic acid molecule(s) which are of a size sufficient to allow production of viable virus, as well as heterologous sequence(s) to be expressed.

As used herein, "Eukaryotic Layered Vector Initiation System," "ELVIS, "or "ELVIS vector" refers to an assembly which is capable of directing the expression of a sequence(s) or gene(s) of interest. The eukaryotic layered vector initiation system should contain a 5' promoter which is capable of initiating in vivo (i. e., within a cell) the synthesis of RNA from cDNA, and a viral vector sequence which is capable of directing its own replication in a eukaryotic cell and also expressing a heterologous sequence. In preferred embodiments, the nucleic acid vector sequence is an alphavirus-derived sequence and is comprised of a 5' sequence which is capable of initiating transcription of an alphavirus RNA (also referred to as 5' CSE), as well as sequences which, when expressed, code for-biologically active alphavirus nonstructural proteins (e.g, nsP1, nsP2, nsP3, nsP4), and an alphavirus RNA polymerase recognition sequence (also referred to as 3' CSE). In addition, the vector sequence may include a viral subgenomic "junction region" promoter, sequences from one or more structural protein genes or portions thereof, extraneous nucleic acid molecule(s) which are of a size sufficient to allow optimal amplification, a heterologous sequence to be expressed, one or more restriction sites for insertion ofheterologous sequences, as well as a polyadenylation sequence. The eukaryotic layered vector initiation system may also contain splice recognition sequences, a catalytic ribozyme processing sequence, a nuclear export signal, and a transcription termination sequence.

"Alphavirus vector construct" refers to an assembly which is capable of directing the expression of a sequence or gene of interest. Such vector constructs are generally comprised of a 5' sequence which is capable of initiating transcription of an alphavirus RNA (also referred to as 5' CSE), as well as sequences which, when expressed, code for biologically active alphavirus nonstructural proteins (e.g., nsP1, nsP2, nsP3, nsP4), an alphavirus RNA polymerase recognition sequence (also referred to as 3' CSE), and a polyadenylate tract. In addition, the vector construct may include a viral subgenomic "junction region" promoter, sequences from one or more structural protein genes or portions thereof, extraneous nucleic acid molecule(s) which are of a size sufficient to allow production of viable virus, a 5' promoter which is capable of initiating the synthesis of viral RNA from cDNA in vitro or in vivo, a heterologous sequence to be expressed, and one or more restriction sites for insertion of heterologous sequences.

As used herein, the phrase "vector construct" generally refers to any assembly which is capable of directing the expression of a nucleic acid sequence(s) or gene(s) of interest. The vector construct typically includes transcriptional promoter/enhancer or locus defining element(s), or other elements which control gene expression by other means such as alternate splicing, nuclear RNA export, post translational modification of messenger, or post-transcriptional modification of protein. In addition, the vector construct typically includes a sequence which, when transcribed, is operably linked to the sequence(s) or gene(s) of interest and acts as a translation initiation sequence. The vector construct may also optionally include a signal which directs polyadenylation, a selectable marker, as well as one or more restriction sites and a translation termination sequence. In addition, if the vector construct is placed into a retrovirus, the vector construct may include a packaging signal, long terminal repeats (LTRs), and positive and negative strand primer binding sites appropriate to the retrovirus used (if these are not already present). Examples of vector constructs include ELVIS vectors, which comprise the cDNA complement of RNA vector constructs, RNA vector constructs themselves, alphavirus vector constructs, CMV vector constructs and the like.

One specific type of vector construct is a "plasmid", which refers to a circular double stranded DNA, loop into which additional DNA segments can be ligated. Specific plasmids described below include pCMV and pSINCP.

According to some embodiments of the present invention, compositions and methods are provided which treat, including prophylactically and/or therapeutically immunize, a host animal against viral, fungal, mycoplasma, bacterial, or protozoan infections, as well as against tumors. The methods of the present invention are useful for conferring prophylactic and/or therapeutic immunity to a mammal, preferably a human. The methods of the present invention can also be practiced on mammals, other than humans, including mammals in biomedical research settings.

### B. General Methods

### 1 Polymer Microparticles with Adsorbed Macromolecules

Polymer microparticles, including PLA and PLG microparticles, efficiently adsorb biologically active macromolecules. Further, these microparticles adsorb a great variety of molecules, including charged and/or bulky macromolecules. Thus the macromolecule/microparticles used in connection with the present invention can be used as a delivery system to deliver the biologically active components in order to treat, prevent and/or diagnose a wide variety of diseases.

A wide variety of macromolecules can be delivered in association with the microparticles including, but not limited to, pharmaceuticals such as antibiotics and antiviral agents, nonsteroidal antiinflammatory drugs, analgesics, vasodilators, cardiovascular drugs, psychotropics, neuroleptics, antidepressants, antiparkinson drugs, beta blockers, calcium channel blockers, bradykinin inhibitors, ACE-inhibitors, vasodilators, prolactin inhibitors, steroids, hormone antagonists, antihistamines, serotonin antagonists, heparin, chemotherapeutic agents, antineoplastics and growth factors, including but not limited to PDGF, EGF, KGF, IGF-1 and IGF-2, FGF, polynucleotides which encode therapeutic or immunogenic proteins, immunogenic proteins and epitopes thereof for use in vaccines, hormones including peptide hormones such as insulin, proinsulin, growth hormone, GHRH, LHRH, EGF, somatostatin, SNX-111, BNP, insulinotropin, ANP, FSH, LH, PSH and hCG, gonadal steroid hormones (androgens, estrogens and progesterone), thyroid-stimulating hormone, inhibin, cholecystokinin, ACTH, CRF, dynorphins, endorphins, endothelin, fibronectin fragments, galanin, gastrin, insulinotropin, glucagon, GTP-binding protein fragments, guanylin, the leukokinins, magainin, mastoparans, dermaseptin, systemin, neuromedins, neurotensin, pancreastatin, pancreatic polypeptide, substance P, secretin, thymosin, and the like, enzymes, transcription or translation mediators, intermediates in metabolic pathways, immunomodulators, such as any of the various cytokines including interleukin-1, interleukin-2, interleukin 3, interleukin-4, and gamma-interferon, antigens, and adjuvants.

In some preferred embodiments of the invention, the macromolecule is nucleic acid, more preferably a vector construct such as an ELVIS vector, or RNA vector construct. One particular advantage of the present invention is the ability of the microparticles with adsorbed ELVIS vector to generate cell-mediated immune responses in a vertebrate subject. The ability of the antigen/microparticles of the present invention to elicit a cell-mediated immune response against a selected antigen provides a powerful tool against infection by a wide variety of pathogens. Accordingly, the antigen/ microparticles of the present invention can be incorporated into vaccine compositions.

Thus, in addition to a conventional antibody response, the systems herein described can provide for, e.g., the association of the expressed antigens with class I MHC molecules such that an *in vivo* cellular immune response to the antigen of interest can be mounted which stimulates the production of CTLs to allow for future recognition of the antigen. Furthermore, the methods may elicit an antigen specific response by helper T-cells. Accordingly, the methods of the present invention will find use with any macromolecule for which cellular and/or humoral immune responses are desired, preferably antigens derived from viral pathogens that may induce antibodies, T-cell helper epitopes and T-cell cytotoxic epitopes. Such antigens include, but are not limited to, those encoded by human and animal viruses and can correspond to either structural or nonstructural proteins.

The microparticles of the present invention are particularly useful for immunization against intracellular viruses which normally elicit poor immune responses. For example, the present invention will find use for stimulating an immune response against a wide variety of proteins from the herpesvirus family, including proteins derived from herpes simplex virus (HSV) types 1 and 2, such as HSV-1 and HSV-2 glycoproteins gB, gD and gH; antigens derived from varicella zoster virus (VZV), Epstein-Barr virus (EBV) and cytomegalovirus (CMV) including CMV gB and gH; and antigens derived from other human herpesviruses such as HHV6 and HHV7. (See, e.g. Chee et al., *Cytomegaloviruses* (J.K. McDougall, ed., Springer-Verlag 1990) pp. 125-169, for a review of the protein coding content of cytomegalovirus; McGeoch et al., *J. Gen. Virol.* (1988) 69:1531-1574, for a discussion of the various HSV-1 encoded proteins; U.S. PatentNo. 5,171,568 for a discussion of HSV-1 and HSV-2 gB and gD proteins and the genes encoding therefor; Baer et al., *Nature* (1984) 310:207-211, for the identification of protein coding sequences in an EBV genome; and Davison and Scott, *J. Gen. Virol.* (1986) 67:1759-1816, for a review ofVZV.)

Antigens from the hepatitis family of viruses, including hepatitis A virus (HAV), hepatitis B virus (HBV), hepatitis C virus (HCV), the delta hepatitis virus (HDV), hepatitis E virus (HEV) and hepatitis G virus (HGV), can also be conveniently used in the techniques described herein. By way of example, the viral genomic sequence of HCV is known, as are methods for obtaining the sequence. See, e.g., International Publication Nos. WO 89/04669; WO 90/11089; and WO 90/14436. The HCV genome encodes several viral proteins, including E1 (also known as E) and E2 (also known as E2/NSI) and an N-terminal nucleocapsid protein (termed "core") (see, Houghton et al, *Hepatology* (1991) 14:381-388, for a discussion ofHCV proteins, including E1 and E2). Each of these proteins, as well as antigenic fragments thereof, will find use in the present composition and methods.

Similarly, the sequence for the 8-antigen from HDV is known (see, e.g., U.S. Patent No. 5,378,814) and this antigen can also be conveniently used in the present composition and methods. Additionally, antigens derived from HBV, such as the core antigen, the surface antigen, SAg, as well as the presurface sequences, pre-S1 and pre-S2 (formerly called pre-S), as well as combinations of the above, such as SAg/pre-S1, SAg/pre-S2, SAg/pre-S1/pre-S2, and pre-S1/pre-S2, will find use herein. See, e.g., "HBV Vaccines - from the laboratory to license: a case study" in Mackett, M. and Williamson, J.D., *Human Vaccines and Vaccination*, pp. 159-176, for a discussion of HBV structure; and U.S. Patent Nos. 4,722,840, 5,098,704, 5,324,513, incorporated herein by reference in their entireties; Beames et al., *J. Virol.* (1995) 69:6833-6838, Bimbaum et al., *J. Virol.* (1990) 64:3319-3330; and Zhou et al., *J. Virol.* (1991) 65:5457-5464.

Antigens derived from other viruses will also find use in the claimed compositions and methods, such as without limitation, proteins from members of the families Picomaviridae (e.g., polioviruses, etc.); Caliciviridae; Togaviridae (e.g., rubella virus, dengue virus, etc.); Flaviviridae; Coronaviridae; Reoviridae; Birnaviridae; Rhabodoviridae (e.g., rabies virus, etc.); Filoviridae; Paramyxoviridae (e.g., mumps virus, measles virus, respiratory syncytial virus, etc.); Orthomyxoviridae (e.g., influenza virus types A, B and C, etc.); Bunyaviridae; Arenaviridae; Retroviradae (e.g., HTLV-I; HTLV-II; HIV-1 (also known as HTLV-III, LAV, ARV, hTLR, etc.)), including but not limited to antigens from the isolates HIV_{IIIb}, HIV_{SF2}, HIV_{LAV}, HIV_{LAI}, HIV_{MN}); HIV-1_{CM235}, HIV-1_{US4}; HIV-2; simian immunodeficiency virus (SIV) among others. Additionally, antigens may also be derived from human papillomavirus (HPV) and the tick-borne encephalitis viruses. See, e.g. Virology, 3rd Edition (W.K. Joklik ed. 1988); *Fundamental Virology,* 2nd Edition (B.N. Fields and D.M. Knipe, eds. 1991), for a description of these and other viruses.

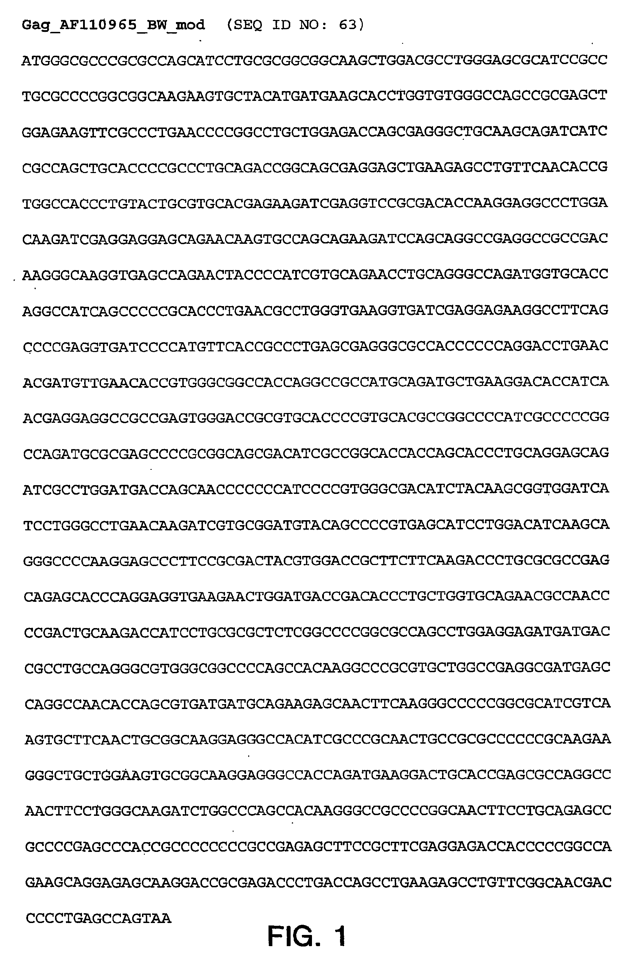

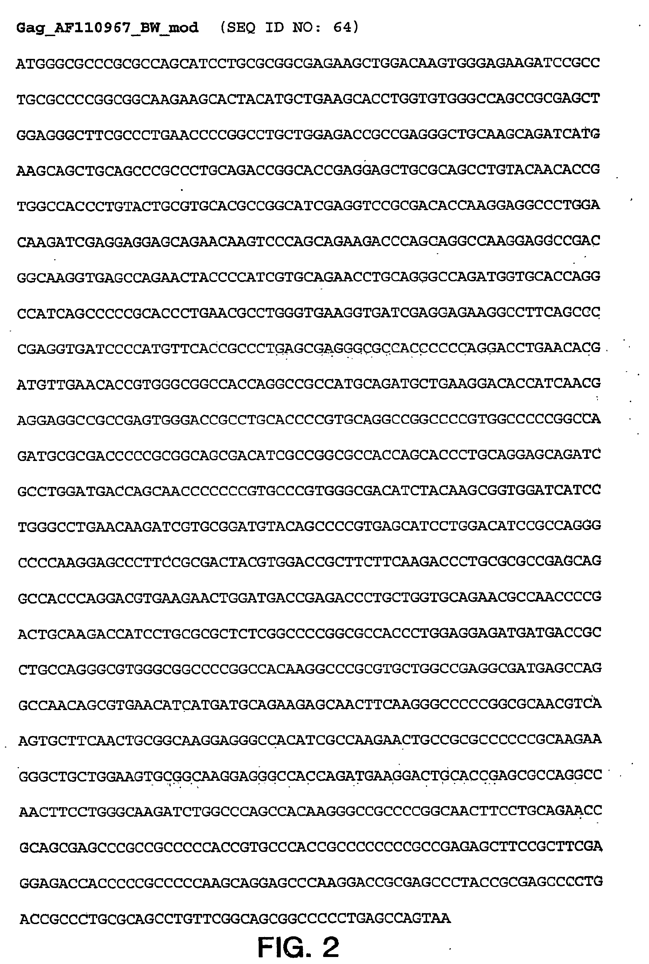

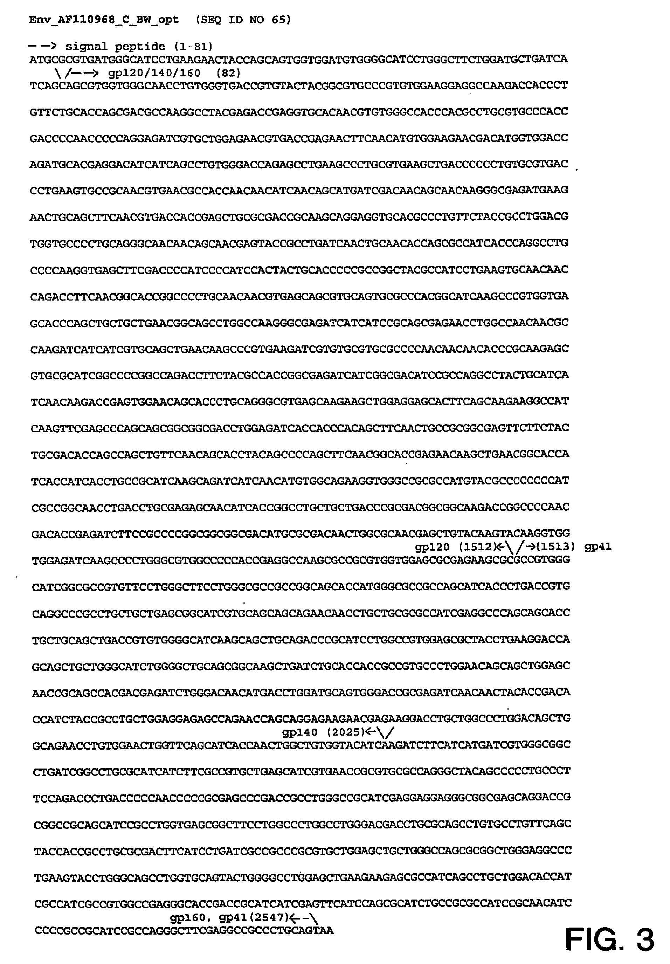

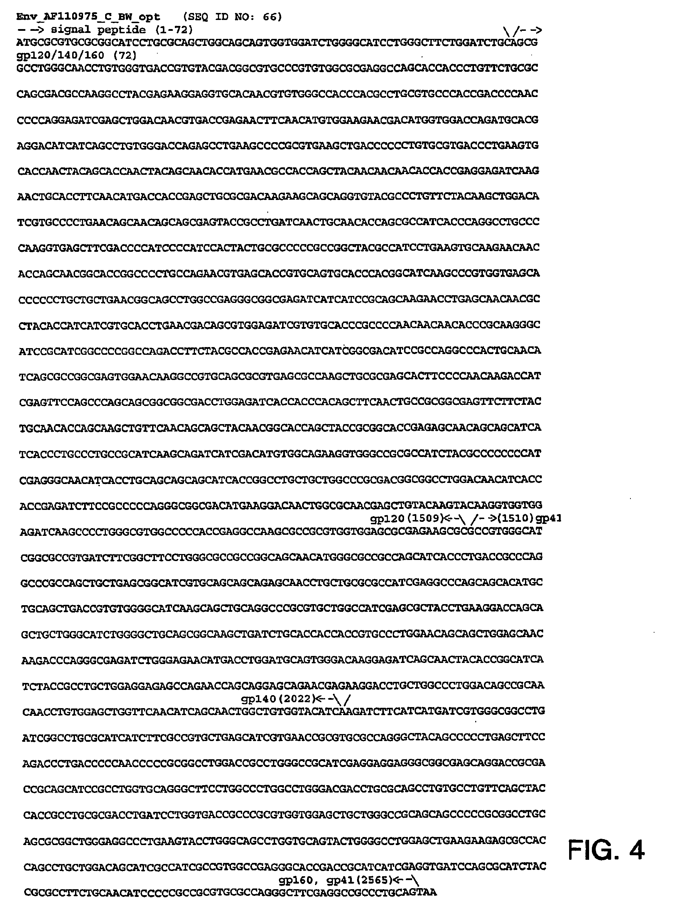

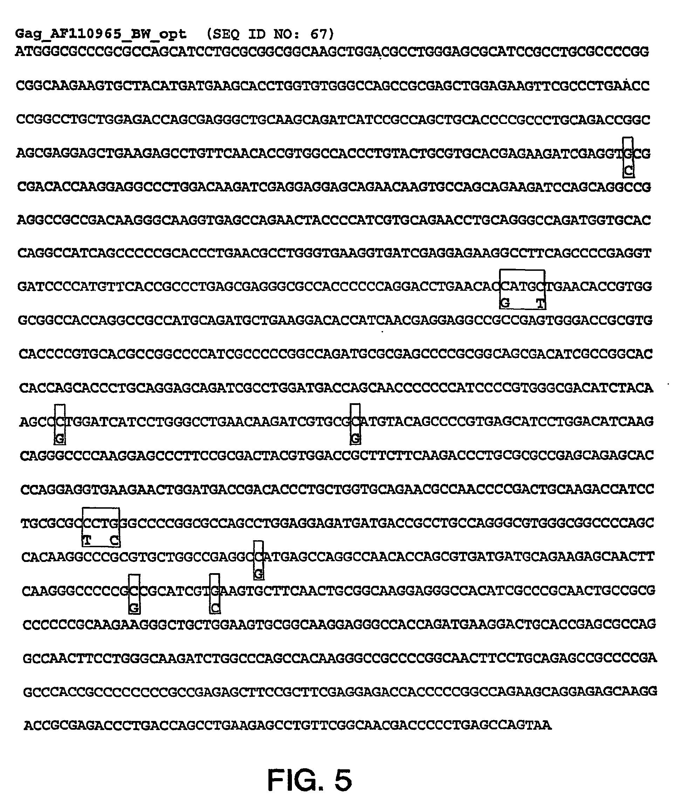

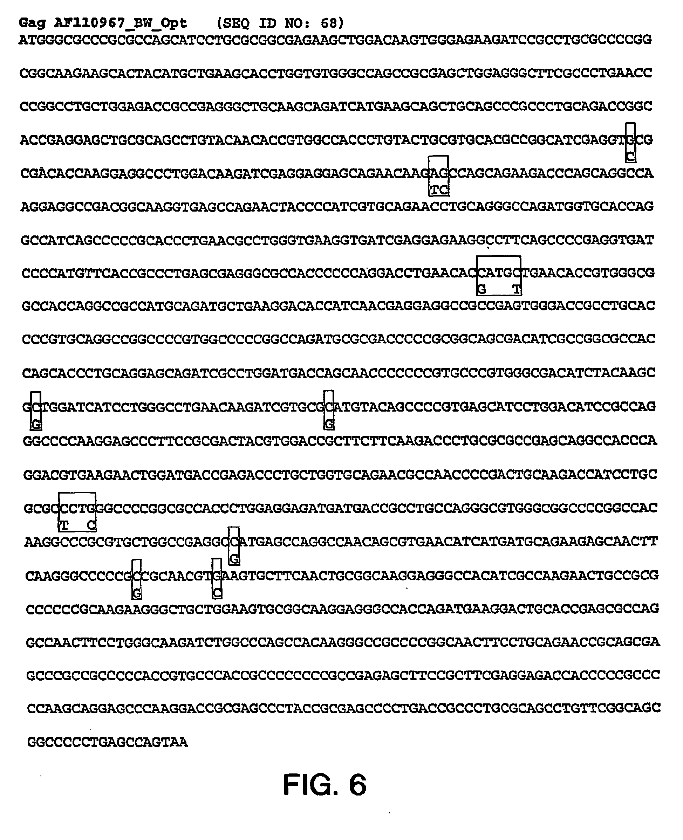

More particularly, the gp120 or gp140 envelope proteins from any of the above HIV isolates, including members of the various genetic subtypes of HIV, are known and reported (see, e.g., Myers et al., Los Alamos Database, Los Alamos National Laboratory, Los Alamos, New Mexico (1992); Myers et al, *Human Retroviruses and Aids, 1990,* Los Alamos, New Mexico: Los Alamos National Laboratory; and Modrow et al, *J*. *Virol.* (1987) 61:570-578, for a comparison of the envelope sequences of a variety of HIV isolates) and antigens derived from any of these isolates will find use in the present methods. Furthermore, the invention is equally applicable to other immunogenic proteins derived from any of the various HIV isolates, including any of the various envelope proteins such as gp160 and gp41, gag antigens such as p24gag and p55gag, as well as proteins derived from the pol and tat regions. Any of these proteins and antigens may also be modified for use in the present invention. For example, Figures 1, 2, 5, and 6 provide DNA sequences encoding modified gag antigens (SEQ ID NOs: 63, 64, 67, and 68), and Figures 3 and 4 provide DNA sequences encoding modified envelope antigens (SEQ ID NOs: 65 and 66).

Influenza virus is another example of a virus for which the present invention will be particularly useful. Specifically, the envelope glycoproteins HA and NA of influenza A are of particular interest for generating an immune response. Numerous HA subtypes of influenza A have been identified (Kawaoka et al., *Virology* (1990) 179:759-767; Webster et al., "Antigenic variation among type A influenza viruses," p. 127-168. In: P. Palese and D.W. Kingsbury (ed.), *Genetics of influenza viruses.* Springer-V erlag, New York). Thus, proteins derived from any of these isolates can also be used in the compositions and methods described herein.

The compositions and methods described herein will also find use with numerous bacterial antigens, such as those derived from organisms that cause diphtheria, cholera, tuberculosis, tetanus, pertussis, meningitis, and other pathogenic states, including, without limitation, *Bordetella pertussis*, *Neisseria meningitides* (A, B, C, Y), *Neisseria gonorrhoeae, Helicobacter pylori*, and *Haemophilus influenza. Hemophilus influenza* type B (HIB), *Helicobacter pylori,* and combinations thereof. Examples of antigens from *Neisseria meningitides* B are disclosed in the following co-owned patent applications: PCT/US99/09346; PCT IB98/01665; and PCT IB99/00103. Examples of parasitic antigens include those derived from organisms causing malaria and Lyme disease.

Additional antigens for use with the invention, some of which are also listed elsewhere in this application, include the following (references are listed immediately below):
- A protein antigen from *N. meningitidis* serogroup B, such as those in Refs. 1 to 7 below.
- an outer-membrane vesicle (OMV) preparation from *N*. *meningitidis* serogroup B, such as those disclosed in Refs. 8, 9, 10, 11 etc. below.
- a saccharide antigen from *N. meningitidis* serogroup A, C, W135 and/or Y, such as the oligosaccharide disclosed in Ref. 12 below from serogroup C (see also Ref. 13).
- a saccharide antigen from *Streptococcus pneumoniae* [e.g. Refs. 14, 15, 16].
- an antigen from *N*. *gonorrhoeae* [e.g., Refs. 1, 2, 3].
- an antigen from *Chlamydia pneumoniae* [e.g., Refs. 17, 18, 19, 20, 21, 22, 23].
- an antigen from *Chlamydia trachomatis* [e.g. 24].
- an antigen from hepatitis A virus, such as inactivated virus [e.g., Refs. 25, 26].
- an antigen from hepatitis B virus, such as the surface and/or core antigens [e.g., Refs. 26, 27].
- an antigen from hepatitis C virus [e.g. Ref. 28].
- an antigen from *Bordetella pertussis,* such as pertussis holotoxin (PT) and filamentous haemaglutinin (FHA) from *B*. *pertussis,* optionally also in combination with pertactin and/or agglutinogens 2 and 3 [e.g., Refs. 29 & 30].
- a diphtheria antigen, such as diphtheria toxoid [e.g., chapter 3 of Ref. 31] e.g. the CRM₁₉₇ mutant [e.g., Ref. 32].
- a tetanus antigen, such as a tetanus toxoid [e.g., chapter 4 of Ref. 31].
- a protein antigen from *Helicobacter pylori* such as CagA [e.g. Ref. 33], VacA [e.g. Ref. 33], NAP [e.g. Ref. 34], HopX [e.g. Ref. 35], HopY [e.g. Ref. 35] and/or urease.
- a saccharide antigen from *Haemophilus influenzae* B [e.g. Ref. 13].
- *an* antigen from *Porphyramonas gingivalis* [e.g. Ref 36].
- polio antigen(s) [e.g. Refs. 37, 38] such as IPV or OPV.
- rabies antigen(s) [e.g. Ref. 39] such as lyophilized inactivated virus [e.g. Ref. 40, Rabavert™).
- measles, mumps and/or rubella antigens [e.g., chapters 9, 10 and 11 of Ref. 31].
- influenza antigen(s) [e.g. chapter 19 of Ref. 31], such as the haemagglutinin and/or neuraminidase surface proteins.
- an antigen from *Moraxella catarrhalis* [e.g., time 41].
- an antigen from *Streptococcus agalactiae* (Group B streptococcus) [e.g. Refs. 42, 43]
- an antigen from *Streptococcus pyogenes* (Group A streptococcus) [e.g. Refs. 43,44, 45].
- an antigen from *Staphylococcus aureus* [e.g. Ref. 46].
- Compositions comprising one or more of these antigens.

Where a saccharide or carbohydrate antigen is used, it is preferably conjugated to a carrier protein in order to enhance immunogenicity [e.g. Refs. 47 to 56]. Preferred carrier proteins are bacterial toxins or toxoids, such as diphtheria or tetanus toxoids. The CRM₁₉₇ diphtheria toxoid is particularly preferred. Other suitable carrier proteins include *N. meningitidis* outer membrane protein [e.g. Ref 57], synthetic peptides [e.g. Refs. 58, 59], heat shock proteins [e.g. Ref. 60], pertussis proteins [e.g. Refs. 61, 62], protein D from *H*. *Influenzae* [e.g. Ref. 63], toxin A or B from C. *difficile* [e.g. Ref. 64], etc. Where a mixture comprises capsular saccharides from both serogroups A and C, it is preferred that the ratio (w/w) of MenA saccharide:MenC saccharide is greater than 1 (e.g. 2:1, 3:1, 4:1, 5:1, 10:1 or higher). Saccharides from different serogroups of *N*. *meningitidis* may be conjugated to the same or different carrier proteins.

Any suitable conjugation reaction can be used, with any suitable linker where necessary. Toxic protein antigens may be detoxified where necessary (e.g. detoxification of pertussis toxin by chemical and/or means [Ref. 30].

Where diphtheria antigen is included in the composition it is preferred also to include tetanus antigen and pertussis antigens. Similarly, where a tetanus antigen is included it is preferred also to include diphtheria and pertussis antigens. Similarly, where a pertussis antigen is included it is preferred also to include diphtheria and tetanus antigens.

It is readily apparent that the subject invention can be used to deliver a wide variety of macromolecules and hence to treat, prevent and/or diagnose a large number of diseases. In some embodiments, the macromolecule/microparticle compositions of the present invention can be used for site-specific targeted delivery. For example, intravenous administration of the macromolecule/microparticle compositions can be used for targeting the lung, liver, spleen, blood circulation, or bone marrow.

The adsorption of macromolecules to the surface of the adsorbent microparticles (or to submicron emulsions of the present invention) occurs via any bonding-interaction mechanism, including, but not limited to, ionic bonding, hydrogen bonding, covalent bonding, Van der Waals bonding, physical entrapment, and bonding through hydrophilic/hydrophobic interactions. Those of ordinary skill in the art may readily select detergents appropriate for the type of macromolecule to be adsorbed.

For example, microparticles manufactured in the presence of charged detergents, such as anionic or cationic detergents, may yield microparticles with a surface having a net negative or a net positive charge, which can adsorb a wide variety of molecules. For example, microparticles manufactured with anionic detergents, such as sodium dodecyl sulfate (SDS), i.e. SDS-PLG microparticles, adsorb positively charged antigens, such as proteins. Similarly, microparticles manufactured with cationic detergents, such as hexadecyltrimethylammonium bromide (CTAB), i.e. CTAB-PLG microparticles, adsorb negatively charged macromolecules, such as DNA. Where the macromolecules to be adsorbed have regions of positive and negative charge, cationic, anionic, nonionic or zwitterioinic detergents may be appropriate.

Biodegradable polymers for manufacturing microparticles for use with the present invention are readily commercially available from, e.g., Boehringer Ingelheim, Germany and Birmingham Polymers, Inc., Birmingham, AL. For example, useful polymers for forming the microparticles herein include homopolymers, copolymers and polymer blends derived from the following:
polybydroxybutyric acid (also known as polyhydroxybutyrate); polyhydroxy valeric acid (also known as polyhydroxyvalerate); polyglycolic acid (PGA) (also known as polyglycolide): polylactic acid (PLA) (also known as polylactide); polydioxanone; polycaprolactone; polyorthoester; and
polyanhydride. More preferred are poly(α-hydroxy acids), such as poly(L-lactide), poly(D,L-lactide) (both known as "PLA" herein), poly(hydoxybuiyrate), copolymers of D,L-lactide and
glycolide, such as poly(D,L-lactide-co-glycolide) (designated as "PLG" or "PLGA" herein) or a copolymer of D,L-lactide and caprolactone. Particularly preferred polymers for use herein are PLA and PLG polymers. These polymers are available in a variety of molecular weights, and the appropriate molecular weight for a given use is readily determined by one of skill in the art. Thus, e.g., for PLA, a suitable molecular weight will be on the order of about 2000 to 5000. For PLG, suitable molecular weights will generally range from about 10,000 to about 200,000, preferably about 15,000 to about 150,000.

If a copolymer such as PLG is used to form the microparticles, a variety of lactide:glycolide ratios will find use herein and the ratio is largely a matter of choice, depending in part on the coadministered macromolecule and the rate of degradation desired. For example, a 50:50 PLG polymer, containing 50% D,L-lactide and 50% glycolide, will provide a fast resorbing copolymer while 75:25 PLG degrades more slowly, and 85:15 and 90:10, even more slowly, due to the increased lactide component. It is readily apparent that a suitable ratio of lactide:glycolide is easily determined by one of skill in the art based, for example, on the nature of the antigen and disorder in question. Moreover, in embodiments of the present invention wherein antigen or adjuvants are entrapped within microparticles, mixtures of microparticles with varying lactide:glycolide ratios will find use herein in order to achieve the desired release kinetics for a given macromolecule and to provide for both a primary and secondary immune response. Degradation rate of the microparticles of the present invention can also be controlled by such factors as polymer molecular weight and polymer crystallinity. PLG copolymers with varying lactide:glycolide ratios and molecular weights are readily available commercially from a number of sources including from Boehringer Ingelheim, Germany and Birmingham Polymers, Inc., Birmingham, AL. These polymers can also be synthesized by simple polycondensation of the lactic acid component using techniques well known in the art, such as described in Tabata et al, *J. Biomed Mater. Res.* (1988) 22:837-858.

Where used, preferred poly(D,L-lactide-co-glycolide) polymers are those having a lactide/glycolide molar ratio ranging from 30:70 to 70:30, more preferably 40:60 to 60:40, and having a molecular weight ranging from 10,000 to 100,000 Daltons, more preferably from 30,000 Daltons to 70,000 Daltons.

The polymer microparticles are prepared using any of several methods well known in the art. For example, in some embodiments, double emulsion/solvent evaporation techniques, such as those described in U.S. Patent No. 3,523,907 and Ogawa et al., *Chem*. *Pharm*. *Bull.* (1988) 36:1095-1103, can be used herein to make the microparticles. These techniques involve the formation of a primary emulsion consisting of droplets of polymer solution, which is subsequently mixed with a continuous aqueous phase containing a particle stabilizer/ surfactant.

Alternatively, a water-in-oil-in-water (w/o/w) solvent evaporation system can be used to form the microparticles, as described by O'Hagan et al., *Vaccine* (1993) 11:965-969, PCT/US99/17308 (WO 00/06123) to O'Hagan et al. and Jeffery et al., Pharm. Res. (1993) 10:362. In this technique, the particular polymer is typically combined with an organic solvent, such as ethyl acetate, dimethylchloride (also called methylene chloride and dichloromethane), acetonitrile, acetone, chloroform, and the like. The polymer will be provided in about a 1-30%, preferably about a 2-15%, more preferably about a 3-10% and most preferably, about a 4-6% solution, in organic solvent. The polymer solution is then combined with an aqueous solution and emulsified to form an o/w emulsion. The aqueous solution can be, for example, deionized water, normal saline, or a buffered solution such as phosphate-buffered saline (PBS) or a sodium citrate/ethylenediaminetetraacetic acid (sodium citrate/ETDA) buffer solution. Preferably, the volume ratio of polymer solution to aqueous liquid ranges from about 5:1 to about 20:1, more preferably about 10:1. Emulsification is conducted using any equipment appropriate for this task, and is typically a high-shear device such as, e.g., an homogenizer.

A volume of the o/w emulsion is then optionally preferably combined with a larger volume of an aqueous solution, which preferably contains a cationic, anionic, or nonionic detergent. The volume ratio of aqueous solution to o/w emulsion typically ranges from about 2:1 to 10:1, more typically about 4:1. Examples of anionic, cationic and nonionic detergents appropriate for the practice of the invention are listed above and include SDS, CTAB and PVA, respectively. Certain macromolecules may adsorb more readily to microparticles having a combination of stabilizers and/or detergents, for example, a combination of PVA and DOTAP. Moreover, in some instances, it may be desirable to add detergent to the above organic solution. Where a nonionic detergent such as PVA an emulsion stabilizer is used, it is typically provided in about a 2-15% solution, more typically about a 4-10% solution. Where a cationic or anionic detergent is used, it is typically provided in about a 0.05-5% solution, more typically about a 0.25-1% solution. Generally, a weight to weight detergent to polymer ratio in the range of from about 0.00001:1 to about 0.5:1 will be used, more preferably from about 0.0001:1 1 to about 0.5:1, more preferably from about 0.001:1 to about 0.5:1, and even more preferably from about 0.005:1 to about 0.5:1.

The mixture is then homogenized to produce a stable w/o/w double emulsion. Organic solvents are then evaporated. The formulation parameters can be manipulated to allow the preparation of small microparticles on the order of 0.05 µm (50 mn) to larger microparticles 50 µm or even larger. See, e.g., Jeffery et al., *Pharm. Res.* (1993) 10:362-368; McGee et aL, *J Microencap.* (1996). For example, reduced agitation results in larger microparticles, as does an increase in internal phase volume. Small particles are produced by low aqueous phase volumes with high concentrations of emulsion stabilizers.

Additional information can be found in U.S. Application Serial No. , Attorney Docket Nos. PP16502.002, entitled "Microparticles with Adsorbed Macromolecules" filed September 28, 2001.

The formulation parameters can be manipulated to allow the preparation of small microparticles on the order of 0.05 µm (50 nm) to larger microparticles 50 µm or even larger. See, e.g., Jeffery et al., *Pharm. Res.* (1993) 10:362-368; McGee et al., *J*. *Microencap.* (1996). For example, reduced agitation results in larger microparticles, as does an increase in internal phase volume. Small particles are produced by low aqueous phase volumes with high concentrations of emulsion stabilizers.

Microparticles can also be formed using spray-drying and coacervation as described in, e.g., Thomasin et al., *J. Controlled Release* (1996) 41:131; U.S. Patent No. 2,800,457; Masters, K. (1976) *Spray Drying* 2nd Ed. Wiley, New York; air-suspension coating techniques, such as pan coating and Wurster coating, as described by Hall et al., (1980) The "Wurster Process" in *Controlled Release Technologies: Methods, Theory, and Applications* (A.F. Kydonieus, ed.), Vol. 2, pp. 133-154 CRC Press, Boca Raton, Florida and Deasy, P.B., *Crit. Rev. Ther. Drug Carrier Syst.* (1988) S(2):99-139; and ionic gelation as described by, e.g., Lim et al., *Science* (1980) 210:908-910.

Particle size can be determined by, e.g., laser light scattering, using for example, a spectrometer incorporating a helium-neon laser. Generally, particle size is determined at room temperature and involves multiple analyses of the sample in question (e.g., 5-10 times) to yield an average value for the particle diameter. Particle size is also readily determined using scanning electron microscopy (SEM).

Alternative embodiments of the present invention utilize microparticle preparations comprising a submicron emulsion, which preferably includes an ionic surfactant. For instance, MF59 or others may be used as the base oil-containing submicron emulsion, while ionic surfactants may include, but are not limited to, Dioleoyl-3-Trimethylammonium-Propane (DOTAP), Dioleoyl-sn-Glycero-3-Ethylphosphocholine (DEPC) and dioleoyl-phosphatidic acid (DPA), each of which are soluble in squalene. Prototypic ionic emulsions may be formulated by dissolving each of the detergents in squalene/10% Span 85 at concentrations ranging from 4-52 mg/ml squalene. The squalene/surfactant mixtures may be emulsified with 0.5% Tween 80/H₂O at 5ml squalene/100 ml H₂O. A pre-emulsion may be formed by homogenization with a Silverson homogenizer (5 minutes, 5000 RPM) and final emulsions may be made by microfluidization (~10,000psi, 5 passes, Microfluidizer 110S). Additional discussion concerning submicron emulsions can be found *infra.*

Following preparation, microparticles can be stored as is or freeze-dried for future use. Typically, in order to adsorb macromolecules to the microparticles, the microparticle preparation is simply mixed with the macromolecule of interest and the resulting formulation can again be lyophilized prior to use. Generally, macromolecules are added to the microparticles to yield microparticles with adsorbed macromolecules having a weight to weight ratio of from about 0.0001:1 to 0.25:1 macromolecules to microparticles, preferably, 0.001:1 to 0.1, more preferably 0.01 to 0.05. Macromolecule content of the microparticles can be determined using standard techniques.

As noted above, macromolecules for use in connection with the present invention include proteins, preferably antigen molecules, and nucleic acids, preferably vector constructs capable of expressing a nucleic acid sequence, such as CMV-based vectors, ELVIS vectors or RNA vector constructs.

The polymer microparticles of the present invention may have macromolecules entrapped or encapsulated within them, as well as having macromolecules adsorbed thereon. Thus, for example, one of skill in the art may prepare in accordance with the invention microparticles having encapsulated adjuvants with ELVIS vector adsorbed thereon, or microparticles having encapsulated antigen with RNA vector construct adsorbed thereon. The invention contemplates a variety of combinations of nucleic acid macromolecules adsorbed on and entrapped within microparticles, along with other nucleic acids as well as other antigenic molecules. In some preferred embodiments, the microparticles of the invention have ELVIS vectors or RNA vector constructs adsorbed thereon.

Additionally, any of the embodiments of the microparticles of the invention may be delivered in conjunction with electroporation.

Once the macromolecule-adsorbed microparticles and/or submicron emulsion microparticles are produced, they are formulated, along with any desired adjuvants, into pharmaceutical compositions including vaccines, to treat, prevent and/or diagnose a wide variety of disorders, as described above. The compositions will generally include one or more pharmaceutically acceptable excipients. For example, vehicles such as water, saline, glycerol, polyethylene-glycol hyaluronic acid, ethanol, etc. may be used. Other excipients such as wetting or emulsifying agents, biological buffering substances, and the like, may be present in such vehicles. A biological buffer can be virtually any solution which is pharmacologically acceptable and which provides the formulation with the desired pH, i.e., a pH in the physiological range. Examples of buffer solutions include saline, phosphate buffered saline, Tris buffered saline, Hank's buffered saline, and the like. Other excipients known in the art can also be introduced into the final dosage form, including binders, disiategrants, fillers (diluents), lubricants, glidants (flow enhancers), compression aids, colors, sweeteners, preservatives, suspensing/dispersing agents, film formers/coatings, flavors and printing inks.

The compositions of the invention will comprise a therapeutically effective amount of one or more macromolecules of interest. That is, an amount of macromolecule/microparticle will be included in the compositions, which will cause the subject to produce a sufficient response, in order to prevent, reduce, eliminate or diagnose symptoms. The exact amount necessary will vary, depending on the subject being treated; the age and general condition of the subject to be treated; the severity of the condition being treated; in the case of an immunological response, the capacity of the subject's immune system to synthesize antibodies; the degree of protection desired and the particular antigen selected and its mode of administration, among other factors. An appropriate effective amount can be readily determined by one of skill in the art. Thus, a therapeutically effective amount will fall in a relatively broad range that can be determined through routine trials. For example, for purposes of the present invention, where the macromolecule is a polynucleotide, an effective dose will typically range from about 1 ng to about 10 mg, more preferably from about 10 ng to about 1 mg, and most preferably about 100 µg to about 1 mg of the macromolecule delivered per dose; where the macromolecule is an antigen, an effective dose will typically range from about 1 µg to about 100 mg, more preferably from about 10 µg to about 1 mg, and most preferably about 50 µg to about 1 mg of the macromolecule delivered per dose.

Once formulated, the compositions of the invention can be administered parenterally, e.g., by injection. The compositions can be injected either subcutaneously, intraperitoneally, intravenously or intramuscularly. Other modes of administration include nasal, mucosal, rectal, vaginal, oral and pulmonary administration, suppositories, and transdermal or transcutaneous applications. Dosage treatment may be a single dose schedule or a multiple dose schedule. A multiple dose schedule is one in which a primary course of administration may be with 1-10 separate doses, followed by other doses given at subsequent time intervals, chosen to maintain and/or reinforce the therapeutic response, for example at 1-4 months for a second dose, and if needed, a subsequent dose(s) after several months.

In certain embodiments of the invention, a series of one or more injections of a vector construct (which comprises a heterologous nucleic acid sequence encoding an antigen) is followed by a series of one or more injections of antigen (also referred to herein as "boosts"). As a specific example, the vector construct may be administered in three injections: (a) at a time of initial administration, (b) at a time period ranging 1-8 weeks from the initial administration, and (c) at a time period ranging 4-32 weeks from the initial administration, while the antigen may be administered in two injections: (a) at a time period ranging from 8-50 weeks from the initial administration and (b) at a time period ranging from 8-100 weeks from the initial administration.

The dosage regimen will also, at least in part, be determined by the need of the subject and be dependent on the judgment of the practitioner.

Furthermore, if prevention of disease is desired, the microparticles with adsorbed vector constructs are generally administered prior to primary infection with the pathogen of interest. If treatment of disease (other than prevention) is desired, e.g., the reduction of symptoms or recurrences, the microparticles with adsorbed vector constructs are generally administered subsequent to primary infection.

### 2. Oil Droplet Emulsions

In other embodiments of the present invention, an oil droplet emulsion (particularly, a submicron emulsion) is prepared comprising a metabolizable oil and an emulsifying agent. Molecules such as an oligonucleotide comprising at least one CpG motif may be combined with the oil droplet emulsion to form an adjuvant.

The oil droplet emulsion preferably comprises a metabolizable oil and an emulsifying agent, wherein the oil and the emulsifying agent are present in the form of an oil-in-water emulsion having oil droplets substantially all of which are less than one micron in diameter. Submicron emulsions, with droplets in this preferred size range, show a surprising superiority over other emulsions containing oil and emulsifying agents in which the oil droplets are significantly larger than those provided by the present invention. In preferred embodiments, the emulsion is positively charged as a result of a cationic detergent being used as the emulsifying agent or, alternatively, contains a cationic detergent in addition to the emulsifying agent. This allows for the adsorption of nucleotide antigenic molecules, such as CpG oligonucleotides or vector constructs.. Alternatively, the use of an anionic detergent allows for the adsorption of molecules such as proteins.

Although individual components of the submicron emulsion compositions of the present invention are generally known, such compositions have not been combined in the same manner. Accordingly, the individual components, although described below both generally and in some detail for preferred embodiments, are well known in the art, and the terms used herein, such as metabolizable oil, emulsifying agent, immunostimulating agent, muramyl peptide, and lipophilic muramyl peptide, are sufficiently well known to describe these compounds to one skilled in the art without further description.

One component of these compositions is a metabolizable, non-toxic oil, preferably one of about 6 to about 30 carbon atoms including, but not limited to, alkanes, alkenes, alkynes, and their corresponding acids and alcohols, the ethers and esters thereof, and mixtures thereof The oil can be any vegetable oil, fish oil, animal oil or synthetically prepared oil which can be metabolized by the body of the host animal to which the adjuvant will be administered and which is not toxic to the subject. The host animal is typically a mammal, and preferably a human. Mineral oil and similar toxic petroleum distillate oils are expressly excluded from this invention.

The oil component of this invention can also be any long chain alkane, alkene or alkyne, or an acid or alcohol derivative thereof either as the free acid, its salt or an ester such as a mono-, or di- or triester, such as the triglycerides and esters of 1,2-propanediol or similar poly-hydroxy alcohols. Alcohols can be acylated employing amino- or poly-functional acid, for example acetic acid, propanoic acid, citric acid or the like. Ethers derived from long chain alcohols which are oils and meet the other criteria set forth herein can also be used.

The individual alkane, alkene or alkyne moiety and its acid or alcohol derivatives will generally have about 6 to about 30 carbon atoms. The moiety can have a straight or branched chain structure. It can be fully saturated or have one or more double or triple bonds. Where mono or poly ester- or ether-based oils are employed, the limitation of about 6 to about 30 carbons applies to the individual fatty acid or fatty alcohol moieties, not the total carbon count.

Any metabolizable oil, particularly from an animal, fish or vegetable source, can be used herein. It is essential that the oil be metabolized by the host to which it is administered, otherwise the oil component can cause abscesses, granulomas or even carcinomas, or (when used in veterinary practice) can make the meat of vaccinated birds and animals unacceptable for human consumption due to the deleterious effect the unmetabolized oil can have on the consumer.

For a detailed description of such submicron emulsions, see International Publication No. WO 90/14837, and commonly owned International Patent Application PCT/US00/03331.

The oil component of these adjuvants and immunogenic compositions will be present in an amount from about 0.5% to about 20% by volume but preferably no more than about 15%, especially in an amount of about 1% to about 12%. It is most preferred to use from about 1% to about 4% oil.

The aqueous portion of these submicron emulsion compositions is preferably buffered saline or, more preferably, unadulterated water. Because these compositions are intended for parenteral administration, it is preferable to make up final buffered solutions used as immunogenic compositions so that the tonicity, i.e., osmolality, is essentially the same as normal physiological fluids in order to prevent post-administration swelling or rapid absorption of the composition because of differential ion concentrations between the composition and physiological fluids. It is also preferable to buffer the saline in order to maintain pH compatible with normal physiological conditions. Also, in certain instances, it can be necessary to maintain the pH at a particular level in order to ensure the stability of certain composition components such as the glycopeptides.

Any physiologically acceptable buffer can be used herein, but phosphate buffers are preferred. Other acceptable buffers such acetate, tris, bicarbonate, carbonate, or the like can be used as substitutes for phosphate buffers. The pH of the aqueous component will preferably be between about 6.0-8.0.

When the submicron emulsion is initially prepared, however, unadulterated water is preferred as the aqueous component of the emulsion. Increasing the salt concentration makes it more difficult to achieve the desired small droplet size. When the final immunogenic compositions is prepared from the adjuvant, the antigenic material can be added in a buffer at an appropriate osmolality to provide the desired immunogenic composition.

The quantity of the aqueous component employed in these compositions will be that amount necessary to bring the value of the composition to unity. That is, a quantity of aqueous component sufficient to make 100% will be mixed, with the other components listed above, in order to bring the compositions to volume.

A substantial number of emulsifying and suspending agents are generally used in the pharmaceutical sciences. These include naturally derived materials such as gums from trees, vegetable protein, sugar-based polymers such as alginates and cellulose, and the like. Certain oxypolymers or polymers having a hydroxide or other hydrophilic substituent on the carbon backbone have surfactant activity, for example, povidone, polyvinyl alcohol, and glycol ether-based mono- and poly-functional compounds. Long chain fatty-acid-derived compounds form a third substantial group of emulsifying and suspending agents which could be used in this invention. Any of the foregoing surfactants are useful so long as they are non-toxic.

Specific examples of suitable emulsifying agents (also referred to as surfactants or detergents) which can be used in accordance with the present invention are disclosed in commonly owned International patent application PCT/US00/0331. Surfactants are generally divided into four basic types: anionic, cationic, zwitterionic, and nonionic. Examples of anionic detergents include, but are not limited to, alginic acid, caprylic acid, cholic acid, 1-decanesulfonic acid, deoxycholic acid, 1-dodecanesulfonic acid, N-lauroylsarcosine, and taurocholic acid, and the like. Cationic detergents include, but are not limited to, cetrimide (hexadecyltrimethylammonium bromide, or CTAB), benzalkonium chloride, dimethyl dioctodecyl ammonium (DDA) bromide, DOTAP, dodecyltrimethylammonium bromide, benzyldimethylhexadecyl ammonium chloride, cetylpyridinium chloride, methylbenzethonium chloride, and 4-picoline dodecyl sulfate, and the like. Examples of zwitterionic detergents include, but are not limited to, 3-[(3-cholamidopropyl)-dimethylammonio]-1-propanesulfonate (commonly abbreviated CHAPS), 3-[(cholamidopropyl)-dimethylammonio]-2-hydroxy-1-propanesulfonate (generally abbreviated CHAPSO) N-dodecyl-N,N-dimethyl-3-ammonio-1-propanesulfonate, and lyso-α-phosphatidylcholine, and the like. Examples of nonionic detergents include, but are not limited to, decanoyl-N-methylglucamide, diethylene glycol monopentyl ether, n-dodecyl β-D-glucopyranoside, ethylene oxide condensates of fatty alcohols (e.g., sold under the trade name Lubrol), polyoxyethylene ethers of fatty acids (particularly C₁₂-C₂₀ fatty acids), polyoxyethylene sorbitan fatty acid ethers (e.g., sold under the trade name Tween), and sorbitan fatty acid ethers (e.g., sold under the trade name Span), and the like.

A particularly useful group of surfactants are the sorbitan-based non-ionic surfactants, such as the commercially available SPAN® or ARLACEL®, usually with a letter or number designation which distinguishes between the various mono-, di- and triester substituted sorbitans. A related group of surfactants comprises polyoxyethylene sorbitan monoesters and polyoxyethylene sorbitan triesters, commercially available under the mark TWEEN®. The TWEEN® surfactants can be combined with a related sorbitan monoester or triester surfactants to promote emulsion stability.

The size of the oil droplets can be varied by changing the ratio of detergent to oil (increasing the ratio decreases droplet size, operating pressure (increasing operating pressure reduces droplet size), temperature (increasing temperature decreases droplet size), and adding an amphipathic immunostimulating agent (adding such agents decreases droplet size). Actual droplet size will vary with the particular detergent, oil, and immunostimulating agent (if any) and with the particular operating conditions selected. Droplet size can be verified by use of sizing instruments, such as the commercial Sub-Micron Particle Analyzer (Model N4MD) manufactured by the Coulter Corporation, and the parameters can be varied using the guidelines set forth above until substantially all droplets are less than 1 micron in diameter, preferably less than 0.8 microns in diameter, and most preferably less than 0.5 microns in diameter. By substantially all is meant at least about 80% (by number), preferably at least about 90%, more preferably at least about 95%, and most preferably at least about 98%. The particle size distribution is typically Gaussian, so that the average diameter is smaller than the stated limits.

A preferred oil droplet emulsion is MF59. MF59 can be made according to the procedures described in, for example, Ott *et al., Vaccine Design: The Subunit And Aduvant Approach, 1995,* MF. Powell and M.J. Newman, Eds., Plenum Press, New York, p. 277-296; Singh *et al., Vaccine,* 1998, 76,1822-1827; Ott *et al., Vaccine, 1995,* 13, 1557-1562; and Valensi *et al, J. Immunol.,* **1994,** 153, 4029-39, the disclosures of which are incorporated herein by reference in their entirety.

Other oil droplet emulsions include, for example, SAF, containing 10% Squalane, 0.4% Tween 80, 5% pluronic-blocked polymer L121, and thr-MDP either microfluidized into a submicron emulsion or vortexed to generate a larger particle size emulsion, and Ribi® adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (DetoxJ) (for a further discussion of suitable submicron oil-in-water emulsions for use herein, see commonly owned, patent application no. 09/015,736, filed on January 29, 1998).

After preparing the microparticles of the invention, whether of the polymer type or the submicron emulsion type, macromolecules such as polypeptides and vector constructs may be adsorbed thereto as previously discussed. The submicron emulsion microparticles of the present invention may also have macromolecules entrapped or encapsulated within them, as well as having macromolecules adsorbed thereon. Thus, for example, one of skill in the art may prepare in accordance with the invention microparticles having encapsulated adjuvants with ELVIS vector adsorbed thereon,. or microparticles having encapsulated antigen with RNA vector construct adsorbed thereon. The invention contemplates a variety of combinations of nucleic acid macromolecules adsorbed on and entrapped within microparticles, along with other nucleic acids as well as other antigenic molecules. Preferably, the microparticles of the invention have ELVIS vectors or RNA vector constructs adsorbed thereon Additionally, any of the embodiments of the microparticles of the invention may be delivered in conjunction with electroporation.

### 3. ELVIS vectors

ELVIS vectors are Eukaryotic Layered Vector Initiation Systems, which are generally described in U.S. Patents 5,814,482 and 6,015,686, cited above, as well as in International Patent Applications WO 97/38087 and WO 99/18226. In one embodiment, an ELVIS vector is derived from the genome of an alphavirus, more preferably from Sindbis virus (SIN), Semliki Forest virus (SFV), Venezuelan equine encephalitis virus (VEE); or Ross River virus (RRV). The alphavirus is an RNA virus of approximately 11-12 kb in length, which contains a 5' cap and a 3' polyadenylate tail. The mature infectious virus is composed of the genomic RNA enveloped by the nucleocapsid and envelope proteins. Alphavirus infection ofhost cells occurs by a receptor specific event and culminates in release of genomic RNA into the cytoplasm. During viral replication, the viral-encoded envelope glycoproteins E1 and E2 are synthesized and embedded in the host cell membrane, through which progeny virions bud and release to the outside of the host cell.

Replication of the viral genome begins with the genomic RNA strand serving as the template for synthesis of a complementary negative RNA strand. The negative RNA strand then serves as a template for full-length genomic RNA, and for an internally initiated positive-strand subgenomic RNA. The nonstructural proteins are translated from the genomic strand, while the structural proteins are translated from the subgenomic strand. All the viral genes are expressed first as polyproteins, then post-translationally processed into individual proteins by proteolytic cleavage.

An alphavirus vector replicon may be built by replacing certain portions of the viral genome (e.g., structural protein genes) with a selected heterologous nucleic acid sequence. Thus, in certain embodiments, an alphavirus replicon vector may comprise a 5' sequence capable of initiating transcription of an alphavirus, a nucleotide sequence encoding the alphavirus nonstructural proteins, an alphaviral junction region promoter, an alphavirus RNA polymerase recognition site, and a 3' polyadenylate tract. Additionally, the alphavirus vector replicon may be contained as a cDNA copy within an alphavirus vector construct. Such vector constructs typically comprise a 5' promoter capable of initiating synthesis of RNA from cDNA positioned upstream and operably associated with the vector cDNA, such that transcription produces the vector replicon RNA The vector construct also may contain and a 3' sequence controlling transcription termination. A heterologous nucleic acid sequence may be present upstream or downstream of the viral junction region.

The ELVIS vector capitalizes on the mechanism of RNA virus replication to achieve delivery of a heterologous nucleotide sequence of interest by using a double-layered approach (for example, based on the above-described alphavirus vector construct). In general, an ELVIS vector provides a layered expression system capable of amplifying the amount of RNA encoding the gene product of interest because the first layer initiates transcription of a second layer. Thus, a typical ELVIS vector comprises a 5' promoter capable of initiating synthesis of RNA from cDNA, a cDNA complement of a construct capable of autonomous replication in a cell, and which construct is also capable of expressing a heterologous nucleic acid sequence, and a 3' sequence controlling transcription termination. The construct capable of autonomous replication and expression of the selected nucleic acid sequence may be an alphavirus vector construct. Thus, the first layer of the DNA ELVIS vector transcribes the RNA alphavirus vector construct, from which expression of the selected heterologous nucleic acid sequence is achieved.

An alphavirus-based ELVIS vector may be constructed by first preparing a cDNA complementary to an alphavirus genome. The cDNA corresponding to the genomic RNA is then deleted of sequences encoding one or more viral structural proteins which then may be replaced with heterologous DNA encoding the gene-product of interest, thereby preventing packaging of mature virus and enabling amplification of the heterologous sequence. The modified cDNA containing the heterologous sequence is then inserted within the first layer of the ELVIS vector. Upon entry into the cell and nucleus, the ELVIS vector will be transcribed and the resulting mRNA molecules, which are RNA vectors capable of self-replication, will begin to replicate and translate polypeptides, including the heterologous gene of interest.

While a typical Sindbis-derived alphavirus vector construct is preferred, other alphavirus species may be readily used according to the teachings provided herein. Alternatively, vectors derived from any RNA virus may be utilized, particularly those from positive-stranded viruses.

The construction of an ELVIS vector, in general, is described in U.S. Patents 5,814,482 and 6,015,686. Briefly, RNA is obtained from an RNA virus, then cDNA is synthesized by PCR amplification using appropriate primers for particular genes or portions of the RNA virus, which primers may also contain additional restriction sites as necessary. The cDNA fragments are then cloned into a plasmid and transformed into an appropriate host such as *E*. *coli*. Positive colonies are grown for plasmid purification, and then plasmids are assembled into the desired ELVIS vector with a portion having heterologous DNA such as a reporter gene (e.g., GFP) or a desired gene coding for an antigen. Example 3 below describes a particular preferred ELVIS vector (pSINCP) used in accordance with the instant invention.

### 4. RNA and pCMV vector constructs

In other embodiments of the present invention, an RNA vector construct or RNA replicon vector is used directly, without the requirement for introduction of DNA into a cell and transport to the nucleus where transcription would occur. By using the RNA vector for direct delivery into the cytoplasm of the host cell, autonomously replication and translation of the heterologous nucleic acid sequence occurs efficiently. In this embodiment, the RNA vector construct or RNA replicon vector is obtained by in vitro transcription from a DNA-based vector construct. Preferably, the RNA vector construct or RNA replicon vector is derived from the genome of an alphavirus, more preferably from Sindbis virus (SIN), Semliki Forest virus (SFV), Venezuelan equine encephalitis virus (VEE), or Ross River virus (RRV). In other embodiments, the RNA vector construct is derived from a virus other than an alphavirus. Preferably, such other viruses used for the derivation of RNA vector constructs are positive-stranded RNA viruses, and more preferably they are picomaviruses, flaviviruses, rubiviruses, or coronaviruses. Compositions and methods for in vitro transcription of alphavirus-based RNA vectors is provided in detail elsewhere (see U.S. Patent 5,842,723 and Polo et al., 1999, PNAS 96:4598-603). The RNA vector is then adsorbed to a microparticle of the invention for delivery as detailed herein. While a typical alphavirus RNA vector from SIN, SFV, VEE or RRV is preferred, similar vectors derived from other alphavirus species may be readily substituted.

In other embodiments of the present invention, pCMV vector constructs are used. Such vector constructs are well known in the art. A particularly preferred pCMV vector contains the immediate-early enhancer/promoter of CMV and a bovine growth hormone terminator. It is described in detail in Chapman, B. S., et al. 1991. "Effect of intron A from human cytomegalovirus (Towne) immediate-early gene on heterologous expression in mammalian cells." Nucleic Acids Res. 19:3979-86.

### 5. Adjuvants

Adjuvants may optionally be used to enhance the effectiveness of the pharmaceutical compositions, with Th1 stimulating adjuvants being particularly preferred. The adjuvants may be administered concurrently with the microparticles of the present invention, e.g., in the same composition or in separate compositions. Alternatively, an adjuvant may be administered prior or subsequent to the microparticle compositions of the present invention. In another embodiment, the adjuvant, such as an immunological adjuvant, may be encapsulated in the microparticle. Adjuvants, just as any macromolecules, may be encapsulated within the microparticles using any of the several methods known in the art. See, e.g., U. S. Patent No. 3,523,907; Ogawa et al, *Chem Pharm. Bull.* (1988) 36:1095-1103; O'Hagan et al., *Vaccine* (1993) 11:965-969 and Jefferey et al*., Pharm. Res.* (1993) 10:362. Alternatively, adjuvants may be adsorbed on the microparticle as described above for any macromolecule. Alternatively, adjuvants may comprise the oil droplet emulsions of the present invention.

Immunological adjuvants include, but are not limited to: (1) aluminum salts (alum), such as aluminum hydroxide, aluminum phosphate, aluminum sulfate, etc.; (2) other oil-in water emulsion formulations (with or without other specific immunostimulating agents such as muramyl peptides (see below) or bacterial cell wall components), such as for example (a) MF59 (International Publication No. WO90/14837; Chapter 10 in *Vaccine design: the subunit an adjuvant approach*, eds. Powell & Newman, Plenum Press 1995), containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, MA), (b) SAF, containing 10% Squalane, 0.4% Tween 80, 5% pluronic-blocked polymer L121, and thr-MDP (see below) either microfluidized into a submicron emulsion or vortexed to generate a larger particle size emulsion, and (c) Ribi™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™) (for a further discussion of suitable submicron oil-in-water emulsions for use herein, see commonly owned, patent application no. 09/015,736, filed on January 29, 1998); (3) saponin adjuvants, such as Quil A, or QS21 (e.g., Stimulon™ (Cambridge Bioscience, Worcester, MA)) may be used or particle generated therefrom such as ISCOMs (immunostimulating complexes), which ICOMS may be devoid of additional detergent e.g., WO00/07621; (4) Complete Freunds Adjuvant (CFA) and Incomplete Freunds Adjuvant (IFA); (5) cytokines, such as interleukins (e.g. IL-1, IL-2, IL-4, IL-5, IL-6, IL-7, IL-12 (WO99/44636), etc.), interferons (e.g. gamma interferon), macrophage colony stimulating factor (M-CSF), tumor necrosis factor (TNF), etc.; (6) monophosphoryl lipid A (MPL) or 3-O-deacylated MPL (3dMPL) e.g. GB-2220221, EP-A-0689454, optionally in the substantial absence of alum when used with pneumococcal saccharides e.g. WO00/56358; (7) combinations of 3dMPL with, for example, QS21 and/or oil-in-water emulsions, e.g., EP-A 0835318, EP-A-0735898, EP-A 0761231; (8) oligonucleotides comprising CpG motifs (Roman et al., *Nat. Med.,* 1997, 3, 849-854; Weiner et al., *PNAS USA,* 1997, 94, 10833-10837; Davis et al., *J. Immunol.* 1988, 160, 870-876; Chu et al., *J*. *Exp. Med.,* 1997, 186, 1623-1631; Lipford et al., *Eur. J. Immunol.* 1997, 27, 2340-2344; Moldoveanu et al., *Vaccine,* 1988, 16, 1216-1224, Krieg et al., *Nature,* 1995, 374, 546-549; Klinman et al., *PNAS USA,* 1996, 93, 2879-2883: Ballas et al., *J*. *Immunol.,* 1996, 157, 1840-1845; Cowdery et al., *J. Immunol.,* 1996, 156, 4570-4575; Halpern et al, *Cell. Immunol.,* 1996, 167, 72-78; Yamamoto et al., *Jpn. J. Cancer Res.,* 1988, 79, 866-873; Stacey et al., *J. Immunol,* 1996,157, 2116-2122; Messina et al., *J*. *Immunol*., 1991, 147, 1759-1764; Yi et al., *J. Immunol.,* 1996, 157, 4918-4925; Yi et al., *J. Immunol.,* 1996,157, 5394-5402; Yi et al., *J. Immunol.,* 1998, 160, 4755-4761; and Yi et al., *J*. *Immunol.,* 1998, 160, 5898-5906; International patent applications WO96/02555, WO98/16247, WO98/18810, WO98/40100, WO98/55495, WO98/37919 and WO98/52581) i.e. containing at least one CG dinucleotide, with 5 methylcytosine optionally being used in place of cytosine; (9) a polyoxyethylene ether or a polyoxyethylene ester e.g. WO99/52549; (10) a polyoxyethylene sorbitan ester surfactant in combination with an octoxynol (WO01/21207) or a polyoxyethylene alkyl ether or ester surfactant in combination with at least one additional non-ionic surfactant such as an octoxynol (WO01/21152); (11) a saponin and an immunostimulatory oligonucleotide (e.g., a CpG oligonucleotide) (WO00/62800); (12) an immunostimulant and a particle of metal salt e.g. WO00/23105; (13) a saponin and an oil-in-water emulsion e.g. WO99/11241; (14) a saponin (e.g., QS21) + 3dMPL + IL-12 (optionally + a sterol) e.g. WO98/57659; (15) detoxified mutants of a bacterial ADP-ribosylating toxin such as a cholera toxin (CT), a pertussis toxin (PT), or an *E. coli* heat-labile toxin (LT), particularly LT-K63 (where lysine is substituted for the wild-type amino acid at position 63) LT-R72 (where arginine is substituted for the wild-type amino acid at position 72), CT-S109 (where serine is substituted for the wild-type amino acid at position 109), and PT-K9/G129 (where lysine is substituted for the wild-type amino acid at position 9 and glycine substituted at position 129) (see, e.g., International Publication Nos. WO93/13202 and WO92/19265); and (16) other substances that act as immunostimulating agents to enhance the effectiveness of the composition.. Alum (especially aluminum phosphate and/or hydroxide) and MF59 are preferred.

Muramyl peptides include, but are not limited to, N-acetyl-muramyl-L-threonyl-D-isoglutamine (thr-MDP), N-acteyl-normuramyl-L-alanyl-D-isogluatme (nor-MDP), N-acetylmuramyl-L-alanyl-D-isogluatminyl-L-alanine-2-(1-2'-dipalmitoyl-*sn*-glycero-3-huydroxyphosphoryloxy)-ethylamine (MTP-PE), etc.

For additional examples of adjuvants, see *Vaccine Design, The Subunit and the Adjuvant Approach,* Powell, M.F. and Newman, MJ, eds., Plenum Press, 1995)

Thus, an optional additional component of the compositions of the present invention preferably is an adjuvant such as aluminum salts or an oligonucleotide which comprises at least one CpG motif. As used herein, the phrase "CpG motif' refers to a dinucleotide portion of an oligonucleotide which comprises a cytosine nucleotide followed by a guanosine nucleotide. Such oligonucleotides can be prepared using conventional oligonucleotide synthesis well known to the skilled artisan. Preferably, the oligonucleotides of the invention comprise a modified backbone, such as a phosphorothioate or peptide nucleic acid, so as to confer nuclease resistance to the oligonucleotide. Modified backbones are well known to those skilled in the art. Preferred peptide nucleic acids are described in detail in U.S. Patent Numbers 5,821,060, 5,789,573, 5,736,392, and 5,721,102, Japanese Patent No. 10231290, European Patent No. 839,828, and PCT Publication Numbers WO 98/42735, WO 98/42876, WO 98/36098, WO 98/27105, WO 98/20162, WO 98/16550, WO 98/15648, WO 98/04571, WO 97/41150, WO 97/39024, and WO 97/38013, the disclosures of which are incorporated herein by reference in their entirety.

The oligonucleotide preferably comprises between about 6 and about 100 nucleotides, more preferably between about 8 and about 50 nucleotides, most preferably between about 10 and about 40 nucleotides. In addition, the oligonucleotides of the invention can comprise substitutions of the sugar moieties and nitrogenous base moieties. Preferred oligonucleotides are disclosed in, for example, Krieg *et al., Proc. Natl. Acad. Sci. USA,* **1998**, 95,12631-12636, Klinman *et al., Proc. Natl. Acad. Sci. USA,* **1996**, 93, 2879-2883, Weiner *et al., Proc. Natl. Acad Sci. USA,* **1997,** 94, 10833-10837, Chu *et al., J. Exp. Med,* **1997**,186, 1623-1631, Brazolot-Millan *et al., Proc. Natl. Acad. Sci. USA,* **1998**, 95, 15553-15558, Ballas *et al., J. Immunol.,* **1996**, *157,* 1840-1845, *Cowdery et al., J. Immunol.,* **1996**, *156,* 4570-4575, Halpern *et al., Cell. Immunol.,* **1996**, 167, 72-78, Yamamoto *et al., Jpn. J. Cancer Res.,* **1988,** 79, 866-873, Stacey *et al., J. Immunol.,* **1996**, 157, 2116-2122, *Messina et al*., *J. Immunol.,* **1991,** *147,1759-1764,* Yi *et al., J. Immunol.,* **1996**, 157, 4918-4925, Yi *et al*., *J*. *Immunol*., ***1996*,** 157, 5394-5402, Yi *et al., J. Immunol.,* **1998,** 160, 4755-4761, Roman *et al., Nat. Med*., **1997**, 3, 849-854, Davis *et al., J. Immunol*., **1998,** *160*, 870-876, *Lipford et al., Eur. J. Immunol.,* **1997**, *27*, 2340-2344, Moldoveanu *et al., Vaccine,* **1988**, *16*, 1216-1224, Yi *et al., J. Immunol.,* **1998,** *160*, 5898-5906, PCT Publication WO 96/02555, PCT Publication WO 98/16247, PCT Publication WO 98/18810, PCT Publication VO 98/40100, PCT Publication WO 98/55495, PCT Publication WO 98/37919, and PCT Publication WO 98/52581, the disclosures of which are incorporated herein by reference in their entirety. It is to be understood that the oligonucleotides of the invention comprise at least one CpG motif but can contain a plurality of CpG motifs.

Preferred oligonucleotides comprise nucleotide sequences such as, for example, tccatgacgttcctgacgtt (SEQ ID NO:1), ataatcgacgttcaagcaag (SEQ ID NO:2), ggggtcaacgttgagggggg (SEQ ID NO:3), tctcccagcgtgcgccat (SEQ ID NO:4), gagaacgctcgaccttcgat (SEQ ID NO:5), tccatgtcgttcctgatgct (SEQ ID NO:6), tccatgacgttcctgatgct (SEQ ID NO:7), gctagacgttagcgt (SEQ ID NO:8), atcgactctcgagcgttctc (SEQ ID NO:9), gaaccttccatgctgttccg (SEQ ID NO:10), gctagatgttagcgt (SEQ ID NO:11), tcaacgtt (SEQ ID NO:12), gcaacgtt (SEQ ID NO:13), tcgacgtc (SEQ ID NO:14), tcagcgct (SEQ ID NO:15), tcaacgct (SEQ ID NO:16), tcatcgat (SEQ ID NO:17), tcttcgaa (SEQ ID NO:18), tgactgtgaacgttcgagatga (SEQ ID NO:19), tgactgtgaacgttagcgatga (SEQ ID NO:20), tgactgtgaacgttagagcgga (SEQ ID NO:21), gtttgcgcaacgttgttgccat (SEQ ID NO:22), atggcaacaacgttgcgcaaac (SEQ ID NO:23), cattggaaaacgttcttcgggg (SEQ ID NO:24), ccccgaagaacgttttccaatg (SEQ ID NO:25), attgacgtcaat (SEQ ID NO:26), ctttccattgacgtcaatgggt (SEQ ID NO:27), and tccatacgttcctgacgtt (SEQ ID NO:28). In preferred embodiments of the invention, the oligonucleotide comprises a CpG motif flanked by two purines at the 5' side of the motif and two pyrimidines at the 3' side of the motif It is to be understood, however, that any oligonucleotide comprising a CpG motif can be used in the present invention as long as the oligonucleotide induces an increase in Th1 lymphocyte stimulation when combined with the microparticle compositions described herein.

### 6. Antigens

The present invention is also directed to immunogenic compositions comprising the microparticles described above with adsorbed macromolecules, preferably vector constructs encoding antigens and/or antigen per se. Generally, an antigen stimulates the proliferation of T-lymphocytes, preferably Th1 lymphocytes, with receptors for the antigen and can react with the lymphocytes to initiate the series of responses designated cell-mediated immunity. An antigen may thus induce a CTL response, and/or a humoral response, and may induce cytokine production.

An epitope is within the scope of this definition of antigen. An epitope is that portion of an antigenic molecule or antigenic complex that determines its immunological specificity. Commonly, an epitope is a peptide or polysaccharide in naturally occurring antigens. In artificial antigens it can be a low molecular weight substance such as an arsanilic acid derivative. An epitope will react specifically *in vivo* or *in vitro* with homologous antibodies or T lymphocytes. Alternative descriptors are antigenic determinant, antigenic structural grouping and haptenic grouping.

In preferred embodiments of the invention, the antigenic substance is derived from a virus such as, for example, human immunodeficiency virus (HIV), hepatitis B virus (HBV), hepatitis C virus (HCV), herpes simplex virus (HSV), cytomegalovirus (CMV), influenza virus (flu), and rabies virus. Preferably, the antigenic substance is selected from the group consisting of HSV glycoprotein gD , HIV glycoprotein gp120, HIV p55 gag, and polypeptides from the pol and tat regions. In other preferred embodiments of the invention, the antigenic substance is derived from a bacterium such as, for example, *Helicobacter pylori, Haemophilus influenza,* cholera, diphtheria, tetanus, *Neisseria meningitidis,* and pertussis. In other preferred embodiments of the invention, the antigenic substance is from a parasite such as, for example, a malaria parasite. In another preferred embodiment of the present invention, the antigen is adsorbed to the surface of a microparticle of the present invention.

Antigens can be produced by methods known in the art or can be purchased from commercial sources. Antigens within the scope of this invention include whole inactivated virus particles, isolated virus proteins and protein subunits, whole cells and bacteria, cell membrane and cell wall proteins, and the like. Some preferred antigens are described below.

Herpes simplex virus (HSV) rgD2 is a recombinant protein produced in genetically engineered Chinese hamster ovary cells. This protein has the normal anchor region truncated, resulting in a glycosylated protein secreted into tissue culture medium The gD2 can be purified in the CHO medium to greater than 90% purity. Human immunodeficiency virus (HIV) env-2-3 is a recombinant form of the HIV enveloped protein produced in genetically engineered *Saccharomyces cerevisae.* This protein represents the entire protein region of HIV gp120 but is non glycosylated and denatured as purified from the yeast. HIV gp120 is a fully glycosylated, secreted form of gp120 produced in CHO cells in a fashion similar to the gD2 above. Additional HSV antigens suitable for use in immunogenic compositions are described in PCT Publications W0 85/04587 and W0 88/02634, the disclosures of which are incorporated herein by reference in their entirety. Mixtures of gB and gD antigens, which are truncated surface antigens lacking the anchor regions, are particularly preferred

Additional HIV antigens suitable for use in immunogenic compositions are described in U.S. applications serial no. 490,858, filed March 9, 1990, and published European application number 181150 (May 14, 1986), as well as U.S. applications serial nos. 60/168,471; 09/475,515; 09/475,504; and 09/610,313, the disclosures of which are incorporated herein by reference in their entirety.

Cytomegalovirus antigens suitable for use in immunogenic compositions are described in U.S. Patent No. 4,689,225, U.S. application serial number 367,363, filed June 16, 1989 and PCT Publication WO 89/07143, the disclosures of which are incorporated herein by reference in their entirety.

Hepatitis C antigens suitable for use in immunogenic compositions are described in PCT/US88/04125, published European application number 318216 (May 31, 1989), published Japanese application number 1-500565 filed November 18, 1988, Canadian application 583,561, and EPO 3 88,232, disclosures of which are incorporated herein by reference in their entirety. A different set of HCV antigens is described in European patent application 90/302866.0, filed March 16, 1990, and U.S. application serial number 456,637, filed December 21, 1989, and PCT/US90/01348, the disclosures of which are incorporated herein by reference in their entirety.

Immunogenic compositions of the invention can be used to immunize birds and mammals against diseases and infection, including without limitation cholera, diphtheria, tetanus, pertussis, influenza, measles, meningitis, mumps, plague, poliomyelitis, rabies, Rocky Mountain spotted fever, rubella, smallpox, typhoid, typhus, feline leukemia virus, and yellow fever.

Certain immunogenic compositions of the invention will employ an effective amount of an antigen. For example, there may be included an amount of antigen which, in combination with an adjuvant, will cause the subject to produce a specific and sufficient immunological response, so as to impart protection to the subject from the subsequent exposure to a virus, bacterium, fungus, mycoplasma, or parasite.

In other embodiments, a composition comprising an antigen will be used to boost the immunological response of a previously administered vector construct, which preferably comprises a heterologous nucleic acid sequence that encodes the antigen. More preferably, the antigen is associated with (e.g., adsorbed to) the microparticles described herein and/or the antigen is coadministered with an adjuvant.

No single dose designation can be assigned which will provide specific guidance for each and every antigen which can be employed in this invention. The effective amount of antigen will be a function of its inherent activity and purity and is empirically determined by those of ordinary skill in the art via routine experimentation. It is contemplated that the adjuvant compositions of this invention can be used in conjunction with whole cell or viral immunogenic compositions as well as with purified antigens or protein subunit or peptide immunogenic compositions prepared by recombinant DNA techniques or synthesis.

Where the antigen is provided in connection with an emulsion, because the adjuvant compositions of the invention are stable, the antigen and emulsion can typically be mixed by simple shaking. Other techniques, such as passing a mixture of the adjuvant and solution or suspension of the antigen rapidly through a small opening (such as a hypodermic needle), readily provide a useful immunogenic composition.

The immunogenic compositions according to the present invention comprise about 1 nanogramto about 1000 micrograms of nucleic acid, preferably DNA such as, for example, CpG oligonucleotides. In some preferred embodiments, the immunogenic compositions contain about 10 nanograms to about 800 micrograms of nucleic acid. In some preferred embodiments, the immunogenic compositions contain about 0.1 to about 500 micrograms ofnucleic acid. In some preferred embodiments, the immunogenic compositions contain about 1 microgramto about 10 milligrams of nucleic acid. In some preferred embodiments, the immunogenic compositions contain about 250 micrograms to about 1 milligram of nucleic acid. In some preferred embodiments, the immunogenic compositions contain about 500 micrograms to about 1 milligram of nucleic acid. One skilled in the art can readily formulate an immunogenic composition comprising any desired amount of nucleic acid. The immunogenic compositions according to the present invention are provided sterile and pyrogen free. The immunogenic compositions can be conveniently administered in unit dosage form and can be prepared by any of the methods well known in the pharmaceutical art, for example, as described in *Remington's Pharmaceutical Sciences* (Mack Pub. Co., Easton, PA, 1980), the disclosure of which is incorporated herein by reference in its entirety.

The present invention is also directed to methods of stimulating an immune response in a host animal comprising admmistering to the animal one or more immunogenic compositions described above in an amount effective to induce an immune response. The host animal is preferably a mammal, more preferably a human. Preferred routes of administration include, but are not limited to, intramuscular, intraperitoneal, intradermal, subcutaneous, intravenous, intraarterial, intraoccular and oral as well as transdermal or by inhalation or suppository. Most preferred routes of administration include intramuscular, intraperitoneal, intradermal and subcutaneous injection. According to some embodiments of the present invention, the immunogenic compositions are administered to a host animal using a needleless injection device, which are well known and widely available. One having ordinary skill in the art can, following the teachings herein, use needleless injection devices to deliver immunogenic compositions to cells of an individual. Additionally, the embodiments of the invention may be delivered together with electroporation.

The present invention is also directed to methods of immunizing a host animal against a viral, bacterial, or parasitic infection comprising administering to the animal one or more immunogenic compositions described above in an amount effective to induce a protective response. The host animal is preferably a mammal, more preferably a human. Preferred routes of administration are described above. While prophylactic or therapeutic treatment of the host animal can be directed to any pathogen, preferred pathogens, including, but not limited to, the viral, bacterial and parasitic pathogens described above.

The present invention is also directed to methods of inducing an immune response in a host animal comprising administering to the animal one or more immunogenic compositions described above in an amount effective to induce an immune response. The host animal is preferably a mammal, more preferably a human. Preferred routes of administration are described above. One skilled in the art is readily familiar with immune responses and measurements thereof

The present invention contemplates the use of polymer microparticles or submicron emulsion microparticles with adsorbed macromolecule to elicit an immune response alone, or in combination with another macromolecule. That is, the invention encompasses microparticles with adsorbed nucleic acid, submicron emulsions with adsorbed nucleic acid or immunostimulating molecule, and the combination of microparticles with adsorbed nucleic acid together with submicron emulsions with adsorbed nucleic acid or immunostimulating molecule. Electroporation may also be used to improve delivery of the nucleic acid.

As demonstrated by the following Examples, the present invention's polymer microparticles with adsorbed macromolecules elicit strong immune responses. Additionally, the present invention's submicron emulsion microparticles also elicit strong immune responses. The combination of the present invention's microparticles with adsorbed macromolecules is therefore a powerful tool for eliciting immune responses.

### C. Experimental

Below are examples of specific embodiments for carrying out the present invention. The Examples are offered for illustrative purposes only, and are not intended to limit the scope of the present invention in any way.

Efforts have been made to ensure accuracy with respect to numbers used (e.g., amounts, temperatures, etc.), but some experimental error and deviation should, of course, be allowed for.

### Example 1

### Preparation of Polymer Microparticles with Adsorbed Nucleic Acid

PLG-CTAB microparticles were prepared using a modified solvent evaporation process. Briefly, the microparticles were prepared by emulsifying 10 ml of a 5% w/v polymer solution in methylene chloride with 1 ml of T.E. buffer at high speed using an IKA homogenizer. The primary emulsion was then added to 50ml of distilled water containing cetyl trimethyl ammonium bromide (CTAB) (0.5% w/v). This resulted in the formation of a w/o/w emulsion which was stirred at 6000 rpm for 12 hours at room temperature, allowing the methylene chloride to evaporate. The resulting microparticles were washed twice in distilled water by centrifugation at 10,000 g and freeze dried.

For a typical batch of 100 mg of DNA adsorbed microparticles, 100 mg of PLG-CTAB cationic microparticles were weighed into a glass vial and resuspended with 5 ml volume of 200µg/ml of DNA solution (i.e., the plasmid pCMV or pSINCP containing gp140 or p55gag) in T.E. Buffer. The suspension was vortexed for a one minute to uniformly disperse the microparticles in the DNA solution. The vial was set on a shaker (slow speed) at 4C for overnight adsorption. The next day the microparticles were centrifuged down at 8000 rpm on a Beckman centrifuge for 10 minutes and the supernatant was collected for DNA quantitation. The pellet was washed once with 1X TE buffer by resuspending the pellet in 1 X TE buffer, dispersing with a spatula and centrifuging at 8,000 rpm for 10 minutes. The final pellet was resuspended in a minimum amount of de-ionized water (about 2 ml) by dispersing the pellet with a spatula, and freeze dried on a bench top lyophilizer (Labconco) for 24 hours.

The supernatant was assayed for DNA content by reading the absorbance at 260 nm. Amount of DNA adsorbed on the microparticles was calculated by subtracting the amount in the supernatant from the total DNA input (1 mg per 100 mg of microparticles. The total load was estimated by dissolving 5 mg of final formulation in 0.5 M NaOH/1% SDS solution and reading the clear solution after hydrolysis at 260 nm.

### Example 2

### Preparation of Submicron emulsion Microparticles with Adsorbed Nucleic Acid

A submicron emulsion formed from MF59 and DOTAP was prepared by providing DOTAP (in chloroform) in a beaker and allowing it to evaporate down to 200ul. Tween (0.5% w/w), Squalene (5.0% w/w) and Span (0.5% w/w) were added and homogenized for 1 minute using an Omni homogenizer with a 10mm probe at 10K revs/min in order to provide a homogeneous feedstock for final emulsification. This was passed 5 times through a Microfluidizer M110S homogenizer (Microfluidics Co., Newton, MA) at -800 psi. The zeta potential of the emulsion, which is a measure of net surface charge, was measured on a DELSA 440 SX Zetasizer from Coulter and found to be approximately + 55 mV.

DNA (either 1 mg HIV-1 gp140 DNA or 0.5 mg of p55 gag DNA, present in pCMV or pSINCP) was adsorbed by incubation with the submicron emulsion overnight at 4°C.

### Example 3

### Preparation of ELVIS Vectors and Other Vector Constructs for Adsorption to Microparticles

Construction of alphavirus-based ELVIS and replicon vectors was performed using Sindbis virus as a representative example. As will be appreciated, the following may be readily applied to the derivation of vectors from any alphavirus by one of skill in the art. Approximately 10⁷ BHK-21 cells were infected with the SINDCchiron strain of Sindbis virus (ATCC deposit VR-2643, April 13, 1999) at a MOI of 1 PFU/cell At 24 hours post-infection, after development of CPE, total RNA was isolated from the cells using the TRIzol Reagent (GIBCO/BRL) according to the manufacturer's instructions. After purification, viral RNA was dissolved in nuclease-free water, aliquoted, and stored at -80°C for subsequent use in cDNA cloning.

Synthesis of cDNA was accomplished by PCR amplification, using the primer sets shown below (Sindbis nucleotide numbering indicated for each primer):

1 | CCACAAGCTTGATCTAATGTACCAGCCTGATGC | 11472-11450 | (SEQ ID NO:29)
1.1 | CCACGAATTCAGCCAGATGAGTGAGGC | 10364-10381 | (SEQ ID NO:30)
2 | CCACAAGCTTCAATTCGACGTACGCCTCAC | 10394-10375 | (SEQ ID NO:31)
2.1 | CCACGAATTCATATGGGGAAATCATGAGCC | 9614-9634 | (SEQ ID NO:32)
3 | CCACAAGCTTCATAGACCCTCACTGGCTC | 9648-9630 | (SEQ ID NO:33)
3.1 | CCACGAATTCAAGATTAGCACCTCAGGACC | 8878-8899 | (SEQ ID NO:34)
4 | CCACAAGCTTCTACACGGTCCTGAGGTGC | 8908-8887 | (SEQ ID NO:35)
4.1 | CCACGAATTCGTCCGATCATGGATAACTCC | 8294-8315 | (SEQ ID NO:36)
5 | CCACAAGCTTGCGCCACCGAGGAC | 8347-8334 | (SEQ ID NO:37)
5.1 | CCACGAATTCACTGCCATGTGGAGGCC | 7797-7814 | (SEQ ID NO:38)
6 | CCACCTCGAGTTTACCCAACTTAAACAGCC | 7368-7348 | (SEQ ID NO:39)
6.1 | CCACGAGCTCGCGACATTCAATGTCGAATGC | 6426-6446 | (SEQ ID NO:40)
7 | CCACCTCGAGGAACTCCTCCCAATACTCGTC | 6488-6468 | (SEQ ID NO:41)
7.1 | CCACGAGCTCGACCTTGGAGCGCAATGTCC | 5843-5862 | (SEQ ID NO:42)

8 | CCACCTCGAGTTTCGACGTGTCGAGCACC | 5900-5882 (SEQ ID NO:43)
8.1 | CCACGAGCTCGACCATGGAAGCAATCCGC | 4814-4832 (SEQ ID NO:44)
9 | CCACCTCGAGACGACGGGTTATGGTCGAC | 4864-4845 (SEQ ID NO:45)
9.1 | CCACGAGCTCCACGGAGACAGGCACCGC | 4246-4264 (SEQ ID NO:46)
10 | CCACCTCGAGGATCACTTTCTTTCCTAGGCAC | 4299-4277 (SEQ ID NO:47)
10.1 | CCACGAGCTCGAACTCTCCCGTAGATTTCC | 3407-3427 (SEQ ID NO:48)
11 | CCACCTCGAGATCAAGTTGTGTGCCCTTCC | 3464-3445 (SEQ ID NO:49)
11.1 | CCACGAGCTCCCAGGGGATATCATCCTGAC | 2742-2761 (SEQ ID NO:50)
12 | CCACCTCGAGGCTGTCATTACTTCATGTCCG | 2825-2804 (SEQ ID NO:51)
12.1 | CCACGAGCTCGAACCGCAAACTATACCACATTGC | 1976-1999 (SEQ ID NO:52)
13 | CCACCTCGAGCTTGTACTGCTCCTCTTCTG | 2042-2023 (SEQ ID NO:53)
13.1 | CCACGAGCTCGGAGAACGGGTATCGTTCC | 1029-1047 (SEQ ID NO:54
14 | CCACCTCGAGCCGGGATGTACGTGCAC | 1069-1052 (SEQ ID NO:55)
14.1 | CCACGAGCTCATTGACGGCGTAGTACACAC | 1-20 (SEQ ID NO:56)

Primer pairs 1-5 were used for cloning of the virus structural protein genes, while pairs 6-14 were for the virus nonstructural protein genes. Oligonucleotides in pairs 1-5 contained additional sequences representing restriction enzyme sites for *EcoR*I and *Hind*III, which are not present in subgenomic RNA of Sindbis virus. Oligonucleotides 6-14 contained sites for *Sac*I and *Xho*I, which are not present in the whole genome of previously sequenced strains of Sindbis virus (these sites are underlined).

Each reverse transcription (RT) reaction was performed in a 50 µl volume using the SuperscriptII enzyme (GIBCO/BRL), according to the manufacturer's instructions. Reaction mixtures contained the amount of RNA equivalent to 10⁶ cells and 50 pmoles of each primer shown below.
Mixture1: primers1, 3 and 5
Mixture2: primers 2 and 4
Mixture3: primers 6, 9 and 12
Mixture4: primers 8, 11 and 14

RT reactions were frozen and then used subsequently for PCR amplification PCR reactions were performed using Vent DNA polymerase (NEB) as recommended by the manufacturer. Each 50 µl PCR reaction contained 3 µl of RT mixtures described above and 50 pmoles of primers. A total of 14 reactions were performed (Table 1).

**TABLE 1**

| N of fragment | Primers | N of RT reaction. | Length of the fragment (bp.) |
|---|---|---|---|
| 1 | 1 and 1.1 | | 1128 |
| 2 | 2 and 2.1 | 2 | 800 |
| 3 | 3 and 3.1 | 1 | 789 |
| 4 | 4 and 4.1 | 2 | 644 |
| 5 | 5 and 5.1 | 1 | 670 |
| 6 | 6 and 6.1 | 3 | 962 |
| 7 | 7 and 7.1 | 3 | 666 |
| 8 | 8 and 8.1 | 4 | 1107 |
| 9 | 9 and 9.1 | 3 | 638 |
| 10 | 10 and 10.1 | 3 | 912 |
| 11 | 11 and 11.1 | 4 | 743 |
| 12 | 12 and 12.1 | 3 | 870 |
| 13 | 13 and 13.1 | 3 | 1034 |
| 14 | 14 and 14.1 | 4 | 1088 |

PCR reactions for fragments 1-5 were performed using the following conditions: 12 cycles of 95°C for 30 seconds, 56°C for 30 seconds and 74°C for 90 seconds. For fragments 6-14, the number of cycles was changed from 12 to 15. A small aliquot of each reaction mixture was analyzed by agarose gel electrophoresis to confirm the presence of the fragments of the expected size. The remaining reaction mixture was extracted with phenol-chloroform and DNA fragments were precipitated using ethanol.

For cloning, fragments 1-5 were digested with *Hind*III and *Eco*RI, and then ligated with plasmid pRS2 (pUC19 with additional restriction sites in polylinker) treated with the same enzymes. Fragments 6-14 were digested with *Sac*I and *Xho*I and ligated with the same pRS2 plasmid treated with *Sac*I and *Xho*I. All recombinant plasmids were transformed into the *E. coli* XL-1 Blue strain (Stratagene, La Jolla, CA).

In addition, cDNA clones representing the subgenomic promoter region and 3'-end nontranslated regions also were generated using the following primer pairs:

YSIN1F: 5'-GATTCGGTTACTTCCACAGC (SEQ ID NO:57)

YSIN1R: 5'-ACTGACGGCTGTGGTCAGTT (SEQ ID NO:58)

YSIN2F: 5'-GATGTACTTCCGAGGAACTG (SEQ ID NO:59)

YSIN2R: 5'-CCACAAGCTTGAAATGTTAAAAACAAAATTTTGT (SEQ ID NO:60)

Positive colonies for each transformation were grown for plasmid purification using a QIAGEN kit according to the manufacturer's instructions. The fragments, designated p1-p14 correspondingly, were then assembled into the appropriate vector configurations.

The construction of a Eukaryotic Layered Vector Initiation System (ELVIS) and an alphavirus vector construct for in vitro transcription of replicon vector RNA was accomplished using the Sindbis virus cDNA clones p1-p14, plus the subgenomic and 3'-end region fragments as follows. An *Apa*I*-Msc*I fragment, containing the promoter for SP6 RNA polymerase and start of the Sindbis virus genomic RNA, was ligated with the *Msc*I*-Xho*I fragment of cloned fragment 14 in *Apa*I*-Xho*I digested plasmid pRS2. The resulting plasmid was named p15. Next, the *Sac*I*-Eco*RI fragment of p8, the *Eco*RI*-Nsi*I fragment of p7 and the *Nsi*I*-Xho*I fragment of p6 were ligated into *Sac*I*-Xho*I digested pRS2. The resulting plasmid was named p16. Next, the *Sac*I*-Mun*I fragment of p12, the *Mun*I*-Nhe*I fragment of p11 and the *Nhe*I*-Xho*I fragment of p10 were ligated into *SacI-Xho*I digested pRS2 plasmid. The resulting plasmid was named p17. The *Apa*I*-Apa*LI fragment of p15 and the *Apa*LI- *Xho*I fragment of p13 then were ligated into *Apa*I-*Xho*I treated pRS2, resulting in the plasmid named p18. Next, the *Apa*I*-Nsi*I fragment of p18 and the *Nsi*I*-Xho*I fragment of p17 were ligated together in*Apa*I *-Xho*I treated pRS2. The resulting plasmid was named p19. Finally, the *Apa*I*-Avr*II fragment of p19, the *Avr*II*-Sal*GI fragment of p9 and the *Sal*GI*-Bam*HI fragment of p 16 were ligated together into a previously constructed Sindbis replicon vector expressing the GFP reporter (see Dubensky et aL, *J. Virol.* 70:508-519, 1996; Polo et al, 1999, *ibid;* and U.S. Patent 5,843,723), that had been digested *withApaI-BamHI* to remove the existing nonstructural protein genes. The resulting Sindbis vector construct, which contains sequences derived from the SINDCchiron virus strain and also encodes a GFP reporter, was designated SINCR-GFP (also known as DCSP6SINgfp). Preparation ofreplicon RNA from this reporter construct, as well as Sindbis vector constructs expressing various other heterologous sequences (e.g., antigens, described in the specification and below) was performed by linearization of the DNA using *Pme*I, followed by *in vitro* transcription using bacteriophage SP6 polymerase as described previously (Polo et al., *ibid*; Dubensky et al., *ibid*).

Similarly, the same Sindbis sequences were used for assembly into an alphavirus-based Eukaryotic Layered Vector Initiation System (see U.S. 5,814,482 and 6,015,686), in which the transcription of self-amplifying vector RNA takes place directly within ELVIS plasmid DNA-transfected eukaryotic cells via a eukaryotic promoter (e.g., RNA polymerase II promoter). An ELVIS plasmid DNA, which also expressed GFP reporter, was constructed by replacing Sindbis virus derived sequences in an existing ELVIS vector with the corresponding SINCR-GFP sequences from above. Starting with the previously described ELVIS vector pSIN1.5 (Hariharan et al., *J*. *Virol*. 72:950-958,1998), the plasmid backbone first was modified by substituting the plasmid backbone with that from pCMVLink (zur Megede et al., *J*. *Virol*. 74:2628-2635, 2000) using two *Sac*I sites found in each plasmid, to generate the intermediate construct known as ELVIS1.5CB. Next, one of the two *Sac*I sites of ELVIS1.5CB (located adjacent to the SIN 3'-end) was eliminated by partially digesting with *Sac*I, blunt-ending using T4 DNA polymerase, and then ligating into the modified site, a*Pme*I linker 5'-GTTTAAAC-3'. The correct plasmid without the targeted *Sac*I site was designated ELVIS 1. 5CBdISac. This intermediate plasmid then was prepared for insertion of the new SIN nonstructural protein genes by digestion with *Sac*I and *Xho*I. The corresponding nonstructural genes were obtained by PCR amplification from SINCR-GFP using the oligonucleotide primers
5'CCTATGAGCTCGTTTAGTGAACCGTATTGACGGCGTAGTACACAC (SEQ ID NO:61) and 5'CCTATCTCGAGGGTGGTGTTGTAGTATTAGTC (SEQ ID NO:62), followed by digestion with *SacI* and *Xho*I*,* and ligation, to produce the intermediate construct SINCP-Not. Finally, one of the two *Not*I sites present in this construct was eliminated by partial digest and Klenow fill-in, to leave only one *Not*I site in the polylinker. This newly constructed ELVIS vector was designated SINCP (or pSINCP).

Insertion of heterologous sequences (e.g., antigen-encoding genes) into the SINCR or SINCP alphavirus vectors is performed primarily by digestion with *Xho*I/*Not*I or *Xho*I/*Xba*I*,* followed by ligation with a desired DNA fragment that also has *Xho*I/*Not*I or *Xho*I/*Xba*I termini. Alternatively, these sites may be blunt-ended or other polylinker sites may be used (or other heterologous sequences may be replaced) to allow cloning of a greater number of inserts. For example, the HIV-1 p55gag (SF2 strain) and gp140 env (SF162 strain) encoding genes were inserted into these vectors. Specifically, the codon-optimized HIV p55gagmod sequence *(see* commonly owned U.S. Patent Application 09/475,515; zur Megede et al., *ibid*) was inserted by digesting the vectors with *Xho*I/*Xba*I and ligating in the p55gagmod fragment obtained by digestion with *Sal*I/*Xba*I*.* The resulting vectors were designated SINCR-p55gag and SINCP-p55gag. Similarly, codon-optimized HIV gp140 sequences (described in Example 10 and Barnett et al., 2001. J. Virol. 75:5526-40), were inserted into both the SINCR and SINCP plasmids to generate the constructs SINCR-gp140 and SINCP-gp140. Formulation of ELVIS plasmid DNA (pSINCP) and RNA vector replicons transcribed *in vitro* from the SINCR plasmids is performed as described elsewhere in the Examples.

### Example 4

### Immunization of Rhesus Macaques with Antigen with pCMV or pSINCP Plasmids using Microparticles or Submicron emulsions

PLG polymer microparticles and MF59 submicron emulsions were formed as described above in previous Examples 1 and 2. Groups of microparticles and submicron emulsions were made in order to analyze the different effects of immunizing rhesus macaques with a plasmid vector construct, pCMV- gp140 or pCMV-p55gag *(see* commonly owned U.S. Patent Application 09/475,515), on microparticles or in a submicron emulsion, as well as comparing the effect of using an ELVIS plasmid, pSINCP-gp140 or pSINCP-p55gag, constructed as described above. Six groups of animals were immunized with different formulations as follows:
Group 1 used pCMV- gp140 and pCMV - p55 gag without microparticles or submicron emulsions.
Group 2 used pCMV- gp140 and pCMV - p55 gag adsorbed on PLG/CTAB microparticles.
Group 3 used pCMV- gp140 and pCMV - p55 gag adsorbed to an MF59-DOTAP submicron emulsion.
Group 4 used pSINCP- gp140 and pSINCP - p55 gag without microparticles or submicron emulsions.
Group 5 used pSINCP- gp140 and pSINCP - p55 gag adsorbed on PLG/CTAB microparticles.
Group 6, a control, used no antigen, no microparticles, and no submicron emulsions.

For each group of animals, 5 rhesus macaques (only 4 for group 6) were immunized with sufficient quantities of material such that the dosage of vector with gp140 DNA was 1.0 mg each, and vector containing p55 gag DNA was 0.5 mg each, except for the control which had none. The animals were immunized a second time four weeks after the first immunization, and a third time 14 weeks after the first immunization. Serum was analyzed at weeks 2 (2wp1), 6 (2wp2), 11-12 weeks (7wp2), and 16 (2wp3). The route of immunization was IM TA. Following immunizations, plasma anti-p55gag and anti-gp140 IgG titers were measured, the results of which appear below in Tables 2 and 3 as geometric mean titers.

**Table 2**

| **Serum IgG Titer for anti-p55 gag (Geometric Mean)** | | | | | |
|---|---|---|---|---|---|
| **Group** | Form of DNA | 2wp1 | 2wp2 | 7wp2 | 2wp3 |
| **1** | PCMV in saline solution | 7 | 19 | 19 | 118 |
| **2** | PCMV adsorbed on PLG/CTAB particles | 490 | 10770 | 4360 | 1637 |
| **3** | PCMV adsorbed on MF59/DOTAP emulsion | 142 | 5702 | 1480 | 3536 |
| **4** | pSINCP in saline solution | 8 | 7 | 8 | 45 |
| **5** | pSINCP adsorbed on PLG/CTAB particles | 728 | 19256 | 3426 | 856 |
| **6** | none | 12 | 9 | 8 | 7 |

**Table 3**

| **Serum IgG Titer for anti-gp140 (Geometric Mean)** | | | | | |
|---|---|---|---|---|---|
| **Group** | Form of DNA | 2wp1 | 2wp2 | 7wp2 | 2wp3 |
| **1** | pCMV in saline solution | 5 | 517 | 81 | 2460 |
| **2** | pCMV adsorbed on PLG/CTAB particles | 5 | 2762 | 5290 | 1913 |
| **3** | pCMV adsorbed on MF59/DOTAP emulsion | 5 | 564 | 112 | 4823 |
| **4** | pSINCP in saline solution | 8 | 48 | 20 | 70 |
| **5** | pSINCP adsorbed on PLG/CTAB particles | 15 | 11289 | 4266 | 1002 |
| **6** | none | 12 | 14 | 11 | 11 |

The same group of animals as was used for the preceding animals were analyzed for induction of a CTL response. The effector to target (E:T) ratios ranged from approximately 4:1 to 100:1. The results of the CTL assay appear below in Tables 4 and 5. A "responder" is a rhesus macaque which showed 10% or more specific lysis of the target at two or more consecutive E:T ratios.

**Table 4**

| **Number of Responders (p55gag)** | | | | | |
|---|---|---|---|---|---|
| **Group** | Form of DNA | 2wp1 | 2wp2 | 7wp2 | 2wp3 |
| **1** | pCMV in saline solution | 0 | 4 | 1 | 4 |
| **2** | pCMV adsorbed on PLG/CTAB particles | 3 | 3 | 2 | 3 |
| **3** | pCMV aaisorbed on MF59/DOTAP emulsion | 0 | 1 | 1 | 0 |
| **4** | pSINCP in saline solution | 0 | 1 | 0 | 0 |
| **5** | pSINCP adsorbed on PLG/CTAB particles | 0 | 3 | 1 | 1 |
| **6** | none | 0 | 0 | 0 | 0 |

**Table 5**

| **Number of Responders (gp140)** | | | | | |
|---|---|---|---|---|---|
| **Group** | Form of DNA | 2wp1 | 2wp2 | 7wp2 | 2wp3 |
| **1** | PCMV in saline solution | 0 | 0 | 0 | 0 |
| **2** | pCMV adsorbed on PLG/CTAB particles | 0 | 0 | 0 | 0 |
| **3** | pCMV adsorbed on MF59/DOTAP emulsion | 0 | 0 | 0 | 0 |
| **4** | pSINCP in saline solution | 0 | 0 | 0 | 1 |
| **5** | pSINCP adsorbed on PLG/CTAB particles | 0 | 1 | 0 | 1 |
| **6** | none | 0 | 0 | 0 | 0 |

The same animals were also analyzed for lymphoproliferation. This assay measures specific proliferation ofT cells in vitro in response to restimulation with antigen. Rhesus macaque peripheral blood mononuclear cells (PBMC) were purified from heparinized whole blood by centrifugation on Ficoll-Hypaque gradients. PBMC were cultured at the number of 2 x 10⁵ per well in flat bottom microtiter plates in the presence or absence of 3 micrograms/ml of purified recombinant p55gag protein. Six replicate cultures per condition were initiated. After 4 days of culture tritiated thymidine ([³H]TdR) was added (1 microcurie per well). Cultures were continued overnight and harvested the following day. Cells were deposited onto glass microfiber filter sheets. Filter sheets were exposed to scintillation fluid and counted in liquid scintillation counter. For each condition [³H]TdR incorporation, measured as the mean counts per min (cpm) for the 6 replicates was calculated. The results appear below in Table 6. Geometric Mean Stimulation Index (GMSI) is calculated as counts per minute (cpm) of p55gag stimulated cells divided by cpm of unstimulated cells, thus, the larger the GMSI, the more positive the result.

**Table 6**

| **GMSI** | | | | | |
|---|---|---|---|---|---|
| **Group** | Form of DNA | 2wp1 | 2wp2 | 7wp2 | 2wp3 |
| **1** | PCMV in saline solution | 2.6 | 5.1 | 3.0 | 3.2 |
| **2** | pCMV adsorbed on PLG/CTAB particles | 6.6 | 15.4 | 5.9 | 4.6 |
| **3** | pCMV adsorbed on MF59/DOTAP emulsion | 9.1 | 29.5 | 13.6 | 10.5 |
| **4** | pSINCP in saline solution | 7.5 | 6.6 | 4.1 | 4.3 |
| **5** | pSINCP adsorbed on PLG/CTAB particles | 10.4 | 13.8 | 5.4 | 4.4 |
| **6** | none | 1.4 | 1.5 | 1.3 | 1.2 |

The same animals were also analyzed for induction of intracellular cytokine production. This assay measures specific production of cytokines by T cells in vitro in response to brief restimulation with antigen. Rhesus macaque peripheral blood mononuclear cells (PBMC) were purified fromheparizined whole blood by centrifugation on Ficoll-Hypaque gradients. Aliquots of 1 ×10⁶ PBMC were stimulated with a pool of synthetic overlapping peptides that span the gag (or env) protein sequence in the presence of a co-stimulatory anti.-CD28 monoclonal antibody. Brefeldin A was added to allow the accumulation of newly synthesized cytokines within cells. After overnight incubation PBMC were stained with commercially available, fluorescently labeled monoclonal antibodies for the presence of intracellular interferon-γ (IFN-γ) and tumor necrosis factor-α (TNF-α) and for cell surface CD4 and CD8 markers. Stained cell samples were analyzed on a flow cytometer and data were acquired for approximately 50,000 - 100,000 PBMC. The frequency of cytokine-positive cells was determined for each sample using commercially available software. The results for gp140 and p55gag are shown in Tables 7 and 8, respectively. The Tables show the number of responding animals, where a responder is defined as an animal scoring greater than 100 CD4 cells per 100,000 expressing TNF-α and IFN- y as measured by intracellular staining.

**Table 7**

| **gp140 cytokine responders** | | | | |
|---|---|---|---|---|
| **Group** | Form of DNA | 2wp2 | 7wp2 | 2wp3 |
| **1** | pCMV in saline solution | 1 | 0 | 0 |
| **2** | pCMV adsorbed on PLG/CTAB particles | 1 | 2 | 0 |
| **3** | pCMV adsorbed on MF59/DOTAP emulsion | 0 | 0 | 0 |
| **4** | pSINCP in saline solution | 0 | 0 | 0 |
| **5** | pSINCP adsorbed on PLG/CTAB particles | 0 | 1 | 0 |
| **6** | None | 0 | 0 | 0 |

**Table 8**

| **p55gag cytokine responders** | | | | |
|---|---|---|---|---|
| **Group** | Form of DNA | 2wp2 | 7wp2 | 2wp3 |
| **1** | pCMV in saline solution | 0 | 0 | 0 |
| **2** | pCMV adsorbed on PLG/CTAB particles | 2 | 1 | 0 |
| **3** | pCMV adsorbed on MF59/DOTAP emulsion | 0 | 0 | 0 |
| **4** | pSINCP in saline solution | 0 | 0 | 0 |
| **5** | pSINCP adsorbed on PLG/CTAB particles | 1 | 1 | 0 |
| **6** | none | 0 | 0 | 0 |

### Example 5

### RNA Vector Constructs

RNA vector constructs (e.g., replicons) may be adsorbed to microparticles for delivery of heterologous nucleic acid sequences to the cells of animals. The RNA vector construct generally comprises a viral RNA which has had a region of the genomic RNA (e.g., structural protein gene) replaced with the selected heterologous sequence, derived from the DNA coding sequence for the gene-product of interest. Representative examples of RNA vector constructs include, but are not limited to, alphavirus RNA vectors (see for example, US Patent 5843723, PCT publication WO 99/18226, and Polo et al., 1999, PNAS 96:4598-4603), picornavirus RNA vectors (see for example, US Patent 6156538, and Vignuzzi et al., 2001, J Gen Virol 82:1737-47), flavivirus RNA vectors (see for example, Varnavski et al. 1999, Virology 255:366-75), and rubivirus RNA vectors (see for example, Pugachev et al., 2000, J Virol. 74:10811-5).RNA vector constructs for use in the present invention generally may be obtained from plasmid cDNA constructs as a source of starting material, by the standard process of in vitro transcription (see references above). Similarly to plasmid DNA, these RNA vector constructs then may be adsorbed to microparticles of the invention as described elsewhere in the examples. For example, the RNA vector constructs are adsorbed onto the microparticles by incubating 100 mg of cationic microparticles in a 1 mg/ml solution of DNA at 4°C for 6 hr. The microparticles are then separated by centrifugation, the pellet washed with TE buffer, and the microparticles freeze-dried. Reconstitution and delivery of the PLG-formulated RNA vector constructs is similar to that described for DNA, using for example at least 1 ug, 10 ug, 100ug, or 1000 ug of formulated RNA vector construct for delivery.

### Example 6

### Adjuvants in Mice

An experiment with mice was performed to analyze the effect of the adjuvant aluminum phosphate (alum) in mice. Polymer microparticles were prepared as above described with or without pCMV-p55gag adsorbed thereon. 10 micrograms of DNA, whether naked or adsorbed to the PLG microparticles was injected in groups of 6 CB6 F1 mice on weeks 0 and 6, without or without alum The results, as geometric mean titers of antibody, are shown below in Table 9.

**Table 9**

| **Senun IgG Titer for anti-p55 gag (Geometric Mean/Standard Error)** | | | | | |
|---|---|---|---|---|---|
| **Group** | Form of DNA | 3 weeks | 6 weeks | 9 weeks | 12 weeks |
| **1** | pCMV in saline solution | 28 | 149 | 6238 | 5274 |
| **2** | pCMV adsorbed on PLG microparticles | 5022 | 21992 | 346856 | 171301 |
| **3** | pCMV in saline solution plus alum | 1536 | 3039 | 195070 | 92438 |

### Example 7

### Electroporation with Microparticles and ELVIS Vectors and RNA Vector Constructs

Electroporation may be used in combination with polymer microparticles or submicron emulsion microparticles made with any of the nucleic acids described above, such as plasmid DNA, ELVIS vectors, and RNA vector constructs.

### Examples 8

### Induction of Immune Response in Rhesus with Prime and Boost Immunizations

An experiment was performed to determine the effect of priming with DNA and boosting with protein adsorbed to PLG microparticles. Particularly, PLG-SDS microparticles were prepared as described above and in commonly owned International patent application PCT/US99/17308, and purified recombinant p55gag protein was adsorbed thereto. A group of animals was immunized with 1 mg pCMV-p55gag. The animals were immunized again at 4 weeks, then again at 8 weeks. The animals were boosted with p55gag protein adsorbed to PLG microparticles at 41 weeks. The results are shown in Table 10 below, which shows antibody titer for responders, induction of helper T cell lymphoproliferation (mean stimulation index of responders), and induction of CTL (number of responders, based on greater than 10% lysis at two or more consecutive E:T ratios). Results were measured at 14 weeks post 3^{rd} prime for the prime columns, and 2 weeks post boost for the boost columns. Numbers in parentheses indicate the number of responders out of a total of 4 animals

**Table 10**

| **Vaccine** | | **Antibody Titer** | | **Lymphoproliferation (SI)** | | **CTL** | |
|---|---|---|---|---|---|---|---|
| **prime** | **boost** | **prime** | **boost** | **prime** | **boost** | **prime** | **boost** |
| pCMV-p55gag | PLG/ p55gag | 44 (1) | 1777 (4) | 2.5 (2) | 21.8 (4) | 4 | 4 |

### Example 9

### Induction of Neutralizing Antibodies

The sera from the rhesus macaques in Example 5 above were tested for inhibition of two different HIV-1 strains (SF2 and SF162) using PMBC-grown virus stocks and a CCR5+/CXCR4+/CD4+ T cell line based assay. Sera were used at a dilution of 1:20. Inhibitory activity was measured and expressed as a percentage inhibition. The inhibitory activity of the animals' sera prior to immunizations were subtracted from the results for each animal. The results for each of the five animals in each group are shown in Table 11 below.

**Table 11**

| **Percent Inhibition** | | | | | |
|---|---|---|---|---|---|
| **Group** | Form of DNA | HIV-1_{SF2} | | HIV-1_{SF162} | |
| | | 2wp2 | 2wp3 | 2wp2 | 2wp3 |
| **1** | pCMV in saline solution | 0 | 0 | 0 | 0 |
| | | 0 | 0 | 0 | 17 |
| | | 0 | 8 | 0 | 0 |
| | | 0 | 0 | 0 | 12 |
| | | 0 | 0 | 0 | 52 |
| **2** | pCMV adsorbed on | 58 | 40 | 3 | 0 |
| | PLG/CTAB particles | 0 | 41 | 6 | 0 |
| | | 0 | 0 | 0 | 0 |
| | | 72 | 79 | 0 | 0 |
| | | 48 | 61 | 11 | 10 |
| **3** | pCMV adsorbed on | 0 | 100 | 0 | 0 |
| | MF59/DOTAP emulsion | 0 | 0 | 0 | 0 |
| | | 76 | 100 | 3 | 4 |
| | | 81 | 100 | 0 | 0 |
| | | 15 | 93 | 0 | 0 |
| **4** | pSINCP in saline solution | 0 | 0 | 0 | 0 |
| | | 0 | 0 | 0 | 0 |
| | | 0 | 0 | 12 | 1 |
| | | 0 | 0 | 0 | 0 |
| | | 0 | 0 | 16 | 0 |
| **5** | pSINCP adsorbed on | 73 | 50 | 19 | 0 |
| | PLG/CTAB particles | 43 | 0 | 29 | 0 |
| | | 91 | 0 | 4 | 0 |
| | | 69 | 0 | 32 | 6 |
| | | 91 | 23 | not available | 0 |

### Example 10

### Preparation of Plasmids

Plasmids encoding HIV-1 p55gag and gp140env driven by the human cytomegalovirus (CMV) promoter were grown in *Escherichia coli* strain DH5α, purified using a Qiagen Endofree Plasmid Giga kit (Qiagen, Inc.), and resuspended in 0.9% sodium chloride (Abbott Laboratories, North Chicago, III). The pCMV vector used contains the immediate-early enhancer/promoter of CMV and a bovine growth hormone terminator and is described in detail elsewhere (Chapman, B. S., et al. 1991. "Effect of intron A from human cytomegalovirus (Towne) immediate-early gene on heterologous expression in mammalian cells." Nucleic Acids Res. **19**:3979-86). The HIV gag plasmid DNA vaccine (pCMVgag) contains a synthetically constructed p55gag gene, with codons reflecting mammalian usage, derived from the HIV-1 SF2 strain as previously described (zur Megede, J., et al. 2000. "Increased expression and immunogenicity of sequence-modified human immunodeficiency virus type 1 gag gene." J Virol. **74**:2628-35). The HIV env plasmid DNA vaccine (pCMVgp140) consisted of a human tissue plasminogen activator (tPA) signal sequence and the gp140 from HIV-1 SF162 strain, codon optimized for high level expression in mammalian cells (Barnett, S. W., et. 2001. "The ability of an oligomeric human immunodeficiency virus type 1 (HIV-1) envelope antigen to elicit neutralizing antibodies against primary HIV-1 isolates is improved following partial deletion of the second hypervariable region." J Virol. **75**:5526-40). The SINCP plasmid vector with either HIV-1 p55gag or gp140env has been described in Example 3 above.

### Example 11

### Preparation of Proteins

The protein and cDNA sequences for the gp160env. SF162 have been published in Cheng-Mayer, C., M. Quiroga, J. W. Tung, D. Dina, and J. A. Levy. 1990. "Viral determinants of human immunodeficiency virus type 1 T-cell or macrophage tropism, cytopathogenicity, and CD4 antigen modulation." J Virol. 64:4390-8. These sequences can be found under Genbank accession number M65024. Recombinant HIV-1 g140.SF162(dV2) protein was expressed in Chinese hamster ovary cells and purified as previously described (Barnett, S. W., ET. 2001. J Virol. 75:5526-40). Recombinant HIV-1.SF2 p55 gag protein was expressed in yeast and purified by cation exchange chromatography (Chiron Corporation, Emeryville, CA). The p55gag cDNA sequence from the SF2 strain of HIV-1 (Genbank accession number K02007) was cloned into a ubiquitin expression vector, resulting in the addition of glycine and arginine to the N-terminus of the wild-type sequence. The recombinant p55gag protein was extracted from the yeast cell pellet using 50 mM phosphate, 6M urea, pH7.9, followed by S-fractogel (cationic) ion exchange chromatography. Elution of the p55gag was obtained with a linear NaCl gradient (peak at 0.4m NaCl). The estimated purity was 90% by SDS-PAGE.

### Example 12

### DNA-adsorbed poly(lactide-co-glycolide) PLG microparticles

PLG polymer (RG505) was obtained from Boehringer Ingelheim. Cationic microparticles were prepared using a modified solvent evaporation process. Briefly, the microparticles were prepared by emulsifying 10 ml of a 5% (wt/vol) polymer solution in methylene chloride with1 ml of phosphate-buffered saline (PBS) at high speed using an *IKA* homogenizer. The primary emulsion was then added to 50 ml of distilled water containing cetyltrimethylammonium bromide (CTAB) (0.5% wt/vol), resulting in the formation of a water-in-oil-in-water emulsion, which was stirred at 6,000 rpm for 12 h at room temperature, allowing the methylene chloride to evaporate. The resulting microparticles were washed twice in distilled water by centrifugation at 10,000g and freeze-dried. Plasmid DNA from Example 11 was adsorbed onto the microparticles by incubating 100 mg of cationic microparticles 5ml of a 200 microgram/ml solution of DNA at 4°C for 6h. The microparticles were then separated by centrifugation, the pellet was washed with TE (Tris-EDTA) buffer, and the microparticles were freeze-dried.

### Example 13

### Protein-adsorbed PLG microparticles

Blank microparticles were prepared by a solvent evaporation technique. Briefly, microparticles were prepared by homogenizing 10ml 6% w/v polymer solution in methylene chloride, with 40 ml of distilled water containing SDS (1% w/v) at high speed using a 10mm probe. This resulted in an oil in water emulsion, which was stirred at 1000 rpm for 12 hours at room temperature, and the methylene chloride was allowed to evaporate. The resulting microparticles were filtered through a 38um mesh, washed 3 times in distilled water, and freeze-dried. The size distribution of the microparticles was determined using a particles size analyzer (Master sizer, Malvem Instruments, UK).

50mg lyopliilized SDS blank particles were incubated with 0.5mg of p55 gag protein from Example 12 an 10ml 25mM Borate buffer pH9 with 6M Urea. Particles were left on a lab rocker, (Aliquot mixer, Miles labs) at room temperature for 5 hours. The microparticles were separated from the incubation medium by centrifugation, and the SDS pellet was washed once with Borate buffer with 6M Urea then three times with distilled water, and lyophilized.

The loading level of protein adsorbed to microparticles was determined by dissolving 10mg of the microparticles in 2ml of 5% SDS-0.2M sodium hydroxide solution at room temperature. Protein concentration was measured by BCA protein assay (Pierce, Rockford, Illinois). The Zeta potential for both blank and adsorbed microparticles was measured using a Malvern Zeta analyzer (Malvem Instruments, UK).

### Example 14

### Preparation of Protein with MF59 Adjuvant

Recombinant HIV-1 g140.SF162(dV2) protein from Example 12 was combined with MF59 adjuvant as previously described (Barnett, S. W., et. 2001. J Virol. 75:5526-40).

### Example 15

### Immunization

Male and female rhesus macaques were housed at Southern Research Institute (Frederick, MD).

Plasmid DNA immunization was performed at weeks 0, 4, and 14. Rhesus were given intramuscular injections of 0.5 mg of pCMVgag from Example 11 (in saline or formulated with PLG/CTAB microparticles as described in Example 13 or formulated with MF59/DOTAP as described in Example 2) and 1.0 mg of pCMVenv from Example 11 (in saline or formulated with PLG/CTAB microparticles as described in Example 13) at 4 separate sites per animal (0.25 mg pCMVgag in upper right arm and upper right leg; 0.5 mg pCMVenv in upper left arm and upper left leg). Alternatively, rhesus were given intramuscular injections of 0.5 mg of pSINCPgag from Example 11 (in saline or formulated with PLG/CTAB microparticles as described in Example 13) and 1.0 mg of pSINCPenv from Example 11 (in saline or formulated with PLG/CTAB microparticles as described in Example 13) at 4 separate sites per animal.

Rhesus were boosted by intramuscular injection of 0.2 mg recombinant p55gag protein/PLG microparticles from Example 14 at week 29 and with 0.1 mg recombinant gp140env(dV2) protein/MF59 adjuvant from Example 15 at week 38.

### Example 16

### Antibody responses

At various times following immunization, heparinized blood was collected from anesthetized animals and plasma was recovered by centrifugation. Anti-HIV Gag and Env antibodies were measured by enzyme-linked immunosorbent assay (ELISA) as follows. Wells of microtiter plates were coated with recombinant HIV-1.SF2 p55gag protein or recombinant HIV-1.SF162 gp140env protein at 5 microgram/ml in PBS, 50 microliters per well, and incubated at 4°C overnight. The plates were washed six times with wash buffer (PBS, 0.3% Tween 20) and blocked at 37°C for 1 h with 200 microliters per well of blocking buffer (PBS, 0.3% Tween 20, 5% goat serum). Test samples were diluted 1:25 and then serially diluted threefold in blocking buffer. The block solution was aspirated, and then the plates were incubated at room temperature for 1 h with 70 microliters per well of each plasma dilution. After being washed six times, the plates were incubated for 1 h at 37°C with horseradish peroxidase-conjugated anti-IgG (1:8,000 dilution). Following six washes, the plates were developed with TMB substrate for 15 minutes. The reaction was stopped with 2N HCl and the optical densities (OD) measured at a wavelength of 450 nm. The titer was calculated to be the reciprocal of the dilution at which an OD₄₅₀ nm of 0.5 was achieved.

**Table 12. Rhesus Auti-gag plasma antibody titers**

| | | Time⁽¹⁾ | Pre | 2 wks post 1st | 2 wks post 2nd | 7 wks post 2nd | 2 wks post 3rd | 6 wks post 3rd | 10 wks post 3rd | 13 wks post 3rd | 2 wks post protein (4) |
|---|---|---|---|---|---|---|---|---|---|---|---|
| Plasmid DNA | Formulation | Week | 0 | 2 | 6 | 11 | 16 | 20 | 24 | 27 | 31 |
| pCMV-p55gag | saline | Geo mean⁽²⁾ | 5 | 6 | 19 | 19 | 118 | 66 | 91 | 41 | 1684 |
| | | LL⁽³⁾ | 5 | 5 | 8 | 8 | 68 | 32 | 60 | 22 | 955 |
| | | UL⁽³⁾ | 5 | 8 | 42 | 41 | 206 | 133 | 138 | 78 | 2971 |
| | | | | | | | | | | | |
| pCMV-p55gag | PLG/CTAB | Geo mean | 6 | 490 | 10770 | 4360 | 1637 | 550 | 349 | 286 | 9968 |
| | | LL | 5 | 302 | 6672 | 2325 | 970 | 340 | 190 | 188 | 7370 |
| | | UL | 8 | 795 | 17384 | 8179 | 2762 | 890 | 642 | 435 | 13484 |
| | | | | | | | | | | | |
| pCMV-p55gag | MF59/DOTAP | Geo mean | 7 | 142 | 5702 | 1479 | 3536 | 1481 | 1183 | 854 | 2126 |
| | | LL | 5 | 35 | 2275 | 577 | 1158 | 535 | 478 | 235 | 468 |
| | | UL | 9 | 568 | 14293 | 3796 | 10797 | 4098 | 2931 | 3103 | 9664 |
| | | | | | | | | | | | |
| pSINCP-p55gag | saline | Geo mean | 6 | 8 | 7 | 8 | 45 | 24 | 42 | 32 | 1003 |
| | | LL | 5 | 5 | 5 | 5 | 18 | 13 | 23 | 14 | 386 |
| | | UL | 8 | 11 | 11 | 13 | 114 | 46 | 78 | 72 | 2606 |
| | | | | | | | | | | | |
| pSINICP-p55gag | PLG/CTAB | Geo mean | 8 | 728 | 19256 | 3427 | 856 | 489 | 648 | 687 | 23543 |
| | | LL | 6 | 635 | 10711 | 2259 | 596 | 331 | 431 | 396 | 13750 |
| | | UL | 11 | 835 | 34619 | 5199 | 1229 | 723 | 975 | 1194 | 40312 |
| | | | | | | | | | | | |
| none | | Geomean | 9 | 12 | 9 | 8 | 7 | 7 | 8 | 8 | 7 |
| | | LL | 6 | 7 | 5 | 5 | 5 | 5 | 5 | 5 | 5 |
| | | UL | 13 | 19 | 16 | 14 | 9 | 11 | 14 | 14 | 9 |

| | | | | | | | | | | | |
|---|---|---|---|---|---|---|---|---|---|---|---|
| ⁽¹⁾ Relative to plasmid immunizations done at weeks 0, 4, and 14 | | | | | | | | | | | |
| ⁽²⁾ Geometric mean for the group | | | | | | | | | | | |
| ⁽³⁾ Group arithmetic means and standard errors calculated from log-transformed titers. LL = antilog (arithmetic mean - standard error). UL = antilog (arithmetic mean + standard error) | | | | | | | | | | | |
| ⁽⁴⁾ Recombinant p55gag protein adsorbed to anionic PLG microparticles administered at week 29 | | | | | | | | | | | |

**Table 13. Rhesus Anti-env plasma antibody titers**

| | | Time ⁽¹⁾ | Pre | 2 wks post 1st | 2 wks post 2nd | 7 wks post 2nd | 2 wks post 3rd | 6 wks post 3rd | 10 wks post 3rd | 13 wks post 3rd | 17 wks post 3rd | 23 wks post 3rd | 2 wks post protein | 8 wks post protei n⁽⁴⁾ |
|---|---|---|---|---|---|---|---|---|---|---|---|---|---|---|
| Plasmid DNA | Formulation | Week | 0 | 2 | 6 | 11 | 16 | 20 | 24 | 27 | 31 | 37 | 40 | 46 |
| pCMV-gp140env | saline | Geo mean⁽²⁾ | 5 | 5 | 589 | 81 | 2460 | 342 | 30 | 26 | 24 | 13 | 35807 | 17926 |
| | | LL⁽³⁾ | 5 | 5 | 273 | 32 | 1703 | 227 | 10 | 9 | 9 | 5 | 28151 | 14079 |
| | | UL⁽³⁾ | 5 | 5 | 1272 | 202 | 3555 | 515 | 94 | 72 | 69 | 31 | 45544 | 22824 |
| | | | | | | | | | | | | | | |
| pCMV-gp140env | PLG/CTAB | Geo mean | 6 | 5 | 3200 | 5290 | 1913 | 236 | 261 | 43 | 42 | 29 | 27939 | 13992 |
| | | LL | 5 | 5 | 1306 | 2430 | 879 | 87 | 201 | 18 | 17 | 13 | 18016 | 9019 |
| | | UL | 8 | 5 | 7841 | 11515 | 4163 | 640 | 338 | 105 | 101 | 64 | 43329 | 21707 |
| | | | | | | | | | | | | | | |
| pCMV-gp140env | MF59/DO TAP | Geo mean | 7 | 5 | 659 | 112 | 4823 | 589 | 258 | 223 | 171 | 121 | 5698 | 3301 |
| | | LL | 5 | 5 | 176 | 51 | 2125 | 169 | 90 | 79 | 64 | 48 | 972 | 645 |
| | | UL | 9 | 5 | 2461 | 248 | 10946 | 2052 | 742 | 630 | 451 | 304 | 33409 | 16903 |
| | | | | | | | | | | | | | | |
| pSINCP-gp140env | saline | Geo mean | 6 | 5 | 44 | 20 | 70 | 36 | 12 | 12 | 14 | 12 | 15294 | 7660 |
| | | LL | 5 | 5 | 12 | 8 | 13 | 10 | 5 | 5 | 5 | 5 | 7853 | 3931 |
| | | UL | 8 | 5 | 168 | 49 | 366 | 127 | 31 | 30 | 37 | 31 | 29787 | 14926 |
| | | | | | | | | | | | | | | |
| pSINCP-gp140env | PLG/CTA B | Geo mean | 8 | 15 | 8735 | 4266 | 1002 | 228 | 20 | 33 | 18 | 15 | 33801 | 16921 |
| | | LL | 6 | 8 | 5015 | 2817 | 656 | 152 | 8 | 15 | 8 | 8 | 27002 | 13521 |
| | | UL | 11 | | 3015214 | 6459 | 1531 | 341 | 46 | 74 | 38 | 31 | 42313 | 21175 |
| | | | | | | | | | | | | | | |
| none | none | Geo mean | 9 | 12 | 11 | 11 | 11 | 11 | 11 | 11 | 10 | 13 | 12 | 12 |
| | | LL | 6 | 5 | 5 | 5 | 5 | 5 | 5 | 5 | 5 | 5 | 5 | 5 |
| | | UL | 13 | 28 | 23 | 25 | 26 | 24 | 22 | 25 | 22 | 32 | 29 | 30 |

| | | | | | | | | | | | | | | |
|---|---|---|---|---|---|---|---|---|---|---|---|---|---|---|
| ⁽¹⁾ Relative to plasmid immunizations done at weeks 0, 4, and 14 | | | | | | | | | | | | | | |
| ⁽²⁾ Geometric mean for the group | | | | | | | | | | | | | | |
| ⁽³⁾ Group arithmetic means and standard errors calculated from log-transfouned titers. LL = antilog (anthmetic mean - standard error). UL = analog (arithmetic mean + standard error) | | | | | | | | | | | | | | |
| ⁽⁴⁾ Recombinant oligomeric gp140env(ΔV2) protein/MF59 adjuvant administered at week 38 | | | | | | | | | | | | | | |

### Example 17

### Cytolytic T Lymphocyte (CTL) responses

A pool of 51 synthetic peptides 20 amino acids (aa) long, overlapping by 10 aa, and spanning p55 gag, and a pool of 66 synthetic peptides 20 aa long, overlapping by 10 aa, and spanning gp140 were prepared. Rhesus macaque peripheral blood mononuclear cells (PBMC) were separated from heparinized blood by centrifugation on Ficoll-Paque (Pharmacia Biotech, Piscataway, N.J.) gradients. PBMC were cultured for 8 days in 24-well plates at 3 x 10⁶ per well in 1.5 ml of AIM-V/RPMI 1640 (50:50) culture medium (Gibco-BRL, Grand Island, N.Y.) supplemented with 10% fetal bovine serum Gag-specific CTL were stimulated by the addition of the gag peptide pool and env-specific CTL were stimulated by the addition of the env peptide pool. Cultures were supplemented with recombinant human interleukin-7 (IL-7; 15 ng/ml; R&D Systems, Minneapolis, Minn.). Human recombinant IL-2 (20 IU/ml; Proleukin;Chiron) was added on days 1, 3, and 6. Stable rhesus B-lymphoblastoid cell lines (B-LCL) were derived by exposing PBMC to herpesvirus papio-containing culture supernatant from the S594 cell line (Falk, L., et al. 1976. Properties of a baboon lymphotropic herpesvirus related to Epstein-Barr virus. Int J Cancer. 18:798-807. Rabin, H., et al. 1976. Virological studies of baboon (Papio hamadryas) lymphoma: isolation and characterization of foamyviruses. J Med Primatol. 5:13-22.) in the presence of 0.5 microgram/ml cyclosporin A (Sigma, St. Louis, MO). Autologous B-LCL were infected with recombinant vaccinia virus (rW) encoding HIV-1.SF2 gag-pol (rVVgag-pol) or HIV-1.SF162 gp160env (rVVgp160env) (PFU:cell ratio of 10) and concurrently labeled with Na[⁵¹Cr]₂O₄ (NEN, Boston, MA) at 25 microcurie per 1×10⁶ B-LCL. After overnight culture at 37°C, rVV-infected, ⁵¹Cr-labeled B-LCL were washed and then added (2,500 per round-bottomed well) to duplicate wells containing threefold serial dilutions of cultured PBMC. Then 10⁵ unlabeled, uninfected B-LCL were added per well to inhibit nonspecific cytolysis. After 4h incubation at 37°C, 50 microliters of culture supernatants were harvested and added to LumaPlates (Packard, Meriden, CT), and radioactivity was counted (counts per minute (cpm)) with a Microbeta 1450 liquid scintillation counter (Wallac, Gaithersburg, MD). ⁵¹Cr released from lysed targets was normalized by using the formula: % Specific ⁵¹Cr Release = 100% x (mean experimental cpm - SR)/(MR - SR), where SR = mean cpm from targets alone and MR = mean cpm from targets exposed to Triton X-100. An animal was determined to have a positive, p55gag-specific response if at two consecutive dilutions of the gag peptide pool-stimulated PBMC the lysis of rVVgag-pol-infected B-LCL exceeded lysis of rVVgp160env-infected B-LCL by at least 10% and if at two consecutive dilutions of cultured PBMC the lysis of rVVgag-pol-infected B-LCL by the gag peptide pool-stimulated PBMC exceed lysis of rVV gag-pol-infected B-LCL by env peptide pool-stimulated B-LCL by at least 10%. An animal was determined to have a positive, gp160-specific response if at two consecutive dilutions of the env peptide pool-stimulated PBMC the lysis of rVV gp160env-infected B-LCL exceeded lysis of rVV gag-pol-infected B-LCL by at least 10% and if at two consecutive dilutions of cultured PBMC the lysis of rVV gp160env-infected B-LCL by the env peptide pool-stimulated PBMC exceed lysis of rVV gp160env-infected B-LCL by gag peptide pool-stimulated B-LCL by at least 10%.

**Table 14. Gag-specific CTL Induction of p55gag-specific CTL by vaccines Number of positive animals per group (n=5)**

| | Week -2 | 2 | 6 | 11 | 16 | 20 | 24 |
|---|---|---|---|---|---|---|---|
| Vaccine | Time:pre | 2wp1st | 2wp2nd | 7wp2nd | 2wp3rd | 6wp3rd | 10wp3rd |
| pSINCP | 0 | 0 | 1 | 0 | 0 | 0 | |
| pSINCP/PLG | 0 | 0 | 3 | 1 | 1 | 0 | |
| pCMV | 0 | 0 | 4 | 1 | 4 | 3 | 1 |
| pCMV/PLG | 0 | 3 | 3 | 2 | 3 | 1 | 1 |
| pCMV/MF59 | 0 | 0 | 1 | 1 | 0 | 0 | |
| None | 0 | 0 | 0 | 0 | 0 | 0 | |

**Table 15. Env-specific CTL Induction of gp140env-specific CTL by vaccines Number of positive animals per group (n=5)**

| | Week:-2 | 2 | 6 | 11 | 16 | 20 | 24 |
|---|---|---|---|---|---|---|---|
| Vaccine | Time:pre | 2wp1st | 2wp2nd | 7wp2nd | 2wp3rd | 6wp3rd | 10wp3rd |
| pSINCP | 0 | 0 | 0 | 0 | 1 | 0 | |
| pSINCP/PLG | 0 | 0 | 1 | 0 | 1 | 0 | |
| pCMV | 0 | 0 | 0 | 0 | 0 | 1 | 1 |
| pCMV/PLG | 0 | 0 | 0 | 0 | 0 | 0 | 0 |
| pCMV/MF59 | 0 | 0 | 0 | 0 | 0 | 0 | |
| none | 0 | 0 | 0 | 0 | 0 | 0 | |

### Example 18

### Lymphoproliferation assay (LPA)

2 x 10⁵ rhesus PBMC per well were cultured in the presence or absence of recombinant p55gag protein or the pool of synthetic env peptides. Six replicate wells were established for each culture condition. After 4 days of incubation cultures were pulsed overnight with 1 microcurie/well of [³H]TdR. Incorporation of [³H]TdR into cells was determined by liquid scintillation counting (BetaPlate, Wallac, Gaithersburg, MD). Stimulation Index (SI) was calculated as SI = mean cpm (gag or env stimulation)/mean cpm (unstimulated).

**Table 16. Rhesus Anti-gag lymphoproliferation stimulation indices**

| | | Time ⁽¹⁾ | Pre | 2 wks post 1^{st} | 2 wks post 2nd | 7 wks post 2nd | 2 wks post 3rd | 6 wks post 3rd | 10 wks post 3rd | 13 wks post 3rd | 2 wks post prot ein ⁽⁴⁾ | 8 wks post prot ein | 11 wks post protein | 17 wks post protein |
|---|---|---|---|---|---|---|---|---|---|---|---|---|---|---|
| Plasmid DNA | Delivery | Week | 0 | 2 | 6 | 11 | 16 | 20 | 24 | 27 | 31 | 37 | 40 | 46 |
| | | | | | | | | | | | | | | |
| pCMV-p55gag | saline | Geo mean⁽²⁾ | 1 | 3 | 5 | 3 | 3 | 2 | 5 | 2 | 7 | 9 | 4 | 2 |
| | | LL⁽³⁾ | 1 | 2 | 4 | 2 | 3 | 2 | 3 | 2 | 4 | 6 | 2 | 2 |
| | | UL⁽³⁾ | 1 | 3 | 7 | 4 | 4 | 3 | 6 | 3 | 11 | 13 | 7 | 3 |
| | | | | | | | | | | | | | | |
| pCMV-p55gag | PLG/CTAB | Geo mean | 2 | 7 | 15 | 6 | 5 | 5 | 4 | 2 | 8 | 7 | 5 | 4 |
| | | LL | 1 | 3 | 7 | 3 | 3 | 3 | 2 | 2 | 4 | 3 | 2 | 2 |
| | | UL | 2 | 16 | 34 | 12 | 7 | 8 | 8 | 4 | 17 | 15 | 11 | 8 |
| | | | | | | | | | | | | | | |
| pCMV-p55gag | MF59/DOTAP | Geo mean | 1 | 9 | 30 | 14 | 11 | 10 | 9 | 11 | 19 | 10 | 8 | 5 |
| | | LL | 1 | 5 | 13 | 7 | 6 | 6 | 5 | 6 | 9 | 4 | 4 | 3 |
| | | UL | 1 | 18 | 65 | 27 | 17 | 15 | 15 | 21 | 42 | 25 | 14 | 7 |
| | | | | | | | | | | | | | | |
| pSINCP-p55gag | saline | Geo mean | 1 | 8 | 6 | 4 | 4 | 6 | 6 | 3 | 11 | 6 | 6 | 5 |
| | | LL | 1 | 4 | 3 | 2 | 3 | 3 | 3 | 2 | 6 | 4 | 3 | 3 |
| | | UL | 1 | 15 | 13 | 8 | 6 | 10 | 12 | 4 | 17 | 9 | 12 | 8 |
| | | | | | | | | | | | | | | |
| pSINCP-p55gag | PLG/CTAB | Geomean | 1 | 10 | 14 | 5 | 4 | 6 | 7 | 6 | 13 | 6 | 5 | 6 |
| | | LL | 1 | 7 | 9 | 4 | 3 | 4 | 4 | 4 | 10 | 5 | 3 | 4 |
| | | UL | 1 | 15 | 21 | 7 | 6 | 9 | 12 | 11 | 16 | 7 | 8 | 9 |
| | | | | | | | | | | | | | | |
| none | none | Geo mean | 1 | 1 | 2 | 1 | 1 | 1 | 2 | 1 | 2 | 1 | 1 | 1 |
| | | LL | 1 | 1 | 1 | 1 | 1 | 1 | 1 | 1 | 2 | 1 | 1 | 1 |
| | | UL | 2 | 2 | 2 | 2 | 1 | 1 | 2 | 1 | 3 | 1 | 1 | 1 |

| | | | | | | | | | | | | | | |
|---|---|---|---|---|---|---|---|---|---|---|---|---|---|---|
| ⁽¹⁾ Relative to plasmid immunizations done at weeks 0, 4, and 14 | | | | | | | | | | | | | | |
| ⁽²⁾ Geometric mean for the group | | | | | | | | | | | | | | |
| ⁽³⁾ Group arithmetic means and standard errors calculated from log-transformed titers. LL = antilog (arithmetic mean - standard error). UL = antilog (arithmetic mean + standard error) | | | | | | | | | | | | | | |
| ⁽⁴⁾ Recombinant p55gag protein adsorbed to anionic PLG microparticles administered at week 29 | | | | | | | | | | | | | | |

**Table 17. Rhesus anti-env lymphoproliferation stimulation indices**

| | | Time ⁽¹⁾ | Pre | 2 wks post 1st | 2 wks post 2nd | 7 wks post 2nd | 2 wks post 3rd | 6 wks post 3rd | 10 wks post 3rd | 13 wks post 3rd | 17 wks post 3rd | 23 wks post 3rd | 2 wks post prot ein | 8 wks post protein⁽⁴⁾ |
|---|---|---|---|---|---|---|---|---|---|---|---|---|---|---|
| Plasmid DNA | Delivery | Week | 0 | 2 | 6 | 11 | 16 | 20 | 24 | 27 | 31 | 37 | 40 | 46 |
| pCMV-gp140env | saline | Geo mean^{(2,5)} | | | | 4 | 4 | 3 | 4 | 4 | 4 | 4 | 22 | 9 |
| | | LL^{(3,3)} | | | | 3 | 3 | 2 | 3 | 3 | 2 | 2 | 12 | 6 |
| | | UL^{(3,5)} | | | | 6 | 6 | 4 | 5 | 6 | 6 | 5 | 40 | 15 |
| | | | | | | | | | | | | | | |
| pCMV-gp140env | PLG/CTAB | Geo mean | | | | 5 | 2 | 3 | 3 | 2 | 3 | 2 | 15 | 8 |
| | | LL | | | | 3 | 2 | 2 | 2 | 1 | 2 | 2 | 8 | 4 |
| | | UL | | | | 9 | 3 | 4 | 4 | 2 | 5 | 3 | 31 | 16 |
| | | | | | | | | | | | | | | |
| pCMV-gp140env | MF59/DOTAP | Geo mean | | | | 6 | 5 | 5 | 5 | 7 | 7 | 4 | 18 | 12 |
| | | LL | | | | 4 | 4 | 4 | 3 | 5 | 5 | 3 | 13 | 9 |
| | | UL | | | | 9 | 7 | 7 | 7 | 10 | 10 | 6 | 25 | 16 |
| | | | | | | | | | | | | | | |
| pSINCP-gp140env | saline | Geo mean | | | | 9 | 6 | 7 | 8 | 5 | 5 | 4 | 41 | 23 |
| | | LL | | | | 5 | 4 | 4 | 5 | 3 | 3 | 3 | 23 | 12 |
| | | UL | | | | 15 | 9 | 12 | 14 | 9 | 8 | 6 | 74 | 45 |
| | | | | | | | | | | | | | | |
| pSINCP-gp140env | PLG/CTAB | Geomean | | | | 10 | 3 | 4 | 6 | 6 | 4 | 3 | 34 | 26 |
| | | LL | | | | 7 | 3 | 3 | 4 | 3 | 3 | 2 | 19 | 15 |
| | | UL | | | | 15 | 4 | 6 | 10 | 9 | 6 | 4 | 62 | 47 |
| | | | | | | | | | | | | | | |
| none | none | Geomean | | | | 2 | 2 | 1 | 2 | 1 | 2 | 1 | 1 | 1 |
| | | LL | | | | 1 | 1 | 1 | 1 | 1 | 2 | 1 | 1 | 1 |
| | | UL | | | | 3 | 2 | 1 | 2 | 1 | 3 | 1 | 1 | 1 |

| | | | | | | | | | | | | | | |
|---|---|---|---|---|---|---|---|---|---|---|---|---|---|---|
| ⁽¹⁾ Relative to plasmid immunizations done at weeks 0, 4, and 14 | | | | | | | | | | | | | | |
| ⁽²⁾ Geometric mean for the group | | | | | | | | | | | | | | |
| ⁽³⁾ Group arithmetic means and standard errors calculated from log-transformed titers. LL = antilog (arithmetic meanstandard error). UL = antilog (arithmetic mean + standard error) | | | | | | | | | | | | | | |
| ⁽⁴⁾ Recombinant oligomeric gp140env(ΔV2) protein/Mf59 adjuvant administered at week 38 | | | | | | | | | | | | | | |
| ⁽⁵⁾ blank values: assay not performed | | | | | | | | | | | | | | |

### Example 19

### Intracellular cytokine immunofluorescence and flow cytometry

Rhesus PBMC (1 x10⁶ per well) were cultured overnight in the presence of Brefeldin A (Pharmingen, San Diego, CA) and anti-CD28 monoclonal antibody (mAb) (Pharmingen) and in the presence or absence of the gag or env peptide pools. Duplicate wells were prepared for each condition of stimulation. The next day cells were stained with peridinin chlorophyll protein (PerCP)-conjugated anti-CD8 mAb and allophycocyanin (APC)-conjugated anti-CD4 mAb (Becton Dickinson, San Jose, CA), fixed and permeabilized (Cytofix/Cytoperm, Pharmingen), and stained with fluorescein isothiocyanate (FITC)-conjugated anti-tumor necrosis factor-α (TNF-α) mAb and phycoerythrin (PE)-conjugated anti-interferon-γ (IFN-γ) mAb (Pharmingen). Stained cell samples were analyzed using a FACSCalibur™ flow cytometer and CellQuest™ software (Becton Dickinson). The fraction of cells positively stained for IFN-γ and TNF-α was calculated for the CD4+8- and CD8+4- T cell subsets. The number of gag- or env-specific cells was calculated by subtraction of the average IFN-γ/TNF-α, fraction found in the unstimulated control wells from the average IFN-γ/TNF-α fraction found in the gag- or env-stimulated wells.
For a given T cell subset (CD4+8- or CD8+4-) and antigen (gag or env) a response was designated as positive if the fraction of antigen-specific cells was at least 0.1%.

**Table 18. Gag-specific IFNγ⁺/TNFα⁺ CD4+ T cells Induction of p55gag-specific IFNγ⁺/TNFα⁺ CD4+ T cells by vaccines Number of Positive Animals Per Group (n=5)***

| | Pre | Post 1st | Post 2nd | Post 3rd | Post Protein |
|---|---|---|---|---|---|
| pSINCP | 0 | 0 | 0 | 0 | 1 |
| pSINCP/PLG | 0 | 0 | 2 | 0 | 0 |
| pCMV | 0 | 0 | 0 | 0 | 0 |
| pCMV/PLG | 0 | 0 | 2 | 0 | 1 |
| pCMV/B4F59 | 0 | 0 | 0 | 0 | 0 |
| none | 0 | 0 | 0 | 0 | 0 |

| | | | | | |
|---|---|---|---|---|---|
| *Positive: Frequency ≥ 0.1% | | | | | |

**Table 19. Eny-specific IFNγ⁺TNFα⁺ CD4+ T cells Induction of gp140env-specific IFNγ⁺/TNFα⁺ CD4+ T cells by vaccines Number of Positive Animals Per Group (n=5)***

| | Pre | Post 1st | Post 2nd | Post 3rd | Post Protein |
|---|---|---|---|---|---|
| pSINCP | 0 | 0 | 0 | 0 | 3 |
| pSINCP/PLG | 0 | 0 | 1 | 0 | 3 |
| pCMV | 0 | 0 | 1 | 0 | 2 |
| pCMV/PLG | 0 | 0 | 2 | 0 | 3 |
| pCMV/MF59 | 0 | 0 | 0 | 0 | 0 |
| none | 0 | 0 | 0 | 0 | 0 |

| | | | | | |
|---|---|---|---|---|---|
| *Positive: Frequency ≥ 0.1% | | | | | |

**Table 20. Gag-specific IFNγ⁺/TNFα⁺ CD8+ T cells Induction of p55gag-specific IFNγ⁺/TNFα⁺ CD8+T cells by vaccines Number of Positive Animals Per Group (n=5)***

| | Pre | Post 1st | Post 2nd | Post 3rd | Post Protein |
|---|---|---|---|---|---|
| PSINCP | 0 | 0 | 0 | 0 | 0 |
| pSINCP/PLG | 0 | 0 | 0 | 0 | 0 |
| PCMV | 0 | 0 | 0 | 1 | 0 |
| PCMV/PLG | 0 | 0 | 1 | 0 | 0 |
| PCMV/MF59 | 0 | 0 | 0 | 0 | 0 |
| None | 0 | 0 | 0 | 0 | 0 |

| | | | | | |
|---|---|---|---|---|---|
| *Positive: Frequency ≥0.1% | | | | | |

**Table 21. Env-specific IFNγ⁺/TNFα⁺ CD8+ T cells Induction of gp140env-specific IFNγ⁺/TNFα⁺ CD8+ T cells by vaccines Number of Positive Animals Per Group (n=5)***

| | Pre | Post 1st | Post 2nd | Post 3rd | Post Protein |
|---|---|---|---|---|---|
| pSINCP | 0 | 0 | 0 | 1 | 0 |
| pSINCP/PLG | 0 | 0 | 0 | 0 | 0 |
| pCMV | 0 | 0 | 0 | 0 | 0 |
| pCMV/PLG | 0 | 0 | 0 | 0 | 0 |
| pCMV/MF59 | 0 | 0 | 0 | 0 | 0 |
| None | 0 | 0 | 0 | 0 | 0 |

| | | | | | |
|---|---|---|---|---|---|
| *Positive: Frequency ≥0.1% | | | | | |

### REFERENCES:

Ref. 1 - International patent application WO99/24578.
Ref. 2 -International patent application WO99/36544.
Ref. 3 - International patent application WO99/57280.
Ref. 4 - International patent application WO00/22430.
Ref. 5 - Tettelin et al., (2000) *Science* 287:1809-1815.
Ref. 6 - International patent application WO96/29412.
Ref. 7 - Pizza el al. (2000) *Science* 287:1816-1820.
Ref. 8 - International patent application PCT/IB01/00166.
Ref. 9 -Bjune et al. (1991) *Lancet* 338(8775):1093-1096.
Ref. 10 -Fukasawa et al. (1990) *Vaccine* 17:2951-2958.
Ref 11 -Rosenqvist et al. (1998) *Dev. Biol. Stand.* 92:323-333.
Ref. 12- Costantino et al (1992) *Vaccine* 10:691-698.
Ref. 13- Costantino et al (1999) *Vaccine* 17:1251-1263.
Ref. 14- Watson (2000) *Padiatr Infect Dis J* 19:331-332.
Ref. 15- Rubin (2000) *Pediatr Clin North Am* 47:269-285, v.
Ref. 16- Jedrzejas (2001) *Microbiol Mol Biol Rev* 65:187-207.
Ref 17 -International patent application filed on 3rd July 2001 claiming priority from GB-0016363.4].
Ref. 18 -Kalman et al. (1999) *Nature Genetics* 21 :385-389.
Ref 19- Read et al. (2000) *Nucleic Acids Res* 28:1397-406.
Ref. 20- Shirai et al. (2000) *J*. *Infect*. *Dis*. 181(Suppl 3):S524-S527.
Ref. 21 -International patent application WO99/27105.
Ref 22 -International patent application WO00/27994.
Ref. 23 -International patent application WO00/37494.
Ref. 24 -International patent application WO99/28475.
Ref. 25 -Bell (2000) *Pediatr Infect Dis* J 19:1187-1188.
Ref. 26 -Iwarson (1995) *APMIS* 103:321-326.
Ref. 27 -Gerlich et al. (1990) *Vaccine* 8 Suppl:S63-68 & 79-80.
Ref. 28 -Hsu et al. (1999) *Clin Liver Dis* 3:901-915.
Ref. 29 -Gustafsson et al. (1996) *N. Engl. J. Med.* 334:349-355.
Ref. 30 -Rappuoli et al. (1991) *TIBTECH* 9:232-238.
Ref. 31 *-Vaccines* (1988) eds. Plotkin & Mortimer. ISBN 0-7216-1946-0.
Ref. 32 -Del Guidice et al. (1998) *Molecular Aspects of Medicine* 19:1-70.
Ref. 33 -International patent application WO93/18150.
Ref. 34 -International patent application WO99/53310.
Ref. 35 -International patent application WO98/04702.
Ref. 36 -Ross et al. (2001) *Vaccine* 19:4135-4142.
Ref. 37 -Sutter et al. (2000) *Pediatr Clin North Am* 47:287-308.
Ref. 38 -Zimmerman & Spann *(1999) Am Fam Physician* 59:113-118, 125-126.
Ref. 39 -Dreesen (1997) *Vaccine* 15 Suppl:S2-6.
Ref 40 *-MMWR Morb Mortal Wkly Rep* 1998 Jan 16;47(1):12, 19.
Ref. 41 -McMichael (2000) *Vaccine* 19 Suppl 1:S101-107.
Ref. 42 -Schuchat (1999) *Lancet* 353(9146):51-6.
Ref 43 -GB patent applications 0026333.5, 0028727.6 & 0105640.7.
Ref. 44 -Dale (1999) *Infect Dis Clin North Am* 13:227-43, viii
Ref. 45 -Ferretti et al. (2001) *PNAS USA* 98:465 8-4663.
Ref. 46 -Kuroda et al. (2001) *Lancet* 357(9264):1225-1240; see also pages 1218-1219.
Ref. 47 -Ramsay et al. (2001) *Lancet* 357(9251):195-196.
Ref. 48- Lindberg (1999) *Vaccine* 17 Suppl 2:S28-36.
Ref. 49 -Buttery & Moxon (2000) *JR Coll Physicians London* 34:163-168.
Ref. 50 -Ahmad & Chapnick (1999) *Infect Dis Clin North Am* 13:113-133, vii.
Ref. 51 -Goldblatt (1998) *J. Med. Microbiol.* 47:563-567.
Ref. 52 -European patent 0 477 508.
Ref 53 -US Patent No. 5,306,492.
Ref. 54 -International patent application WO98/42721.
Ref. 55 *-Conjugate Vaccines* (eds. Cruse et al.) ISBN 3805549326, particularly vol. 10:48-114.
Ref 56- Hermanson (1996) *Bioconjugate Techniques* ISBN: 0123423368 & 012342335X
Ref. 57 -European patent application 0372501.
Ref 58 -European patent application 0378881.
Ref 59 -European patent application 0427347.
Ref. 60 -International patent application WO93/17712.
Ref. 61 -International patent application WO98/58668.
Ref 62 -European patent application 0471177.
Ref 63 -International patent application WO00/56360.
Ref. 64 -international patent application WO00/61761.

## Claims

1. A method of producing a microparticle having an adsorbent surface to which a vector construct capable of expressing a selected nucleic acid sequence is adsorbed, said method comprising the steps of:
(a) emulsifying a mixture of a polymer solution and a detergent to form an emulsion, wherein the polymer solution comprises a polymer selected from the group consisting of a poly(α-hydroxy acid), a polyhydroxy butyric acid, a polycaprolactone, a polyorthoester, a polyanhydride, and a polycyanoacrylate, wherein the polymer is present at a concentration of about 1% to about 30% in an organic solvent, and wherein the detergent is present in the mixture at a weight to weight detergent to polymer ratio of from about 0.00001:1 to about 0.5:1;
(b) removing the organic solvent from the emulsion, to form said microparticle; and
(c) adsorbing the vector construct to the surface of the microparticle, wherein said vector construct is selected from the group consisting of an ELVIS vector and an RNA vector construct.

2. The method of claim 1, wherein the vector construct comprises a heterologous nucleic acid sequence encoding a member selected from the group consisting of a pharmaceutical, a polypeptide, a hormone, an enzyme, a transcription or translation mediator, an intermediate in a metabolic pathway, an immunomodulator, an antigen, and an adjuvant.

3. The method of claim 2, wherein the heterologous nucleic acid sequence encodes an antigen selected from the group consisting of HIV gp120, HIV gp140, HTV p24gag, HIV p55gag, and Influenza A hemagglutinin antigen.

4. The method of any one of the previous claims, wherein said heterologous nucleic acid sequence encodes an HIV gag polypeptide and comprises a sequence having at least 90% identity to a sequence selected from the group consisting of nucleotides 844-903 of SEQ ID NO:63, nucleotides 841-900 of SEQ ID NO:64, nucleotides 1213-1353 of SEQ ID NO:67, and nucleotides 82-1512 of SEQ ID NO:68.

5. The method of any one of claims 1 to 3, wherein said heterologous nucleic acid sequence encodes an HIV envelope polypeptide and comprises a sequence having at least 90% identity to a sequence selected from the group consisting of nucleotides 1513-2547 of SEQ ID NO:65 and nucleotides 1210-1353 of SEQ ID NO:66.

6. A microparticle made according to the method of any one of the previous claims.

7. A microparticle with an adsorbent surface to which a biologically active macromolecule has been adsorbed comprising: a microparticle selected from the group consisting of (a) a polymer microparticle comprising: (i) a polymer selected from the group consisting of a poly(α-hydroxy acid), a polyhydroxy butyric acid, a polycaprolactone, a polyorthoester, a polyanhydride, and a polycyanoacrylate; and a detergent; and (b) a submicron emulsion comprising: (i) a metabolizable oil; and (ii) one or more emulsifying agents; and
the biologically active macromolecule, wherein the biologically active macromolecule is a nucleic acid molecule comprising at least one vector construct selected from the group consisting of an ELVIS vector and an RNA vector construct.

8. The microparticle of claim 7, wherein the selected microparticle is the submicron emulsion, and (a) the oil is a terpenoid and (b) the one or more emulsifying agents comprise one or more non-ionic detergents and one or more cationic detergents.

9. The microparticle of claim 8, wherein the oil is squalene and the one or more emulsifying agents comprise: a polyoxyethylene sorbitan fatty acid ester, a sorbitan fatty acid ester, and DOTAP.

10. The microparticle of claim 7 wherein said polymer microparticle is selected as said microparticle.

11. The microparticle of claim 10, wherein said polymer microparticle comprises a poly(α-hydroxy acid) selected from the group consisting of poly(L-lactide), poly(D,L-lactide) and poly(D,L-lactide-co-glycolide).

12. The microparticle of claim 10 or 11, further comprising a second biologically active macromolecule entrapped within the microparticle, wherein the second biologically active macromolecule is a member selected from the group consisting of a polynucleotide, a polynucleoside, a pharmaceutical, a polypeptide, a hormone, an enzyme, a transcription or translation mediator, an intermediate in a metabolic pathway, an immunomodulator, an antigen, and an adjuvant.

13. The microparticle of any one of claims 10 to 12 wherein said vector construct is an ELVIS vector.

14. The microparticle of claim 13, wherein said ELVIS vector comprises a cDNA complement of an RNA vector construct derived from a member selected from the group consisting of alphavirus, picornavirus, togavirus, flavivirus, coronavirus, paramyxovirus, and yellow fever virus, and wherein said RNA vector construct further comprises a selected heterologous nucleotide sequence.

15. The microparticle of any one of claims 10 to 12, wherein said vector construct is an RNA vector construct derived from a member selected from the group consisting of alphavirus, picornavirus, togavirus, flavivirus, coronavirus, paramyxovirus, and yellow fever virus, and wherein said RNA vector construct comprises a selected heterologous nucleotide sequence.

16. The microparticle of claim 14 or 15, wherein said vector construct is derived from an alphavirus selected from the group consisting of Sindbis virus, Semliki Forest virus, Venezuelan equine encephalitis virus, or Ross River virus.

17. The microparticle of claim 13 or 14, wherein said vector construct comprises a heterologous nucleic acid sequence encoding a member selected from the group consisting of a pharmaceutical, a polypeptide, a hormone, an enzyme, a transcription or translation mediator, an intermediate in a metabolic pathway, an immunomodulator, an antigen, and an adjuvant.

18. The microparticle of claim 17, wherein said heterologous nucleic acid sequence encodes an antigen selected from the group consisting of HIV gp120, HIV gp140, HIV p24gag, HIV p55gag, and Influenza A hemagglutinin antigen.

19. The microparticle of claim 18, wherein said heterologous nucleic acid sequence encodes an HIV gag polypeptide and comprises a sequence having at least 90% identity to a sequence selected from the group consisting of nucleotides 844-903 of SEQ ID NOs:63, nucleotides 841-900 of SEQ ID NO:64, nucleotides 1213-1353 of SEQ ID NO:67, and nucleotides 82-1512 of SEQ ID NO:68.

20. The microparticle of claim 18, wherein said heterologous nucleic acid sequence encodes an HIV envelope polypeptide and comprises a sequence having at least 90% identity to a sequence selected from the group consisting of nucleotides 1513-2547 of SEQ ID NO:65 and nucleotides 1210-1353 of SEQ ID NO:66.

21. The microparticle of claim 13, wherein said vector construct is a vector selected from the group consisting of the ELVIS vectors pSINCP-gp140 and pSINCP-p55gag.

22. The microparticle of any one of claims 10 to 21 further comprising at least one second biologically active macromolecule adsorbed on the surface thereof, wherein the second biologically active macromolecule is at least one member selected from the group consisting of a polypeptide, a polynucleotide, a polynucleoside, an antigen, a pharmaceutical, a hormone, an enzyme, a transcription or translation mediator, an intermediate in a metabolic pathway, an immunomodulator, and an adjuvant.

23. The microparticle of claim 22, wherein the second biologically active macromolecule is an antigen selected from the group consisting of HIV gp120, HIV gp140, HIV p24gag, HIV p55gag, and Influenza A hemagglutinin antigen, a polynucleotide which encodes HIV gp140, or is an adjuvant.

24. The microparticle of claim 23, wherein the second biologically active macromolecule is an adjuvant that is an aluminum salt.

25. A microparticle composition comprising a microparticle of any one of claims 7 to 24 and a pharmaceutically acceptable excipient.

26. The microparticle composition of claim 25, further comprising an adjuvant.

27. The microparticle composition of claim 26, wherein the adjuvant is (a) a member selected from the group consisting of a CpG oligonucleotide or (b) an aluminum salt which is aluminum phosphate.

28. The microparticle composition of any one of claims 25 to 27 for use in inducing or raising an immune response in a host animal.

29. The microparticle composition of any one of claims 25 to 27 for use in delivering a therapeutically effective amount of a macromolecule to a host animal wherein the host animal is a vertebrate.

30. The microparticle composition of any one of claims 25 to 27 for use in treatment of a disease or as a vaccine.

31. The microparticle composition of claims 28 to 29 wherein said animal is a human.

32. Use of an adsorbed vector construct in the manufacture of a medicament for raising an immune response in a host animal, wherein;
(a) the vector construct comprises a heterologous nucleic acid sequence encoding a first antigen in an amount effective to elicit an immune response;
(b) the vector construct is adsorbed onto microparticles comprising (i) a polymer selected from the group consisting of a poly(α-hydroxy acid), a polyhydroxy butyric acid, a polycaprolactone, a polyorthoester, a polyanhydride and a polycyanoacrylate and (ii) a detergent;
(c) the administration of the adsorbed vector construct is followed by: boosting of the immunological response by administering a second antigen to the host animal; and
(d) the first and the second antigen can be the same or different.

33. Use of an adsorbed vector construct and a second antigen in the manufacture of a medicament for raising an immune response in a host animal, wherein;
(a) the vector construct comprises a heterologous nucleic acid sequence encoding a first antigen in an amount effective to elicit an immune response;
(b) the vector construct is adsorbed onto microparticles comprising (i) a polymer selected from the group consisting of a poly(α-hydroxy acid), a polyhydroxy butyric acid, a polycaprolactone, a polyorthoester, a polyanhydride and a polycyanoacrylate and (ii) a detergent;
(c) the second antigen is administered after the adsorbed vector construct and boosts the immune response in the animal; and
(d) the first antigen and the second antigen can be the same or different.

34. The use of claim 32 or 33 wherein the adsorbed vector construct is selected from a plasmid DNA and an RNA vector construct.

35. The use of claim 34, wherein the plasmid DNA is an ELVIS vector.

36. The use of claim 35, wherein the ELVIS vector comprises a cDNA complement of an RNA vector construct derived from a member selected from the group consisting of alphavirus, picornavirus, togavirus, flavivirus, coronavirus, paramyxovirus, and yellow fever virus.

37. The use of claim 36, wherein the alphavirus is selected from the group consisting of Sindbis virus, Semliki Forest virus, Venezuelan equine encephalitis virus, or Ross River virus.

38. The use of claim 34, wherein the plasmid DNA comprises a CMV promoter/enhancer.

39. The use of any one of claims 32 to 38, wherein the first and second antigens are selected from the group consisting of HIV antigens, hepatitis C virus antigens, and influenza A virus antigens.

40. The use of claim 39, wherein the first and second antigens selected from the group consisting of HIV antigens gp120, gp140, gp160, p24gag and p55gag.

41. The use of any one of claims 32 to 40, wherein the second antigen is adsorbed to microparticles comprising (i) a polymer selected from the group consisting of a poly(α-hydroxy acid), a polyhydroxy butyric acid, a polycaprolactone, a polyorthoester, a polyanhydride, and a polycyanoacrylate and (ii) a detergent.

42. The use of any one of claims 32 to 41, wherein the adsorbed vector construct and/or second antigen is coadministered with an adjuvant.

43. The use of claim 42, wherein the adjuvant is MF59.

44. The use of any one of claims 32 to 43, wherein the polymer comprises a poly(α-hydroxy acid) selected from the group consisting of poly(L-lactide), poly(D,L-lactide) and poly(D,L-lactide-co-glycolide) and wherein the detergent comprises a cationic detergent selected from CTAB, benzalkonium chloride, DDA and DOTAP.

45. The use of any one of claims 32 to 44, wherein the vector construct is administered two or more times before the second antigen is administered.

46. The use of claim 45, wherein the second antigen is also administered two or more times.

47. The use of claim 46, wherein the vector construct is administered (a) at a time of initial administration, (b) at a time period ranging 1-8 weeks from the initial administration, and (c) at a time period ranging 4-32 weeks from the initial administration, and wherein the second antigen is administered (a) at a time period ranging from 8-50 weeks from the initial administration and (b) at a time period ranging from 8-100 weeks from the initial administration.

48. The use of any one of claims 32 to 47, wherein the animal is a mammal selected from rhesus macaque and a human.

49. The use of any one of claims 32 to 48, wherein the vector construct and the second antigen are administered subcutaneously, intraperitoneally, intradermally, intravenously or intramuscularly.

50. The use of claim 32, wherein said immune response comprises a Th1 or CTL immune response.

51. The use of claim 32, wherein said immune response is raised against a viral, bacterial, or parasitic infection.

52. Use of the microparticle composition of claim 25 in the manufacture of a medicament for (a) immunizing a host animal against a viral, bacterial, or parasitic infection, (b) inducing a Th1 or CTL immune response in a host animal or (c) reducing the level of infection in a host animal having a viral, bacterial or parasitic infection.

53. The use of claim 52 wherein said host animal is a human.

## Patentansprüche

1. Verfahren zur Herstellung eines Mikropartikels, welches eine adsorbierende Oberfläche besitzt, an welche ein Vektorkonstrukt adsorbiert ist, welches geeignet ist zur Expression einer ausgewählten Nucleinsäuresequenz, wobei das Verfahren die folgenden Schritte umfasst:
(a) Emulgieren eines Gemisches aus einer Polymerlösung und einem Detergens zur Bildung einer Emulsion, wobei die Polymerlösung ein Polymer umfasst ausgewählt aus der Gruppe bestehend aus einer Poly(α-Hydroxysäure), einer Polyhydroxybuttersäure, einem Polycaprolacton, einem Polyorthoester, einem Polyanhydrid und einem Polycyanoacrylat, wobei das Polymer in einer Konzentration von etwa 1 % bis etwa 30% in einem organischen Lösungsmittel vorliegt ist und wobei das Detergens in dem Gemisch in einem Gewichtsverhältnis Detergens zu Polymer von etwa 0,00001:1 bis etwa 0,5:1 vorliegt;
(b) Entfernen des organischen Lösungsmittels von der Emulsion zur Bildung des Mikropartikels; und
(c) Adsorbieren des Vektorskonstrukts an die Oberfläche des Mikropartikels, wobei das Vektorkonstrukt ausgewählt ist aus der Gruppe bestehend aus einem ELVIS-Vektor und einem RNA-Vektorkonstrukt.

2. Verfahren nach Anspruch 1, wobei das Vektorkonstrukt eine heterologe Nucleinsäuresequenz umfasst, codierend ein Mitglied ausgewählt aus der Gruppe bestehend aus einem Arzneimittel, einem Polypeptid, einem Hormon, einem Enzym, einem Transkriptions- oder Translationsmediator, einem Zwischenprodukt eines Stoffwechselweges, einem Immunmodulator, einem Antigen und einem Adjuvants.

3. Verfahren nach Anspruch 2, wobei die heterologe Nucleinsäuresequenz ein Antigen codiert ausgewählt aus der Gruppe bestehend aus HIV gp120, HIV gp140, HIV p24gag, HIV p55gag und Influenza A Hämagglutininantigen.

4. Verfahren nach einem der vorhergehenden Ansprüche, wobei die heterologe Nucleinsäuresequenz ein HIV gag Polypeptid codiert und eine Sequenz umfasst, welche mindestens 90% Identität besitzt zu einer Sequenz ausgewählt aus der Gruppe bestehend aus Nucleotiden 844 - 903 der SEQ ID Nr.: 63, Nucleotiden 841 - 900 der SEQ ID Nr.: 64, Nucleotiden 1213 - 1353 der SEQ ID Nr.: 67 und Nucleotiden 82 - 1512 der SEQ ID Nr.: 68.

5. Verfahren nach einem der Ansprüche 1 bis 3, wobei die heterologe Nucleinsäuresequenz ein HIV-Hüllpolypeptid codiert und eine Sequenz umfasst, welche mindestens 90% Identität besitzt zu einer Sequenz ausgewählt aus der Gruppe bestehend aus Nucleotiden 1513 - 2547 der SEQ ID Nr.: 65 und Nucleotiden 1210 - 1353 der SEQ ID Nr.: 66.

6. Mikropartikel hergestellt nach einem Verfahren nach einem der vorhergehenden Ansprüche.

7. Mikropartikel mit einer adsorbierenden Oberfläche, an die ein biologisch aktives Makromolekül adsorbiert wurde, umfassend:
ein Mikropartikel ausgewählt aus der Gruppe bestehend aus
(a) einem Polymer-Mikropartikel umfassend: (i) ein Polymer ausgewählt aus der Gruppe bestehend aus einer Poly(α-Hydroxysäure), einer Polyhydroxybuttersäure, einem Polycaprolacton, einem Polyorthoester, einem Polyanhydrid und einem Polycyanoacrylat; und einem Detergens; und
(b) einer submikronen Emulsion umfassend: (i) ein verstoffwechselbares Öl; und (ii) einen oder mehrere emulgierende Stoffe; und
das biologisch aktive Makromolekül, wobei das biologisch aktive Makromolekül ein Nucleinsäuremolekül ist, umfassend mindestens ein Vektorkonstrukt ausgewählt aus der Gruppe bestehend aus einem ELVIS-Vektor und einem RNA-Vektorkonstrukt.

8. Mikropartikel nach Anspruch 7, wobei das ausgewählte Mikropartikel die submikrone Emulsion ist, und (a) das Öl ein Terpenoid ist und (b) der eine oder die mehreren emulgierenden Stoffe ein oder mehrere nicht-ionische Detergenzien umfassen und ein oder mehrere kationische Detergenzien.

9. Mikropartikel nach Anspruch 8, wobei das Öl Squalen ist und der eine oder die mehreren emulgierenden Stoffe umfassen: ein Polyoxyethylensorbitanfettsäureester, ein Sorbitanfettsäureester und DOTAP.

10. Mikropartikel nach Anspruch 7, wobei das Polymer-Mikropartikel ausgewählt ist als das Mikropartikel.

11. Mikropartikel nach Anspruch 10, wobei das Polymer-Mikropartikel eine Poly(a-Hydroxysäure) umfasst ausgewählt aus der Gruppe bestehend aus Poly(L-Lactid), Poly(D, L-Lactid) und Poly(D, L-Lactid-Co-Glycolid).

12. Mikropartikel nach Anspruch 10 oder 11, darüber hinaus umfassend ein zweites biologisch aktives Makromolekül, eingeschlossen in das Mikropartikel, wobei das zweite biologisch aktive Makromolekül ein Mitglied ist, ausgewählt aus der Gruppe bestehend aus einem Polynucleotid, einem Polynucleosid, einem Arzneimittel, einem Polypeptid, einem Hormon, einem Enzym, einem Transkriptions- oder Translationsmediator, einem Zwischenprodukt eines Stoffwechselweges, einem Immunmodulator, einem Antigen und einem Adjuvants.

13. Mikropartikel nach einem der Ansprüche 10 bis 12, wobei das Vektorkonstrukt ein ELVIS-Vektor ist.

14. Mikropartikel nach Anspruch 13, wobei der ELVIS-Vektor eine cDNA umfasst, welche komplementär ist zu einem RNA-Vektorkonstrukt, welches abgeleitet ist von einem Mitglied ausgewählt aus der Gruppe bestehend aus Alphaviren, Picornaviren, Togaviren, Flaviviren, Coronaviren, Paramyxoviren und Gelbfieberviren, und wobei das RNA-Vektorkonstrukt darüber hinaus eine ausgewählte heterologe Nucleotidsequenz umfasst.

15. Mikropartikel nach einem der Ansprüche 10 bis 12, wobei das Vektorkonstrukt ein RNA-Vektorkonstrukt ist, welches abgeleitet ist von einem Mitglied ausgewählt aus der Gruppe bestehend aus Alphaviren, Picornaviren, Togaviren, Flaviviren, Coronaviren, Paramyxoviren und Gelbfieberviren, und wobei das RNA-Vektorkonstrukt darüber hinaus eine ausgewählte heterologe Nucleotidsequenz umfasst.

16. Mikropartikel nach Anspruch 14 oder 15, wobei das Vektorkonstrukt abgeleitet ist von einem Alphavirus ausgewählt aus der Gruppe bestehend aus Sindbisvirus, Semlikiwaldvirus, venezuelanischer Pferdeencephalitisvirus oder Ross River-Virus.

17. Mikropartikel nach Anspruch 13 oder 14, wobei das Vektorkonstrukt eine heterologe Nucleinsäuresequenz umfasst, codierend ein Mitglied ausgewählt aus der Gruppe bestehend aus einem Arzneimittel, einem Polypeptid, einem Hormon, einem Enzym, einem Transkriptions- oder Translationsmediator, einem Zwischenprodukt eines Stoffwechselweges, einem Immunmodulator, einem Antigen und einem Adjuvants.

18. Mikropartikel nach Anspruch 17, wobei die heterologe Nucleinsäuresequenz ein Antigen codiert, ausgewählt aus der Gruppe bestehend aus HIV gp120, HIV gp140, HIV p24gag, HIV p55gag und Influenza A Hämagglutininantigen.

19. Mikropartikel nach Anspruch 18, wobei die heterologe Nucleinsäuresequenz ein HIV gag Polypeptid codiert und eine Sequenz umfasst, welche mindestens 90% Identität zu einer Sequenz besitzt ausgewählt aus der Gruppe bestehend aus Nucleotiden 844 - 903 der SEQ ID Nr.: 63, Nucleotiden 841 - 900 der SEQ ID Nr.: 64, Nucleotiden 1213 - 1353 der SEQ ID Nr.: 67 und Nucleotiden 82 - 1512 der SEQ ID Nr.: 68.

20. Mikropartikel nach Anspruch 18, wobei die heterologe Nucleinsäuresequenz ein HIV Hüllpolypeptid codiert und eine Sequenz umfasst, welche mindestens 90% Identität zu einer Sequenz besitzt ausgewählt aus der Gruppe bestehend aus Nucleotiden 1-513 - 2547 der SEQ ID Nr.: 65 und Nucieotiden 1210 - 1353 der SEQ ID Nr.: 66.

21. Mikropartikel nach Anspruch 13, wobei das Vektorkonstrukt ein Vektor ist, der ausgewählt ist aus der Gruppe bestehend aus den ELVIS-Vektoren pSINCP-gp140 und pSINCP-p55gag.

22. Mikropartikel nach einem der Ansprüche 10 bis 21, darüber hinaus umfassend mindestens ein zweites biologisch aktives Makromolekül, adsorbiert an seine Oberfläche, wobei das zweite biologisch aktive Makromolekül mindestens ein Mitglied ist ausgewählt aus der Gruppe bestehend aus einem Polypeptid, einem Polynucleotid, einem Polynucleosid, einem Antigen, einem Arzneimittel, einem Hormon, einem Enzym, einem Transkriptions- oder Translationsmediator, einem Zwischenprodukt eines Stoffwechselweges, einem Immunmodulator und einem Adjuvants.

23. Mikropartikel nach Anspruch 22, wobei das zweite biologisch aktive Makromolekül ein Antigen ist, ausgewählt aus der Gruppe bestehend aus HIV gp120, HIV gp140, HIV p24gag, HIV p55gag und Influenza A Hämagglutininantigen, ein Polynucleotid codierend HIV gp140 oder ein Adjuvants.

24. Mikropartikel nach Anspruch 23, wobei das zweite biologisch aktive Makromolekül ein Adjuvants ist, welches ein Aluminiumsalz ist.

25. Mikropartikelzusammensetzung umfassend ein Mikropartikel nach einem der Ansprüche 7 bis 24 und einen pharmazeutisch verträglichen Träger.

26. Mikropartikelzusammensetzung nach Anspruch 25, darüber hinaus umfassend ein Adjuvants.

27. Mikropartikelzusammensetzung nach Anspruch 26, wobei das Adjuvants (a) ein Mitglied ausgewählt aus der Gruppe bestehend aus einem CpG Oligonucleotid ist oder (b) ein Aluminiumsalz, welches ein Aluminiumphosphat ist.

28. Mikropartikelzusammensetzung nach einem der Ansprüche 25 bis 27 zur Verwendung bei der Induktion oder Steigerung einer Immunantwort in einem Wirtstier.

29. Mikropartikelzusammensetzung nach einem der Ansprüche 25 bis 27 zur Verwendung bei der Verabreichung einer therapeutisch wirksamen Menge eines Makromoleküls an ein Wirtstier, wobei das Wirtstier ein Vertebrate ist.

30. Mikropartikelzusammensetzung nach einem der Ansprüche 25 bis 27 zur Verwendung in der Behandlung einer Krankheit oder als Impfstoff.

31. Mikropartikelzusammensetzung nach Anspruch 28 bis 29, wobei das Tier ein Mensch ist.

32. Verwendung eines adsorbierten Vektorkonstrukts in der Herstellung eines Arzneimittels zur Auslösung einer Immunantwort in einem Wirtstier, wobei;
(a) das Vektorkonstrukt eine heterologe Nucleinsäuresequenz umfasst, welche ein erstes Antigen in einer Menge ausreichend zum Hervorrufen einer Immunantwort codiert;
(b) das Vektorkonstrukt adsorbiert ist an Mikropartikel umfassend (i) ein Polymer ausgewählt aus der Gruppe bestehend aus einer Poly(α-Hydroxysäure), einer Polyhydroxybuttersäure, einem Polycaprolacton, einem Polyorthoester, einem Polyanhydrid und einem Polycyanoacrylat und (ii) einem Detergens;
(c) der Verabreichung des adsorbierten Vektorkonstrukts das Auffrischen einer Immunantwort durch Verabreichung eines zweiten Antigens an das Wirtstier folgt; und
(d) das erste und das zweite Antigen identisch oder unterschiedlich sein können.

33. Verwendung eines adsorbierten Vektorkonstrukts und eines zweiten Antigens zur Herstellung eines Medikaments zur Auslösung einer Immunantwort in einem Wirtstier, wobei
(a) das Vektorkonstrukt eine heterologe Nucleinsäuesequenz umfasst, welche ein erstes Antigen in einer Menge geeignet zum Hervorrufen einer Immunantwort codiert;
(b) das Vektorkonstrukt adsorbiert ist an Mikropartikel umfassend (i) ein Polymer ausgewählt aus der Gruppe bestehend aus einer Poly(α-Hydroxysäure), einer Polyhydroxybuttersäure, einem Polycaprolacton, einem Polyorthoester, einem Polyanhydrid und einem Polycyanoacrylat und (ii) einem Detergens;
(c) das zweite Antigen verabreicht wird nach dem adsorbierten Vektorkonstrukt und die Immunantwort in dem Tier auffrischt; und
(d) das erste und das zweite Antigen identisch oder unterschiedlich sein können.

34. Verwendung nach Anspruch 32 oder 33, wobei das adsorbierte Vektorkonstrukt ausgewählt ist aus einer Plasmid-DNA und einem RNA-Vektorkonstrukt.

35. Verwendung nach Anspruch 34, wobei die Plasmid-DNA ein ELVIS-Vektor ist.

36. Verwendung nach Anspruch 35, wobei der ELVIS-Vektor eine cDNA umfasst, welche komplementär ist zu einem RNA-Vektorkonstrukt abgeleitet von einem Mitglied ausgewählt aus der Gruppe bestehend aus Alphaviren, Picornaviren, Togaviren, Flaviviren, Coronaviren, Paramyxoviren und Gelbfieberviren.

37. Verwendung nach Anspruch 36, wobei der Alphavirus ausgewählt ist aus der Gruppe bestehend aus Sindbisvirus, Semlikiwaldvirus, venezuelanischer Pferdeencephalitisvirus oder Ross River-Virus.

38. Verwendung nach Anspruch 34, wobei die Plasmid-DNA einen CMV-Promoter/Enhancer umfasst.

39. Verwendung nach einem der Ansprüche 32 bis 38, wobei das erste und zweite Antigen ausgewählt sind aus der Gruppe bestehend aus HIV-Antigenen, Hepatitis C Virus-Antigenen, und Influenza A Virus-Antigenen.

40. Verwendung nach Anspruch 39, wobei das erste und zweite Antigen ausgewählt sind aus der Gruppe bestehend aus HIV-Antigenen gp120, gp140, gp160, p24gag und p55gag.

41. Verwendung nach einem der Ansprüche 32 bis 40, wobei das zweite Antigen adsorbiert ist an Mikropartikel umfassend (i) ein Polymer ausgewählt aus der Gruppe bestehend aus Poly(α-Hydroxysäure), einer Polyhydroxybuttersäure, einem Polycaprolacton, einem Polyorthoester, einem Polyanhydrid und einem Polycyanoacrylat und (ii) einem Detergenz.

42. Verwendung nach einem der Ansprüche 32 bis 41, wobei das adsorbierte Vektorkonstrukt und/oder zweite Antigen zusammen mit einem Adjuvant verabreicht wird.

43. Verwendung nach Anspruch 42, wobei das Adjuvants MF59 ist.

44. Verwendung nach einem der Ansprüche 32 bis 43, wobei das Polymer eine Poly(α-Hydroxysäure) umfasst, ausgewählt aus der Gruppe bestehend aus Poly(L-lactid), Poly(D, L-lactid) und Poly(D, L-lactid-Co-Glycolid) und wobei das Detergenz ein kationisches Detergenz umfasst ausgewählt aus CTAB, Benzalkoniumchlorid, DDA und DOTAP.

45. Verwendung nach einem der Ansprüche 32 bis 44, wobei das Vektorkonstrukt zweimal oder mehrmals verabreicht wird, bevor das zweite Antigen verabreicht wird.

46. Verwendung nach Anspruch 45, wobei das zweite Antigen ebenfalls zweimal oder mehrmals verabreicht wird.

47. Verwendung nach Anspruch 46, wobei das Vektorkonstrukt verabreicht wird (a) zum Zeitpunkt der ersten Verabreichung, (b) in einem Zeitraum zwischen 1 und 8 Wochen nach der ersten Verabreichung, und (c) in einem Zeitraum zwischen 4 und 32 Wochen nach der ersten Verabreichung und wobei das zweite Antigen verabreicht wird (a) in einem Zeitraum von 8 - 50 Wochen nach der ersten Verabreichung und (b) in einem Zeitraum von 8 - 100 Wochen nach der ersten Verabreichung.

48. Verwendung nach einem der Ansprüche 32 bis 47, wobei das Tier ein Säugetier ist, ausgewählt aus Rhesusaffe und Mensch.

49. Verwendung nach einem der Ansprüche 32 bis 48, wobei das Vektorkonstrukt und das zweite Antigen subcutan, intraperitoneal, intradermal, intravenös oder intramuskulär verabreicht werden.

50. Verwendung nach Anspruch 32, wobei die Immunantwort eine Th1 oder CTL-Immunantwort umfasst.

51. Verwendung nach Anspruch 32, wobei die Immunantwort ausgelöst wird gegen eine virale, bakterielle oder parasitäre Infektion.

52. Verwendung der Mikropartikelzusammensetzung nach Anspruch 25 in der Herstellung eines Medikaments zur (a) Immunisierung eines Wirtstiers gegen eine virale, bakterielle oder parasitäre Infektion, (b) zum Auslösen einer Th1 oder CTL-Immunantwort in einem Wirtstier oder (c) zum Reduzieren des Grades einer Infektion in einem Wirtstier, welches eine virale, bakterielle oder parasitäre Infektion hat.

53. Verwendung nach Anspruch 52, wobei das Wirtstier ein Mensch ist.

## Revendications

1. Procédé de production d'une microparticule ayant une surface adsorbante sur laquelle un hybride de vecteur capable d'exprimer une séquence d'acides nucléiques choisie est adsorbé, ledit procédé comprenant les étapes consistant à :
(a) émulsionner un mélange d'une solution polymère et un détergent pour former une émulsion, dans lequel la solution polymère comprend un polymère choisi parmi le groupe constitué d'un acide poly(α-hydroxy), d'un acide polyhydroxybutyrique, d'une polycaprolactone, d'un polyorthoester, d'un polyanhydride et d'un polycyanoacrylate, dans lequel le polymère est présent à une concentration d'environ 1 % à environ 30 % dans un solvant organique, et dans lequel le détergent est présent dans le mélange à un rapport poids en poids détergent au polymère d'environ 0,00001 : 1 à environ 0,5 : 1 ;
(b) retirer le solvant organique de l'émulsion, pour former ladite microparticule ; et
(c) adsorber l'hybride de vecteur sur la surface de la microparticule, dans lequel ledit hybride de vecteur est choisi parmi le groupe constitué d'un vecteur ELVIS et d'un hybride de vecteur à ARN.

2. Procédé selon la revendication 1, dans lequel l'hybride de vecteur comprend une séquence d'acides nucléiques hétérologues codant un élément choisi parmi le groupe constitué d'un produit pharmaceutique, d'un polypeptide, d'une hormone, d'une enzyme, d'un médiateur de la transcription ou de la traduction, d'un intermédiaire dans une voie métabolique, d'un immunomodulateur, d'un antigène et d'un adjuvant.

3. Procédé selon la revendication 2, dans lequel la séquence d'acides nucléiques hétérologues code un antigène choisi parmi le groupe constitué de gp120 du VIH, gp140 du VIH, p24gag du VIH, p55gag du VIH et l'antigène de l'hémagglutinine de la grippe aviaire H5N1.

4. Procédé selon l'une quelconque des revendications précédentes, dans lequel ladite séquence d'acides nucléiques hétérologues code un polypeptide gag du VIH et comprend une séquence ayant au moins une identité à 90 % avec une séquence choisie parmi le groupe constitué des nucléotides 844 à 903 de SEQ ID NO : 63, des nucléotides 841 à 900 de SEQ ID NO : 64, des nucléotides 1213 à 1353 de SEQ ID NO : 67 et des nucléotides 82 à 1512 de SEQ ID NO : 68.

5. Procédé selon l'une quelconque des revendications 1 à 3, dans lequel ladite séquence d'acides nucléiques hétérologues code un polypeptide à enveloppe du VIH et comprend une séquence ayant au moins une identité à 90 % avec une séquence choisie parmi le groupe constitué des nucléotides 1513 à 2547 de SEQ ID NO : 65 et des nucléotides 1210 à 1353 de SEQ ID NO : 66.

6. Microparticule fabriquée conformément au procédé selon l'une quelconque des revendications précédentes.

7. Microparticule ayant une surface adsorbante sur laquelle une macromolécule biologiquement active a été adsorbée comprenant : une microparticule choisie parmi le groupe constitué de (a) une microparticule polymère comprenant : (i) un polymère choisi parmi le groupe constitué d'un acide poly(a-hydroxy), d'un acide polyhydroxybutyrique, d'une polycaprolactone, d'un polyorthoester, d'un polyanhydride et d'un polycyanoacrylate ; et un détergent ; et (b) une émulsion submicronique comprenant : (i) une huile métabolisable ; et (ii) un ou plusieurs agents d'émulsion ; et
la macromolécule biologiquement active, dans laquelle la macromolécule biologiquement active est une molécule d'acides nucléiques comprenant au moins un hybride de vecteur choisi parmi le groupe constitué d'un vecteur ELVIS et d'un hybride de vecteur à ARN.

8. Microparticule selon la revendication 7, dans laquelle la microparticule choisie est l'émulsion submicronique et (a) l'huile est un terpénoide et (b) le ou les agents d'émulsion comprennent un ou plusieurs détergents non ioniques et un ou plusieurs détergents cationique.

9. Microparticule selon la revendication 8, dans laquelle l'huile est un squalène et le ou les agents d'émulsion comprennent : un ester d'acide gras de sorbitan polyoxyéthyléné, un ester d'acide gras de sorbitan et DOTAP.

10. Microparticule selon la revendication 7 dans laquelle ladite microparticule polymère est choisie en tant que ladite microparticule.

11. Microparticule selon la revendication 10, dans laquelle ladite microparticule polymère comprend un acide poly(α-hydroxy) choisi parmi le groupe constitué de poly(L-lactide), poly(D,L-lactide) et poly(D,L-lactide-coglycolide).

12. Microparticule selon la revendication 10 ou 11, comprenant en outre une seconde macromolécule biologiquement active piégée dans la microparticule, dans laquelle la seconde macromolécule biologiquement active est un élément choisi parmi le groupe constitué d'un polynucléotide, d'un polynucléoside, d'un produit pharmaceutique, d'un polypeptide, d'une hormone, d'une enzyme, d'un médiateur de la transcription ou de la traduction, d'un intermédiaire dans une voie métabolique, d'un immunomodulateur, d'un antigène et d'un adjuvant.

13. Microparticule selon l'une quelconque des revendications 10 à 12, dans laquelle ledit hybride de vecteur est un vecteur ELVIS.

14. Microparticule selon la revendication 13, dans laquelle ledit vecteur ELVIS comprend un complément d'ADNc d'un hybride de vecteur à ARN dérivé d'un élément choisi parmi le groupe constitué d'un alphavirus, d'un picornavirus, d'un togavirus, d'un flavivirus, d'un coronavirus, d'un paramyxovirus et d'un virus de la fièvre jaune, et dans laquelle ledit hybride de vecteur à ARN comprend en outre une séquence nucléotidique hétérologue choisie.

15. Microparticule selon l'une quelconque des revendications 10 à 12, dans laquelle ledit hybride de vecteur est un hybride de vecteur à ARN dérivé d'un élément choisi parmi le groupe d'un alphavirus, d'un picornavirus, d'un togavirus, d'un flavivirus, d'un coronavirus, d'un paramyxovirus et d'un virus de la fièvre jaune, et dans laquelle ledit hybride de vecteur à ARN comprend en outre une séquence nucléotidique hétérologue choisie.

16. Microparticule selon la revendication 14 ou 15, dans laquelle ledit hybride de facteur est dérivé d'un alphavirus choisi parmi le groupe constitué d'un virus Sindbis, d'un virus de la forêt de Semliki, d'un virus de l'encéphalite équine du vénézuéla, ou d'un virus de la rivière Ross.

17. Microparticule selon la revendication 13 ou 14, dans laquelle ledit hybride de vecteur comprend une séquence d'acides nucléiques hétérologues codant un élément choisi parmi le groupe constitué d'un produit pharmaceutique, d'un polypeptide, d'une hormone, d'une enzyme, d'un médiateur de la transcription ou de la traduction, d'un intermédiaire dans une voie métabolique, d'un immunomodulateur, d'un antigène et d'un adjuvant.

18. Microparticule selon la revendication 17, dans laquelle ladite séquence d'acides nucléiques hétérologues code un antigène choisi parmi le groupe constitué de gp120 du VIH, gp140 du VIH, p24gag du VIH, p55gag du VIH et de l'antigène hémagglutinine de la grippe aviaire H5N1.

19. Microparticule selon la revendication 18, dans laquelle ladite séquence d'acides nucléiques hétérologues code un polymère gag du VIH et comprend une séquence ayant au moins une identité à 90 % avec une séquence choisie parmi le groupe constitué des nucléotides 844 à 903 de SEQ ID NO : 63, des nucléotides 841 à 900 de SEQ ID NO : 64, des nucléotides 1213 à 1353 de SEQ ID NO : 67 et des nucléotides 82 à 1512 de SEQ ID NO : 68.

20. Microparticule selon la revendication 18, dans laquelle ladite séquence d'acides nucléiques hétérologues code un polypeptide à enveloppe du VIH et comprend une séquence ayant au moins une identité à 90 % avec une séquence choisie parmi le groupe constitué des nucléotides 1513 à 2547 de SEQ ID NO : 65 et des nucléotides 1210 à 1353 de SEQ ID NO : 66.

21. Microparticule selon la revendication 13, dans laquelle ledit hybride de vecteur est un vecteur choisi parmi le groupe constitué des vecteurs ELVIS pSINCP-gp140 et pSINCP-P55gag.

22. Microparticule selon l'une quelconque des revendications 10 à 21 comprenant en outre au moins une seconde macromolécule biologiquement active adsorbée sur sa surface, dans laquelle la seconde macromolécule biologiquement active est au moins un élément choisi parmi le groupe constitué d'un polypeptide, d'un polynucléotide, d'un polynucléoside, d'un antigène, d'un produit pharmaceutique, d'une hormone, d'une enzyme, d'un médiateur de la transcription ou de la traduction, d'un intermédiaire dans une voie métabolique, d'un immunomodulateur et d'un adjuvant.

23. Microparticule selon la revendication 22, dans laquelle la seconde macromolécule biologiquement active est un antigène choisi parmi le groupe constitué de gp120 du VIH, gp140 du VIH, p24gag du VIH, p55gag du VIH et de l'antigène ; de l'hémagglutinine de la grippe aviaire H5N1, d'un polynucléotide qui code gp140 du VIH ou est un adjuvant.

24. Microparticule selon la revendication 23, dans laquelle la seconde macromolécule biologiquement active est un adjuvant qui est un sel d'aluminium.

25. Composition de microparticule comprenant une microparticule selon l'une quelconque des revendications 7 à 24 et un excipient acceptable d'un point de vue pharmaceutique.

26. Composition de microparticule selon la revendication 25, comprenant en outre un adjuvant.

27. Composition de microparticule selon la revendication 26, dans laquelle l'adjuvant est (a) un élément choisi parmi le groupe constitué d'un oligonucléotide CpG ou (b) un sel d'aluminium qui est un phosphate d'aluminium.

28. Composition de microparticule selon l'une quelconque des revendications 25 à 27 à utiliser pour induire ou élever une réponse immunitaire chez un animal hôte.

29. Composition de microparticule selon l'une quelconque des revendications 25 à 27 à utiliser dans la délivrance d'une quantité ayant une efficacité thérapeutique d'une macromolécule à un animal hôte dans laquelle l'animal hôte est un vertébré.

30. Composition de microparticule selon l'une quelconque des revendications 25 à 27 à utiliser dans le traitement d'une maladie ou en tant que vaccin.

31. Composition de microparticule selon les revendications 28 à 29 dans laquelle ledit animal est un humain.

32. Utilisation d'un hybride d'un vecteur adsorbé dans la fabrication d'un médicament pour élever une réponse immunitaire chez un animal hôte, dans laquelle :
(a) l'hybride de vecteur comprend une séquence d'acides nucléiques hétérologues codant un premier antigène dans une quantité efficace pour provoquer une réponse immunitaire ;
(b) l'hybride de vecteur est adsorbé sur des microparticules comprenant (i) un polymère choisi parmi le groupe constitué d'un acide poly(α-hydroxy), d'un acide polyhydroxybutyrique, d'une polycaprolactone, d'un polyorthoester, d'un polyanhydride et d'un polycyanoacrylate ; et (ii) d'un détergent ;
(c) l'administration de l'hybride de vecteur adsorbé est suivi par : la promotion d'une réponse immunologique par administration d'un second antigène à l'animal hôte ; et
(d) le premier et le second antigènes peuvent être identiques ou différents.

33. Utilisation d'un hybride de vecteur adsorbé et d'un second antigène dans la fabrication d'un médicament pour élever une réponse immunitaire chez un animal hôte, dans laquelle :
(a) l'hybride de vecteur comprend une séquence d'acides nucléiques hétérologues codant un premier antigène en une quantité efficace pour provoquer une réponse immunitaire ;
(b) l'hybride de vecteur est adsorbé sur les microparticules comprenant (i) un polymère choisi parmi le groupe constitué d'un acide poly(α-hydroxy), d'un acide polyhydroxybutyrique, d'une polycaprolactone, d'un polyorthoester, d'un polyanhydride et d'un polycyanoacrylate ; et (ii) d'un détergent ;
(c) le second antigène est administré après l'hybride de vecteur adsorbé et promeut la réponse immunitaire chez l'animal ; et
(d) le premier antigène et le second antigène peuvent être identiques ou différents.

34. Utilisation selon la revendication 32 ou 33 dans laquelle l'hybride de vecteur adsorbé est choisi parmi un ADN plasmidique et un hybride de vecteur à ARN.

35. Utilisation selon la revendication 34, dans laquelle l'ADN plasmidique est un vecteur ELVIS.

36. Utilisation selon la revendication 35, dans laquelle le vecteur ELVIS comprend un complément d'ADNc d'un hybride de vecteur à ARN dérivé d'un élément choisi parmi le groupe constitué d'un alphavirus, d'un picornavirus, d'un togavirus, d'un flavivirus, d'un coronavirus, d'un paramyxovirus et d'un virus de la fièvre jaune.

37. Utilisation selon la revendication 36, dans laquelle l'alphavirus est choisi parmi le groupe constitué d'un virus Sindbis, d'un virus de la forêt de Semliki, d'un virus de l'encéphalite équine du Vénézuéla, ou d'un virus de la rivière Ross.

38. Utilisation selon la revendication 34, dans laquelle l'ADN plasmidique comprend un promoteur/stimulateur CMV.

39. Utilisation selon l'une quelconque des revendications 32 à 38, dans laquelle le premier et le second antigènes sont choisis parmi le groupe constitué d'antigènes du VIH, d'antigènes du virus de l'hépatite C et d'antigènes du virus de la grippe aviaire H5N1.

40. Utilisation selon la revendication 39, dans laquelle le premier et le second antigènes sont choisis parmi le groupe constitué d'antigène gp120, gp140, gp160, p24gag et p55gag du VIH.

41. Utilisation selon l'une quelconque des revendications 32 à 40, dans laquelle le second antigène est adsorbé sur des microparticules comprenant (i) un polymère choisi parmi le groupe constitué d'un acide poly(α-hydroxy), d'un acide polyhydroxybutyrique, d'une polycaprolactone, d'un polyorthoester, d'un polyanhydride et d'un polycyanoacrylate ; et (ii) d'un détergent.

42. Utilisation selon l'une quelconque des revendications 32 à 41, dans laquelle l'hybride de vecteur adsorbé et/ou le second antigène est coadministré avec un adjuvant.

43. Utilisation selon la revendication 42, dans laquelle l'adjuvant est MF59.

44. Utilisation selon l'une quelconque des revendications 32 à 43, dans laquelle le polymère comprend un acide poly(α-hydroxy) choisi parmi le groupe constitué de poly(L-lactide), poly(D,L-lactide) et poly(D,L-lactide-co-glycolide) et dans laquelle le détergent comprend un détergent cationique choisi parmi CTAB, le chlorure de benzalkonium, DDA et DOTAP.

45. Utilisation selon l'une quelconque des revendications 32 à 44, dans laquelle l'hybride de vecteur est administré deux fois ou plus avant l'administration du second antigène.

46. Utilisation selon la revendication 45, dans laquelle le second antigène est également administré deux fois ou plus.

47. Utilisation selon la revendication 46, dans laquelle l'hybride de vecteur est administré (a) au moment de l'administration initiale, (b) à une période variant de 1 à 8 semaines à partir de l'administration initiale, et (c) à une période variant de 4 à 32 semaines à partir de l'administration initiale, et dans laquelle le second antigène est administré (a) à une période variant de 8 à 50 semaines à partir de l'administration initiale et (b) à une période variant de 8 à 100 semaines à partir de l'administration initiale.

48. Utilisation selon l'une quelconque des revendications 32 à 47, dans laquelle l'animal est un mammifère choisi parmi le macaque rhésus et un humain.

49. Utilisation selon l'une quelconque des revendications 32 à 48, dans laquelle l'hybride de vecteur et le second antigène sont administrés par voie sous-cutanée, intrapéritonéale, intradermique, intraveineuse ou intramusculaire.

50. Utilisation selon la revendication 32, dans laquelle ladite réponse immunitaire comprend une réponse immunitaire Th1 ou CTL.

51. Utilisation selon la revendication 32, dans laquelle ladite réponse immunitaire est élevée contre une infection virale, bactérienne ou parasitaire.

52. Utilisation de la composition de microparticule selon la revendication 25 dans la fabrication d'un médicament pour (a) immuniser un animal hôte contre une infection virale, bactérienne ou parasitaire, (b) induire une réponse immunitaire Th1 ou CTL chez un animal hôte ou (c) réduire le niveau d'infection chez un animal hôte ayant une infection virale, bactérienne ou parasitaire.

53. Utilisation selon la revendication 52 dans laquelle ledit animal hôte est un humain.
